# EUROPEAN PATENT SPECIFICATION

(11) **EP 1 663 966 B1**
(45) Date of publication and mention of the grant of the patent: **20.06.2007**
(21) Application number: 04765174.0
(22) Date of filing: 14.09.2004
(51) Int. Cl.: C07D 205/04, C07D 417/12, C07D 403/12, C07D 413/12, A61K 31/397, A61P 29/00, A61P 37/08

(54) **1,3-DISUBSTITUTED AZETIDINE DERIVATIVES FOR USE AS CCR-3 RECEPTOR ANTAGONISTS IN THE TREATMENT OF INFLAMMATORY AND ALLERGIC DISEASES**
1,3-DISUBSTITUIERTE AZETIDINDERIVATE ZUR VERWENDUNG ALS ANTAGONISTEN DES CCR-3-REZEPTORS BEI DER BEHANDLUNG VON ENTZÜNDLICHEN UND ALLERGISCHEN KRANKHEITEN
DERIVES D'AZETIDINE 1, 3-DISUBSTITUES A UTILISER EN TANT QU'ANTAGONISTES DU RECEPTEUR CCR3 DANS LE TRAITEMENT DE MALADIES INFLAMMATOIRES ET ALLERGIQUES

(30) Priority: 15.09.2003 GB 0321600; 15.09.2003 GB 0321599
(43) Date of publication of application: 07.06.2006
(73) Proprietor: Novartis AG, 4056 Basel (CH); Novartis Pharma GmbH, 1230 Vienna (AT)
(72) Inventor: LE GRAND, Darren M. Novartis Horsham Research Ctr., Horsham, West Sussex RH12 5AB (GB)
(74) Representative: Hillebrand, Dirk
(86) International application number: PCT/EP2004/010260
(87) International publication number: WO 2005/026113

(56) References cited:
- WO-A-00/35449
- WO-A-03/007939
- WO-A-03/077907

## Description

This invention relates to organic compounds, their preparation and their use as pharmaceuticals.

WO 03/007939, WO 00/35449 and intermediate document WO 03/077907 describe modulators of CCR3.

In one aspect the invention provides compounds of formula Ia or Ib 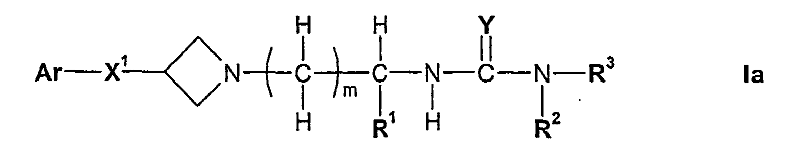 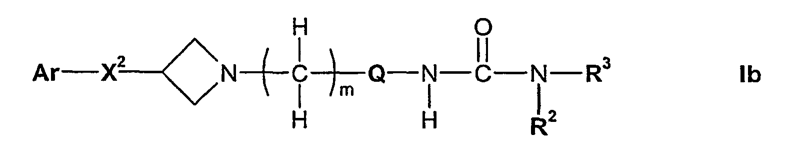 in free or salt form, where
Ar is phenyl optionally substituted by one or more substituents selected from halogen, C₁-C₈-alkyl, cyano or nitro;
X¹ is -S-, -S(=O)- or =S(=O)₂-;
X² is -C(=O)-, -O-, -CH₂-, -S-, -S(=O)- or -S(=O)₂-;
m is 1,2,3 or 4;
R¹ is hydrogen or C₁-C₈-alkyl optionally substituted by hydroxy, C₁-C₈-alkoxy, acyloxy, halogen, carboxy, C₁-C₈-alkoxycarbonyl, -N(R⁴)R⁵, -CON(R⁶)R⁷ or by a monovalent cyclic organic group having 3 to 15 atoms in the ring system;
Q has the formula

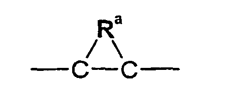

where R^{a} is C₁-C₈-alkylene,
or Q is -C(R^{b})(R^{c})- where R^{b} and R^{c} are independently C₁-C₈-alkyl
or R^{b} and R^{c} together form a C₃-C₁₀-cycloalkyl;
Y is oxygen or sulfur;
R² is hydrogen, C₁-C₈-alkyl or C₃-C₁₀-cycloalkyl and R³ is C₁-C₈-alkyl substituted by phenyl, phenoxy, acyloxy or naphthyl, or R³ is C₃-C₁₀-cycloalkyl optionally having a benzo group fused thereto, a heterocyclic group having 5 to 11 ring atoms of which 1 to 4 are hetero atoms, phenyl or naphthyl, said phenyl, phenoxy or naphthyl groups being optionally substituted by one or more substituents selected from halogen, cyano, hydroxy, acyl, nitro, -SO₂NH₂, C₁-C₈-alkyl optionally substituted by C₁-C₈-alkoxy, C₁-C₈-haloalkyl, C₁-C₈-alkoxy, C₁-C₈-haloalkoxy, C₁-C₈-alkylthio, -SO₂-C₁-C₈-alkyl C₁-C₈-alkoxycarbonyl, C₁-C₈-acylamino optionally substituted on the nitrogen atom by C₁-C₈-alkyl, C₁-C₈-alkylamino, aminocarbonyl, C₁-C₈-alkylamino-carbonyl, di(C₁-C₈-alkyl)amino, di(C₁-C₈-alkyl)aminocarbonyl, di(C₁-C₈-alkyl)aminocarbonyl-methoxy,
or R² and R³ together with the nitrogen atom to which they are attached denote a heterocyclic group having 5 to 10 ring atoms of which 1, 2 or 3 are hetero atoms;
R⁴ and R⁵ are each independently hydrogen or C₁-C₈-alkyl, or R⁴ is hydrogen and R⁵ is hydroxy-C₁-C₈-alkyl, acyl, -SO₂R⁸ or -CON(R⁶)R⁷, or R⁴ and R⁵ together with the nitrogen atom to which they are attached denote a 5-or 6-membered heterocyclic group;
R⁶ and R⁷ are each independently hydrogen or C₁-C₈-alkyl, or R⁶ and R⁷ together with the nitrogen atom to which they are attached denote a 5- or 6-membered heterocyclic group; and
R⁸ is C₁-C₈-alkyl, C₁-C₈-haloalkyl, or phenyl optionally substituted by C₁-C₈-alkyl.

Terms used in the specification have the following meanings:

"C₁-C₈-alkyl" as used herein denotes straight chain or branched alkyl having 1 to 8 carbon atoms. Preferably, C₁-C₈-alkyl is C₁-C₄-alkyl.

"C₁-C₈-alkylene" as used herein denotes straight chain or branched alkylene that contains 1 to 8 carbon atoms. Preferably, C₁-C₈-alkylene is C₁-C₄-alkylene, especially straight chain butylene.

"C₃-C₁₀-cycloalkyl" as used herein denotes cycloalkyl having 3 to 10 ring carbon atoms, for example a monocyclic group such as a cyclopropyl, cyclobutyl, cyclopentyl, cyclohexyl, cycloheptyl, cyclooctyl, cyclononyl or cyclodecyl, any of which can be substituted by one or more, usually one or two, C₁-C₄-alkyl groups, or a bicyclic group such as bicycloheptyl or bicyclooctyl. Preferably C₃-C₁₀-cycloalkyl is C₃-C₆-cycloalkyl, especially cyclopropyl or cyclobutyl.

"C₁-C₈-alkoxy" as used herein denotes straight chain or branched alkoxy having 1 to 8 carbon atoms. Preferably, C₁-C₈-alkoxy is C₁-C₄-alkoxy.

"C₁-C₈-haloalkyl" as used herein denotes C₁-C₈-alkyl as hereinbefore defined substituted by one or more halogen atoms, preferably one, two or three halogen atoms.

"C₁-C₈-haloalkoxy" as used herein denotes C₁-C₈-alkoxy as hereinbefore defined substituted by one or more halogen atoms, preferably one, two or three halogen atoms.

"Aminocarbonyl" as used herein denotes amino attached through the nitrogen atom to a carbonyl group.

"C₁-C₈-alkylamino" and "di(C₁-C₈-alkyl)amino" as used herein denote amino substituted respectively by one or two C₁-C₈-alkyl groups as hereinbefore defined, which may be the same or different. Preferably C₁-C₈-alkylamino and di(C₁-C₈-alkyl)amino are respectively C₁-C₄-alkylamino and di(C₁-C₄-alkyl)amino.

"C₁-C₈-alkylaminocarbonyl" and "di(C₁-C₈-alkyl)aminocarbonyl" as used herein denote aminocarbonyl as hereinbefore defined substituted respectively on the nitrogen atom by one or two C₁-C₈-alkyl groups as hereinbefore defined, which may be the same or different. Preferably C₁-C₈-alkylaminocarbonyl and di(C₁-C₈-alkyl)aminocarbonyl are respectively C₁-C₄-alkylaminocarbonyl and di(C₁-C₄-alkyl)aminocarbonyl.

"C₁-C₈-alkylthio" as used herein denotes C₁-C₈-alkyl as hereinbefore defined linked to -S-.

"Acyl" as used herein denotes alkylcarbonyl, for example C₁-C₈-alkylcarbonyl where C₁-C₈-alkyl may be one of the C₁-C₈-alkyl groups hereinbefore mentioned, optionally substituted by one or more halogen atoms; cycloalkylcarbonyl, for example C₃-C₈-cycloalkylcarbonyl where C₃-C₈-cycloalkyl may be, for example, cyclopropyl, cyclobutyl, cyclopentyl, cyclohexyl, cycloheptyl or cyclooctyl; 5- or 6- membered heterocyclylcarbonyl having one or two hetero atoms selected from nitrogen, oxygen and sulfur in the ring, such as furylcarbonyl or pyridylcarbonyl; arylcarbonyl, for example C₆-C₁₀-arylcarbonyl such as benzoyl; or aralkylcarbonyl, for example C₆ to C₁₀-aryl-C₁-C₄-alkylcarbonyl such as benzylcarbonyl or phenylethylcarbonyl. Preferably acyl is C₁-C₄-alkylcarbonyl.

"Acyloxy" as used herein denotes alkylcarbonyloxy, for example C₁-C₈-alkylcarbonyloxy where C₁-C₈-alkyl may be one of the C₁-C₈-alkyl groups hereinbefore mentioned, optionally substituted by one or more halogen atoms; cycloalkylcarbonyloxy, for example C₃-C₈-cycloalkylcarbonyloxy where C₃-C₈-cycloalkyl may be, for example, cyclopropyl, cyclobutyl, cyclopentyl, cyclohexyl, cycloheptyl or cyclooctyl; 5- or 6- membered heterocyclylcarbonyloxy having one or two hetero atoms selected from nitrogen, oxygen and sulfur in the ring, such as furylcarbonyloxy or pyridylcarbonyloxy; arylcarbonyloxy, for example C₆-C₁₀-arylcarbonyloxy such as benzoyloxy; or aralkylcarbonyloxy, for example C₆ to C₁₀-aryl-C₁-C₄-alkylcarbonyloxy such as benzylcarbonyloxy or phenylethylcarbonyloxy, or aryloxyalkylcarbonyloxy, for example, C₆-C₁₀-aryloxy-C₁-C₈-alkylcarbonyloxy, any of which is optionally substituted in the aryl moiety by at least one substituent selected from C₁-C₈-alkoxy, halogen, C₁-C₈-alkylcarbonyl, aminosulfonyl, C₁-C₈-alkylaminosulfonyl and di(C₁-C₈-alkyl)aminosulfonyl. Preferably acyloxy is C₁-C₄-alkylcarbonyloxy, or benzoyloxy or phenoxy-C₁-C₄-alkylcarbonyloxy optionally substituted in the benzene ring thereof by at least one substituent selected from C₁-C₄-alkoxy, C₁-C₄-alkylcarbonyl or aminosulfonyl.

"Acylamino" as used herein denotes amino substituted by acyl as hereinbefore defined.

"Halogen" as used herein may be fluorine, chlorine, bromine or iodine; preferably it is fluorine, chlorine or bromine.

"C₁-C₆-alkoxycarbonyl" as used herein denotes C₁-C₈-alkoxy as hereinbefore defined attached through the oxygen atom to a carbonyl group.

"Di-(C₁-C₈-alkyl)aminocarbonylmethoxy" as used herein denotes aminocarbonylmethoxy disubstituted on the amino nitrogen atom by C₁-C₈-alkyl as hereinbefore defined, the two C₁-C₈-alkyl groups being the same or different.

"Optionally substituted" means the group referred to can be substituted at one or more positions by any one or any combination of the radicals listed thereafter.

In Ar, the phenyl group may be substituted, for example by one, two or three, preferably one or two halogen atoms, preferably selected from fluorine and chlorine atoms, or by one or two C₁-C₈-alkyl, cyano or nitro groups, or by C₁-C₈-alkyl and one or two halogen, preferably fluorine or chlorine, atoms. When there is one halogen substituent, it is preferably para to the indicated group X¹ or X². When there are two or three halogen substituents, preferably one is para to the indicated group X¹ or X²and at least one of the others is ortho to the para-halogen substituent.

R³ as substituted phenyl may, for example, be substituted by one, two, three, four or five, preferably by one, two or three, of the abovementioned substituents. R³ may be, for example, phenyl substituted by one, two or three substituents selected from halogen, cyano, hydroxy, nitro, C₁-C₄-alkoxy, C₁-C₄-alkoxycarbonyl, C₁-C₄-haloalkoxy, -CO-NH₂, di(C₁-C₄-alkyl)-aminocarbonylmethoxy, C₁-C₄-alkyl optionally substituted by C₁-C₄-alkoxy, C₁-C₄-haloalkyl, C₁-C₄-alkylcarbonyl, C₁-C₄-alkylthio, -SO₂-NH₂, -SO₂-C₁-C₄-alkyl, di(C₁-C₄-alkyl)amino, C₁-C₄-alkylaminocarbonyl or C₁-C₄-alkyl-carbonylamino. R³ as substituted phenyl is preferably phenyl substituted by one or more substituents selected from cyano, halogen, C₁-C₄-alkyl optionally substituted by C₁-C₄-alkoxy, C₁-C₄-alkylcarbonyl, C₁-C₄-alkoxy, C₁-C₄-alkoxycarbonyl, -CO-NH₂,-SO₂-NH₂, -SO₂-C₁-C₄-alkyl, C₁-C₄-alkyl-aminocarbonyl, di(C₁-C₄-alkyl)-aminocarbonyl-methoxy or C₁-C₄-alkyl-carbonylamino, especially cyanophenyl, particularly meta-cyanophenyl, and disubstituted phenyl where one substituent is C₁-C₄-alkoxy or di(C₁-C₄-alkyl)aminocarbonylmethoxy, preferably ortho to the bond linking R³ to the remainder of the molecule shown in formula la or Ib, and the other, preferably para to the C₁-C₄-alkoxy group, is C₁-C₄-alkoxy, halogen, cyano or C₁-C₄-alkyl. When R³ is C₁-C₄-alkyl substituted by optionally substituted phenoxy, the substituent(s) on phenoxy may be, for example, one, two or three substituents selected from halogen, cyano, C₁-C₈-alkyl, C₁-C₈-alkoxy or C₁-C₈-alkylcarbonyl.

R³ as a heterocyclic group may be, for example, a group having 5 to 11 ring atoms of which one, two, three or four, preferably one or two, are hetero atoms selected from nitrogen, oxygen or sulfur, such as pyrrolyl, furyl, thienyl, imidazolyl, pyrazolyl, oxazolyl, isoxazolyl, thiazolyl, thiadiazolyl, triazolyl, tetrazolyl, pyridyl, pyranyl, pyrazinyl, or a 5-, 6- or 7-membered heterocyclic, ring preferably having one or two oxygen or nitrogen ring atoms, fused to a benzene ring, said heterocyclic group being optionally substituted by substituents including halogen, C₁-C₄-alkyl optionally substituted by C₁-C₄-alkoxy, C₁-C₄-alkoxy, -SO₂-C₁-C₈-alkyl, C₃-C₁₀-cycloalkyl, phenyl, phenyl-C₁-C₄-alkyl and C₂-C₄-alkynyl. Preferably a heterocyclic group having 5 to 11 ring atoms of which one, two, three or four, preferably one or two, are hetero atoms selected from nitrogen, oxygen or sulphur is a heterocyclic group having 5, 6 or 7 ring atoms of which one, two, three or four, are hetero atoms selected from nitrogen, oxygen and sulphur. It is especially preferred that the heterocyclic group is thiadiazolyl, pyrazolyl, tetrazolyl or isoxazolyl, optionally substituted by one or more of C₁-C₄-alkyl, C₁-C₄-alkoxy and C₃-C₆-cycloalkyl.

R² and R³ together with the nitrogen atom to which they are attached as a heterocyclic group may be, for example, a group having a 5- or 6-membered ring of which one, two or three are heteroatoms, optionally fused to a benzene ring, such as thiadiazolyl, pyrazolyl, tetrazolyl, piperidinyl, piperazinyl, morpholino, or benzopiperidinyl, optionally substituted by one or more substituents including C₁-C₈-alkyl, C₁-C₈-alkoxy, C₃-C₁₀-cycloalkyl and halogen.

R¹ as optionally substituted C₁-C₈-alkyl is preferably optionally substituted C₁-C₄-alkyl, especially C₁-C₄-alkyl or substituted methyl or ethyl. When R¹ is substituted by a cyclic organic group, the latter may be a carbocyclic or heterocyclic group, for example a C₃-C₁₅-carbocyclic group or a 5- to 7-membered heterocyclic group having one or more, preferably one, two or three, ring hetero atoms selected from nitrogen, oxygen and sulfur. The C₃-C₁₅-carbocyclic group may be, for example, a cycloaliphatic group having 3 to 8 carbon atoms, preferably a C₅-C₆-cycloalkyl such as cyclopentyl, methylcyclopentyl or cyclohexyl. The C₃-C₁₅-carbocyclic group may alternatively be, for example, a C₆-C₁₅ aromatic group, such as phenyl, which is unsubstituted or substituted by C₁-C₈-alkyl, C₁-C₈-alkoxy, halogen, cyano, -CON(R⁴)R⁵, -SO₂N(R⁴)R⁵ or C₁-C₈-alkylsulfonylamino where R⁴ and R⁵ are as hereinbefore defined. The heterocyclic group may have one nitrogen, oxygen or sulfur atom in the ring or it may have two nitrogens, or one oxygen and one or two nitrogens, or one sulfur and one or two nitrogens in the ring. The heterocyclic group is preferably a heterocyclic aromatic group, especially a 5-or 6- membered heterocyclic group such as furyl, imidazolyl, thiazolyl or pyridyl. Preferred embodiments include those in which R¹ is hydrogen or C₁-C₄-alkyl substituted by hydroxy or C₁-C₄-alkoxy.

Throughout this specification and in the claims that follow, unless the context requires otherwise, the word "comprise", or variations such as "comprises" or "comprising", will be understood to imply the inclusion of a stated integer or step or group of integers or steps but not the exclusion of any other integer or step or group of integers or steps.

Preferred compounds of the present invention include those that are
(i) compounds of formula Ia in free or salt form, wherein
   Ar is phenyl substituted by halo;
   X¹ is -S-, -S(=O)- or -S(=O)₂-;
   m is 2;
   R¹ is C₁-C₈-alkyl optionally substituted by hydroxy or C₁-C₈-alkoxy;
   Y is oxygen;
   R² is hydrogen; and
   R³ is a heterocyclic group having 5 to 11 ring atoms of which 1 to 4 are hetero atoms;
   or
(ii) compounds of formula Ib in free or salt form, wherein
   Ar is phenyl substituted by halo;
   X² is -O-, -C(=O)- or -CH₂-;
   m is 1 or 2;
   Q has the formula 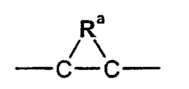 where R^{a} is C₁-C₈-alkylene,
   or Q is -C(R^{b})(R^{c})- where R^{b} and R^{c} are independently C₁-C₈-alkyl
   or R^{b} and R^{c} together form a C₃-C₁₀-cycloalkyl;
   R² is hydrogen; and
   R³ is a heterocyclic group having 5 to 11 ring atoms of which 1 to 4 are hetero atoms.

Especially preferred compounds of the present invention include those that are
(i) compounds of formula Ia in free or salt form, wherein
   Ar is phenyl substituted by halo, preferably chloro;
   X¹ is -S-, -S(=O)- or -S(=O)₂-;
   m is 2;
   R¹ is C₁-C₄-alkyl optionally substituted by hydroxy or C₁-C₄-alkoxy;
   Y is oxygen;
   R² is hydrogen; and
   R³ is a heterocyclic group having 5, 6 or 7 ring atoms of which one, two, three or four, are hetero atoms selected from nitrogen, oxygen and sulphur, said heterocyclic group being optionally substituted by C₁-C₄-alky, C₁-C₄-alkoxy or C₃-C₆-cycloalkyl; or
(ii) compounds of formula Ib in free or salt form, wherein
   Ar is phenyl substituted by halo, preferably chloro;
   X² is -O-, -C(=O)- or -CH₂-;
   m is 1 or 2;
   Q has the formula 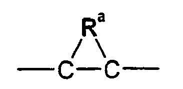 where R^{a} is C₁-C₈-alkylene,
   or Q is -C(R^{b})(R^{c})- where R^{b} and R^{c} are independently C₁-C₄-alkyl
   or R^{b} and R^{c} together form a C₃-C₆-cycloalkyl;
   R² is hydrogen; and
   R³ is a heterocyclic group having 5, 6 or 7 ring atoms of which one, two, three or four, are hetero atoms selected from nitrogen, oxygen and sulphur, said heterocyclic group
   being optionally substituted by C₁-C₄-alkyl or C₃-C₆-cycloalkyl.

The compounds represented by formula Ia or Ib are capable of forming acid addition salts, particularly pharmaceutically acceptable acid addition salts. Pharmaceutically acceptable acid addition salts of the compounds of formula Ia or Ib include those of inorganic acids, for example, hydrohalic acids such as hydrofluoric acid, hydrochloric acid, hydrobromic acid or hydroiodic acid, nitric acid, sulfuric acid, phosphoric acid; and organic acids, for example aliphatic monocarboxylic acids such as formic acid, acetic acid, trifluoroacetic acid, propionic acid and butyric acid, aliphatic hydroxy acids such as lactic acid, citric acid, tartaric acid or malic acid, dicarboxylic acids such as maleic acid or succinic acid, aromatic carboxylic acids such as benzoic acid, p-chlorobenzoic acid, diphenylacetic acid or triphenylacetic acid, aromatic hydroxy acids such as o-hydroxybenzoic acid, p-hydroxybenzoic acid, 1-hydroxynaphthalene-2-carboxylic acid or 3-hydroxynaphthalene-2-carboxylic acid, and sulfonic acids such as methanesulfonic acid or benzenesulfonic acid. These salts may be prepared from compounds of formula Ia or Ib by known salt-forming procedures.

Compounds of formula Ia or Ib which contain acidic, e.g. carboxyl, groups, are also capable of forming salts with bases, in particular pharmaceutically acceptable bases such as those well known in the art; suitable such salts include metal salts, particularly alkali metal or alkaline earth metal salts such as sodium, potassium, magnesium or calcium salts, or salts with ammonia or pharmaceutically acceptable organic amines or heterocyclic bases such as ethanolamines, benzylamines or pyridine. These salts may be prepared from compounds of formula Ia or Ib by known salt-forming procedures.

When R¹ is other than hydrogen, the carbon atom to which R¹ is attached in formula Ia is asymmetric, in which case the compounds exist in individual optically active isomeric forms or as mixtures thereof, e.g. as racemic or diastereomeric mixtures. When X¹ is -S(=O), the sulfur atom is asymmetric, so again the compounds exist in individual optically active isomeric forms or as mixtures thereof, e.g. as racemic or diastereomeric mixtures. In both cases the invention embraces both individual optically active R and S isomers as well as mixtures, e.g. racemic or diastereomeric mixtures, thereof.

When Q has the formula 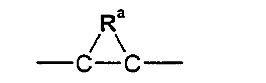 where R^{a} is C₁-C₈-alkylene the compounds can exist in individual optically active isomeric forms or as mixtures thereof, e.g. as racemic or diastereomeric mixtures. When X² is -S(=O), the sulfur atom is asymmetric, so again the compounds exist in individual optically active isomeric forms or as mixtures thereof, e.g. as racemic or diastereomeric mixtures. In both cases the invention embraces both individual optically active R and S isomers as well as mixtures, e.g. racemic or diastereomeric mixtures, thereof.

Specific especially preferred compounds of the invention are those described hereinafter in the Examples.

The invention also provides a process for the preparation of compounds of formulae Ia or Ib which comprises
(i) (A) for the preparation of compounds of formula Ia where R² is hydrogen, reacting a compound of formula IIa 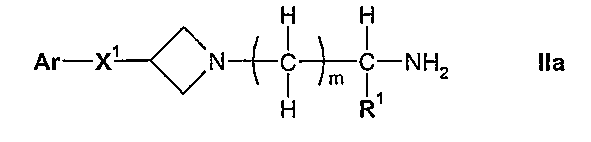 or a protected form thereof, where Ar, X¹, m and R¹ are as hereinbefore defined, with a compound of formula III

   Y=C=N-**R³** **III**

   where Y and R³ are as hereinbefore defined; or
   (B) for the preparation of compounds of formula Ia where Y is oxygen, reacting a compound of formula IIa where Ar, X¹, m and R¹ are as hereinbefore defined, with a compound of formula IV 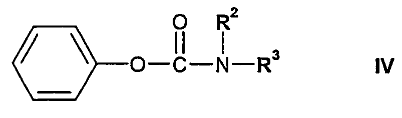 where R²and R³ are as hereinbefore defined; or
   (C) for the preparation of compounds of formula Ia where X¹ is -S(=O)₂-, oxidising a compound of formula Ia in protected form where X¹ is -S- and Ar, m, R¹, Y, R² and R³ are hereinbefore defined;
   (D) for the preparation of compounds of formula Ib, reacting a compound of formula IIb 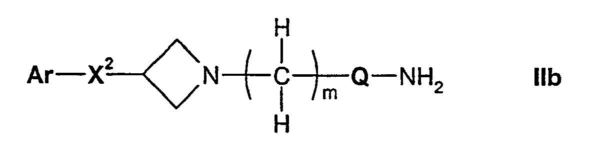 where Ar, X², m and Q are as hereinbefore defined, with a compound of formula IV where R² and R³ are as hereinbefore defined;
   (E) for the preparation of compounds of formula Ib where R² is hydrogen, reacting a compound of formula IIb where Ar, X², m and Q are as hereinbefore defined, with a compound of formula V

      O=C=N-**R³** **V**

      where R³ is as hereinbefore defined; or
   (F) for the preparation of compounds of formula Ib where X² is -S(=O)₂-, oxidising a compound of formula Ib in protected form where X² is -S- and Ar, m, Q, R² and R³ are as hereinbefore defined; and
(ii) recovering the product in free or salt form.

Process variant (A) may be effected using known procedures for reaction of amines with isocyanates or analogously e.g. as hereinafter described in the Examples. The reaction is conveniently carried out in an organic solvent, for example a halohydrocarbon such as dichloromethane (DCM) or an ether such as dioxane. The reaction temperature may be e.g. from 0 °C to 100 °C, conveniently ambient temperature.

Process variant (B) may be effected using known procedures for reaction of amines with carbamic acid phenyl esters or analogously e.g. as hereinafter described in the Examples. The reaction is conveniently carried out in an organic solvent such as dimethyl sulfoxide (DMSO). The reaction temperature may be e.g. from 20 to 100 °C, conveniently ambient temperature.

Process variant (C) may be effected using known procedures for oxidising sulfanyl groups to form sulfonyl groups or analogously e.g. as hereinafter described in the Examples. The oxidising agent used is preferably a perbenzoic acid, especially meta chloro-per-benzoic acid. The reaction is conveniently carried out in an organic solvent such as dichloromethane (DCM). The reaction temperature may be e.g. from 5 to 25 °C, preferably about 15 °C.

Process variant (D) may be effected using known procedures for reaction of amines with carbamic acid phenyl esters or analogously e.g. as hereinafter described in the Examples. The reaction is conveniently carried out in an organic solvent such as dimethyl sulfoxide (DMSO). The reaction temperature may be e.g. from 20 to 100 °C, conveniently ambient temperature.

Process variant (E) may be effected using known procedures for reaction of amines with isocyanates or analogously e.g. as hereinafter described in the Examples. The reaction is conveniently carried out in an organic solvent, for example a halohydrocarbon such as dichloromethane (DCM) or an ether such as dioxane. The reaction temperature may be e.g. from 0 °C to 100 °C, conveniently ambient temperature.

Process variant (F) may be effected using known procedures for oxidising sulfanyl groups to form sulfonyl groups or analogously e.g. as hereinafter described in the Examples. The oxidising agent used is preferably a perbenzoic acid, especially meta chloro-per-benzoic acid. The reaction is conveniently carried out in an organic solvent such as dichloromethane (DCM). The reaction temperature may be e.g. from 5 to 25°C, preferably about 15°C.

Compounds of formula IIa may be prepared by reacting a compound of formula VI 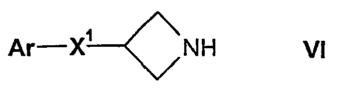 where Ar and X¹ are as hereinbefore defined, with a compound of formula VII 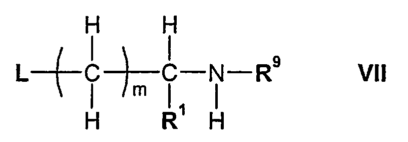 where L is a halogen, preferably bromine or iodo, and R¹ and m are as hereinbefore defined, with the proviso that when R¹ contains a reactive functional group such as a hydroxy group, the reactive group may be in protected form, for example a hydroxy group protected as a tert-butoxy group, and R⁹ is hydrogen or an amine-protective group, for example a tert-butoxycarbonyl group, and, where R⁹ is a protective group, replacing R⁹ in the product by hydrogen, and, where R¹ in the product contains a protected functional group, replacing the protecting group with hydrogen, or analogously e.g. as hereinafter described in the Examples. When R⁹ is hydrogen, reaction between a compound of formula VII and a salt of a compound of formula VI may be effected by the procedures described in US patent US 4559349. When R⁹ is a protective group, reaction between compounds of formulae VI and VII may be effected using known methods, for example in the presence of a tertiary organic base such as triethylamine or 1,8-diaza-bicyclo-[5.4.0]undec-7-ene (DBU), conveniently in an inert organic solvent, for example a polar solvent such as dimethylformamide, the reaction temperature suitably being from 0 to 40°C, preferably ambient temperature. Replacement of a protective group R⁹ by hydrogen may be effected using known procedures; for example, where R⁹ is tert-butoxycarbonyl, by treatment with a carboxylic acid such as trifluoroacetic acid. Replacement of a protecting group in R¹ may be affected using known procedures, for example, when R¹ contains a hydroxy group protected as an ether group, such as tert-butoxy, by treatment with HBr in a carboxylic acid such as acetic acid; when R⁹ is a protective group, this treatment also replaces R⁹ by hydrogen.

Compounds of formula IIb where X² is -O- or -CH₂- may be prepared by reacting a compound of formula VIII 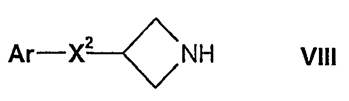 where Ar and X² are as hereinbefore defined, with a compound of formula IX 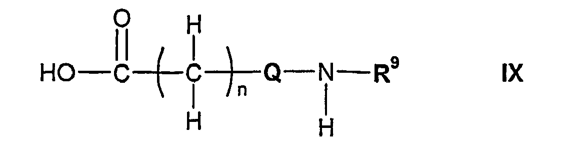 where Q are as hereinbefore defined, n is 0,1, 2 or 3 and R⁹ is hydrogen or an amine-protective group, for example a tert-butoxycarbonyl group, and reducing the resulting compound to convert the carbonyl group to -C(H)-, where R⁹ is a protective group, replacing R⁹ in the product by hydrogen, or analogously e.g. as hereinafter described in the Examples. When R⁹ is hydrogen, reaction between a compound of formula IX and a salt of a compound of formula VIII may be effected by the procedures described in US patent 4559349. When R⁹ is a protective group, reaction between compounds of formulae VIII and IX may be effected using known methods, for example in the presence of a tertiary organic base such as triethylamine or 1,8-diaza-bicyclo[5.4.0]undec-7-ene (DBU), conveniently in an inert organic solvent, for example a polar solvent such as dimethyl-formamide, the reaction temperature suitably being from 0 to 40°C, preferably ambient temperature. The reduction can be carried out using a reducing agent such as LiAlH₄ in an organic solvent such as tetrahydrofuran (THF). Replacement of a protective group R⁹ by hydrogen may be effected using known procedures; for example, where R⁹ is tert-butoxy-carbonyl, by treatment with a carboxylic acid such as trifluoroacetic acid. Replacement of a protecting group in R¹ may be affected using known procedures, for example, when R¹ contains a hydroxy group protected as an ether group, such as tert-butoxy, by treatment with HBr in a carboxylic acid such as acetic acid; when R⁹ is a protective group, this treatment also replaces R⁹ by hydrogen.

Compounds of formula IIb where X² is -C(=O)-, -S-, -S(=O)- or -S(=O)₂- may be prepared by reacting a compound of formula VIII, where Ar and X² are as hereinbefore defined, with a compound of formula X 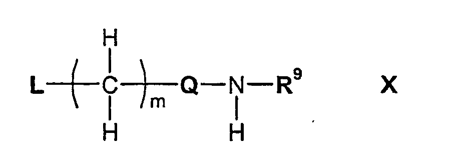 where L is a halogen, preferably bromine or iodo, m and Q are as hereinbefore defined, and R⁹ is hydrogen or an amine-protective group, for example a tert-butoxycarbonyl group, and, where R⁹ is a protective group, replacing R⁹ in the product by hydrogen, or analogously e.g. as hereinafter described in the Examples. When R⁹ is hydrogen, reaction between a compound of formula X and a salt of a compound of formula VIII may be effected by the procedures described in US patent 4559349. When R⁹ is a protective group, reaction between compounds of formulae VIII and X may be effected using known methods, for example in the presence of a tertiary organic base such as triethylamine or 1,8-diazabicyclo-[5.4.0]-undec-7-ene (DBU), conveniently in an inert organic solvent, for example a polar solvent such as dimethylformamide, the reaction temperature suitably being from 0 to 40°C, preferably ambient temperature. Replacement of a protective group R⁹ by hydrogen may be effected using known procedures; for example, where R⁹ is tert-butoxy-carbonyl, by treatment with a carboxylic acid such as trifluoroacetic acid. Replacement of a protecting group in R¹ may be affected using known procedures, for example, when R¹ contains a hydroxy group protected as an ether group, such as tert-butoxy, by treatment with HBr in a carboxylic acid such as acetic acid; when R⁹ is a protective group, this treatment also replaces R⁹ by hydrogen.

Compounds of formula III are commercially available or may be prepared by known methods.

Compounds of formula IV or V are known or may be prepared by known procedures or analogously e.g. as hereinafter described in the Examples.

Compounds of formula VI where X¹ is -S- may be prepared by reacting a compound of formula XI

Ar-SH **XI**

where Ar is as hereinbefore defined, with a compound of formula XII 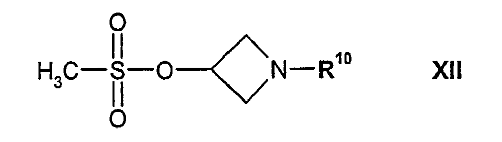 in the presence of sodium hydride where R¹⁰ is a protecting group, and replacing R¹⁰ in the product by hydrogen, or analogously e.g. as hereinafter described in the Examples. The reaction may be carried out in an inert organic solvent such as dimethylformamide (DMF). Suitable reaction temperatures may be from 20°C to 150°C, conveniently from 50 to 70°C. The replacement of R¹⁰ by hydrogen may be effected using known procedures or analogously e.g. as hereinafter described in the Examples.

Compounds of formula VI where X¹ is -S(=O)- may be prepared by reacting the corresponding aryl-sulfanyl-azetidine, preferably a protected form thereof, with an oxidising agent such as a per-benzoic acid, or analogously e.g. as hereinafter described in the Examples. The reaction is conveniently carried out in an organic solvent such as dichloromethane (DCM). The reaction temperature may be e.g. from 5 to 25°C, preferably about 15 °C.

Compounds of formula VI where X¹ is -S(=O)₂- may be prepared by reacting the corresponding aryl-sulfanyl-azetidine, preferably a protected form thereof, with an oxidising agent such as a per-benzoic acid, or analogously e.g. as hereinafter described in the Examples. The reaction is conveniently carried out in an organic solvent such as dichloromethane (DCM). The reaction temperature may be e.g. from 5 to 25 °C, preferably about 15 °C.

Compounds of formula VII are known or may be prepared by known procedures or analogously e.g. as hereinafter described in the Examples.

Compounds of formula VIII where X² is -C(=O)- may be prepared by reacting a compound of formula XIII or XIV 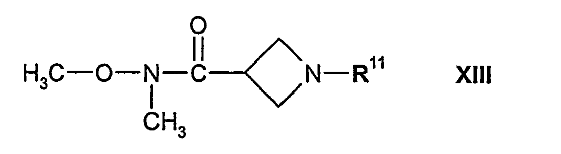 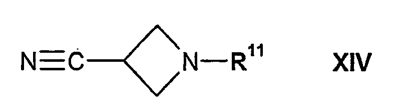 with a compound of formula XV

Ar=MgBr **XV**

where Ar and R¹¹ are as hereinbefore defined, and replacing R¹¹ in the product by hydrogen, or analogously e.g. as hereinafter described in the Examples. Reaction of compounds of formulae XIII/XIV and XV may be effected in an inert organic solvent, e.g. an ether such as THF and/or diethyl ether; suitable reaction temperatures may be from -10°C to 10°C, conveniently from -5 to 5°C. Replacement of R¹¹ in the product by hydrogen may be effected using known procedures or analogously e.g. as hereinafter described in the Examples.

Compounds of formula VIII where X² is -O- may be prepared by reacting a compound of formula XVI 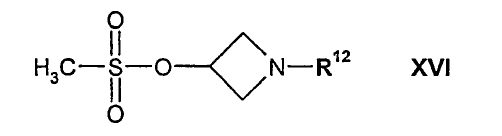 with a compound of formula Ar-OH in the presence of sodium hydride, where Ar is as hereinbefore defined and R¹² is a protecting group, and replacing R¹² in the product by hydrogen, or analogously e.g. as hereinafter described in the Examples. The reaction may be carried out in an inert organic solvent such as DMF. Suitable reaction temperatures may be from 20°C to 150°C, conveniently from 50 to 70°C. The replacement of R¹² by hydrogen may be effected using known procedures or analogously e.g. as hereinafter described in the Examples.

Compounds of formula VIII where X² is -CH₂- are novel and may be prepared by reduction of compounds of formula VIII where X² is -C(=O) -, for example using known reduction procedures or analogously e.g. as hereinafter described in the Examples. This preferably involves of reduction to the corresponding alcohol, conversion to the iodine and then reduction.

Compounds of formula VIII where X² is -S- may be prepared by reacting a compound of formula XVII

Ar-SH **XVII**

where Ar is as hereinbefore defined, with a compound of formula XVIII 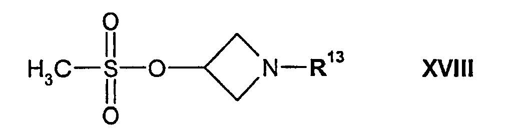 in the presence of sodium hydride where R¹³ is a protecting group, and replacing R¹³ in the product by hydrogen, or analogously e.g. as hereinafter described in the Examples. The reaction may be carried out in an inert organic solvent such as dimethylformamide (DMF). Suitable reaction temperatures may be from 20°C to 150°C, conveniently from 50 to 70°C. The replacement of R¹³ by hydrogen may be effected using known procedures or analogously e.g. as hereinafter described in the Examples.

Compounds of formula VIII where X² is -S(=O)- may be prepared by reacting the corresponding aryl-sulfanyl-azetidine, preferably a protected form thereof, with an oxidising agent such as a per-benzoic acid, or analogously e.g. as hereinafter described in the Examples. The reaction is conveniently carried out in an organic solvent such as dichloromethane (DCM). The reaction temperature may be e.g. from 5 to 25°C, preferably about 15°C.

Compounds of formula VIII where X² is -S(=O)₂- may be prepared by reacting the corresponding aryl-sulfanyl-azetidine, preferably a protected form thereof, with an oxidising agent such as a per-benzoic acid, or analogously e.g. as hereinafter described in the Examples.

The reaction is conveniently carried out in an organic solvent such as dichloromethane (DCM). The reaction temperature may be e.g. from 5 to 25 °C, preferably about 15 °C.

Compounds of formulae IX , X, XI or XII are known or may be prepared by known procedures or analogously e.g. as hereinafter described in the Examples.

Compounds of formula XIII may be prepared by reacting a compound of formula XIX 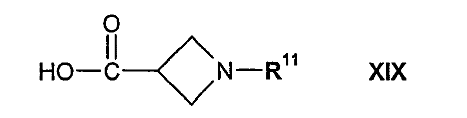 where R¹¹ is as hereinbefore defined, with O,N-dimethylhydroxylamine hydrochloride in the presence of a peptide coupling agent such as di-imidazol-1-yl-methanone, conveniently in an inert organic solvent such as THF, suitably at reflux temperature, or analogously e.g. as hereinafter described in the Examples.

Compounds of formula XIV, XV, XVI, XVII, XVIII or XIX are known or may be prepared by known procedures or analogously e.g. as hereinafter described in the Examples.

Where reference is made herein to protected functional groups or to protecting groups, the protecting groups may be chosen in accordance with the nature of the functional group, for example as described in Protective Groups in Organic Synthesis, T. W. Greene, P.G.M. Wuts, John Wiley & Sons Inc, Third Edition, 1999, which reference also describes procedures suitable for replacement of the protecting groups by hydrogen.

Compounds of formula Ia or Ib in free form may be converted into salt form, and vice versa, in a conventional manner. The compounds in free or salt form can be obtained in the form of hydrates or solvates containing a solvent used for crystallization. Compounds of formula Ia or Ib can be recovered from reaction mixtures and purified in a conventional manner. Isomers, such as enantiomers, may be obtained in a conventional manner, e.g. by fractional crystallization or asymmetric synthesis from correspondingly asymmetrically substituted, e.g. optically active, starting materials.

Compounds of formula Ia or Ib in free or pharmaceutically acceptable salt form, hereinafter referred to alternatively as agents of the invention, are useful as pharmaceuticals. Accordingly the invention also provides a compound of formula Ia or Ib in free or pharmaceutically acceptable salt form for use as a pharmaceutical. The agents of the invention act as CCR-3 receptor antagonists, thereby inhibiting the infiltration and activation of inflammatory cells, particularly eosinophils, and inhibiting allergic response. The inhibitory properties of agents of the invention can be demonstrated in the following assay:

In this assay the effect of agents of the invention on the binding of human eotaxin to human CCR-3 is determined. Recombinant cells expressing human CCR-3 are captured by wheatgerm agglutinin (WGA) polyvinyltoluidene (PVT) SPA beads (available from Amersham), through a specific interaction between the WGA and carbohydrate residues of glycoproteins on the surface of the cells. [¹²⁵I]-human eotaxin (available from Amersham) binds specifically to CCR-3 receptors bringing the [¹²⁵I]-human eotaxin in close proximity to the SPA beads. Emitted â-particles from the [¹²⁵I]-human eotaxin excite, by its proximity, the fluorophore in the beads and produce light. Free [¹²⁵I]-human eotaxin in solution is not in close proximity to the scintillant and hence does not produce light. The scintillation count is therefore a measure of the extent to which the test compound inhibits binding of the eotaxin to the CCR-3.

Preparation of Assay Buffer: 5.96 g HEPES and 7.0 g sodium chloride are dissolved in distilled water and 1M aqueous CaCl₂ (1 ml) and 1M aqueous MgCl₂ (5 ml) are added. The pH is adjusted to 7.6 with NaOH and the solution made to a final volume of 1 L using distilled water. 5 g bovine serum albumin and 0.1 g sodium azide are then dissolved in the solution and the resulting buffer stored at 4°C. A Complete^{™} protease inhibitor cocktail tablet (available from Boehringer) is added per 50 ml of the buffer on the day of use.

Preparation of Homogenisation Buffer: Tris-base (2.42 g) is dissolved in distilled water, the pH of the solution is adjusted to 7.6 with hydrochloric acid and the solution is diluted with distilled water to a final volume of 11. The resulting buffer is stored at 4°C. A Complete^{™} protease inhibitor cocktail tablet is added per 50 ml of the buffer on the day of use.

Preparation of membranes: Confluent rat basophil leukaemia (RBL-2H3) cells stably expressing CCR3 are removed from tissue culture flasks using enzyme-free cell dissociation buffer and resuspended in phosphate-buffered saline. The cells are centrifuged (800 g, 5 minutes), the pellet resuspended in ice-cold homogenisation buffer using 1 ml homogenisation buffer per gram of cells and incubated on ice for 30 minutes. The cells are homogenised on ice with 10 strokes in a glass mortar and pestle. The homogenate is centrifuged (800 g, 5 minutes, 4°C), the supernatant further centrifuged (48,000 g, 30 minutes, 4°C) and the pellet redissolved in Homogenisation Buffer containing 10% (v/v) glycerol. The protein content of the membrane preparation is estimated by the method of Bradford (Anal. Biochem. (1976) 72:248) and aliquots are snap frozen and stored at -80°C.

The assay is performed in a final volume of 250 µl per well of an Optiplate^{™} microplate (ex Canberra Packard). To selected wells of the microplate are added 50 µl of solutions of a test compound in Assay Buffer containing 5 % DMSO (concentrations from 0.01 nM to 10 µM). To determine total binding, 50 µl of the Assay Buffer containing 5 % DMSO is added to other selected wells. To determine non-specific binding, 50 µl of 100 nM human eotaxin (ex R&D Systems) in Assay Buffer containing 5 % DMSO is added to further selected wells. To all wells are added 50 µl [¹²⁵I]-Human eotaxin (ex Amersham) in Assay Buffer containing 5 % DMSO at a concentration of 250 pM (to give a final concentration of 50 pM per well), 50 µl of WGA-PVT SPA beads in Assay Buffer (to give a final concentration of 1.0 mg beads per well) and 100 µl of the membrane preparation at a concentration of 100 µg protein in Assay Buffer (to give a final concentration of 10 µg protein per well). The plate is then incubated for 4 hours at room temperature. The plate is sealed using TopSeal-S^{™} sealing tape (ex Canberra Packard) according to the manufacturer's instructions. The resulting scintillations are counted using a Canberra Packard TopCount^{™} scintillation counter, each well being counted for 1 minute. The concentration of test compound at which 50% inhibition occurs (IC₅₀) is determined from concentration-inhibition curves in a conventional manner.

The compounds of the Examples hereinbelow generally have IC₅₀ values below 1 µM in the above assay. For instance, the compounds of Examples 1, 19, 25, 31, 73, 94 and 131 have IC₅₀ values of 0.278, 0.002, 0.820, 0.117, 0.136, 0.124 and 0.096 µM respectively.

Most of the compounds of the Examples exhibit selectivity for inhibition of CCR-3 binding relative to inhibition of binding of the alpha-1 adrenergic receptor. Some of the compounds e.g. those of Example 19 and 31, are also histamine H1 antagonists.

The inhibitory properties of agents of the invention on binding of the alpha-1 adrenergic receptor can be determined in the following assay:

Cerebral cortices from male Sprague-Dawley rats (175-200 g) are dissected and homogenised in 10 volumes of ice cold 0.32 M sucrose (containing 1 mM MgCl₂ dihydrate and 1mM K₂HPO₄) with a glass/Teflon homogeniser. The membranes are centrifuged at 1000 x g for 15 miN, the pellet discarded and the centrifugation repeated. The supernatants are pooled and centrifuged at 18,000 x g for 15 minutes. The pellet is osmotically shocked in 10 volumes of water and kept on ice for 30 minutes. The suspension is centrifuged at 39,000 x g for 20 minutes, resuspended in Krebs-Henseleit buffer pH 7.4 (1.17 mM MgSO₄ anhydrous, 4.69 mM KCl, 0.7 mM K₂HPO₄ anhydrous, 0.11 M NaCl, 11 mM D-glucose and 25 mM NaHCO₃) containing 20 mM Tris, and kept for 2 days at -20°C. The membranes are then thawed at 20-23°C, washed three times with Krebs-Henseleit buffer by centrifugation at 18,000 x g for 15 minutes, left overnight at 4°C and washed again three times. The final pellet is resuspended with a glass/Teflon homogeniser in 125 ml/100 membranes in the same buffer. A sample is taken to determine the protein concentration (using the Bradford Assay with gamma globulin as the standard) and the remainder aliquoted and stored at -80°C.

The resulting membranes are subjected to a radioligand binding assay. The assay is conducted in triplicate using 96 well plates containing [¹²⁵I]-HEAT (Amersham) (40 pM, K_{d}: 58.9 ± 18.7 pM), unlabelled test compound and membrane (57.1 µg/ml) to yield a final volume of 250 µl (assay buffer containing 50 mM Tris-base and 0.9% (w/v) NaCl, pH 7.4). The plates are incubated at 37°C for 60 minutes, after which rapid vacuum filtration over Whatman^{™} GF/C 96 well filter plates is carried out. Each plate is then washed three times with 10ml of ice cold assay buffer using a Brandel Cell harvester (Gaithersburg, MD). Following drying of the plates for 3 h. at 50°C, 40 µl of Microscint 20 is added to each well, the plates incubated at room temperature for a further 20 minutes and the retained radioactivity quantified in a Packard TopCount NXT^{™} scintillation counter.

Stock solutions of test compounds are dissolved initially in 100 % DMSO and diluted with assay buffer to the required concentrations to yield 1 % (v/v) DMSO. The concentration of test compound at which 50% inhibition occurs (IC₅₀) is determined from concentration-inhibition curves in a conventional manner.

Having regard to their inhibition of binding of CCR-3, agents of the invention are useful in the treatment of conditions mediated by CCR-3, particularly inflammatory or allergic conditions. Treatment in accordance with the invention may be symptomatic or prophylactic.

Accordingly, agents of the invention are useful in the treatment of inflammatory or obstructive airways diseases, resulting, for example, in reduction of tissue damage, bronchial hyperreactivity, remodelling or disease progression. Inflammatory or obstructive airways diseases to which the present invention is applicable include asthma of whatever type or genesis including both intrinsic (non-allergic) asthma and extrinsic (allergic) asthma, mild asthma, moderate asthma, severe asthma, bronchitic asthma, exercise-induced asthma, occupational asthma and asthma induced following bacterial or viral infection. Treatment of asthma is also to be understood as embracing treatment of subjects, e.g. of less than 4 or 5 years of age, exhibiting wheezing symptoms and diagnosed or diagnosable as "wheezy infants", an established patient category of major medical concern and now often identified as incipient or early-phase asthmatics. (For convenience this particular asthmatic condition is referred to as "wheezy-infant syndrome".)

Prophylactic efficacy in the treatment of asthma will be evidenced by reduced frequency or severity of symptomatic attack, e.g. of acute asthmatic or bronchoconstrictor attack, improvement in lung function or improved airways hyperreactivity. It may further be evidenced by reduced requirement for other, symptomatic therapy, i.e. therapy for or intended to restrict or abort symptomatic attack when it occurs, for example anti-inflammatory (e.g. corticosteroid) or bronchodilatory. Prophylactic benefit in asthma may in particular be apparent in subjects prone to "morning dipping". "Morning dipping" is a recognised asthmatic syndrome, common to a substantial percentage of asthmatics and characterised by asthma attack, e.g. between the hours of about 4 to 6 am, i.e. at a time normally substantially distant form any previously administered symptomatic asthma therapy.

Other inflammatory or obstructive airways diseases and conditions to which the present invention is applicable include acute lung injury (ALI), acute/adult respiratory distress syndrome (ARDS), chronic obstructive pulmonary, airways or lung disease (COPD, COAD or COLD), including chronic bronchitis or dyspnea associated therewith, emphysema, as well as exacerbation of airways hyperreactivity consequent to other drug therapy, in particular other inhaled drug therapy. The invention is also applicable to the treatment of bronchitis of whatever type or genesis including, e.g., acute, arachidic, catarrhal, croupus, chronic or phthinoid bronchitis. Further inflammatory or obstructive airways diseases to which the present invention is applicable include pneumoconiosis (an inflammatory, commonly occupational, disease of the lungs, frequently accompanied by airways obstruction, whether chronic or acute, and occasioned by repeated inhalation of dusts) of whatever type or genesis, including, for example, aluminosis, anthracosis, asbestosis, chalicosis, ptilosis, siderosis, silicosis, tabacosis and byssinosis.

Having regard to their anti-inflammatory activity, in particular in relation to inhibition of eosinophil activation, agents of the invention are also useful in the treatment of eosinophil related disorders, e.g. eosinophilia, in particular eosinophil related disorders of the airways (e.g. involving morbid eosinophilic infiltration of pulmonary tissues) including hyper-eosinophilia as it effects the airways and/or lungs as well as, for example, eosinophil-related disorders of the airways consequential or concomitant to Löffler's syndrome, eosinophilic pneumonia, parasitic (in particular metazoan) infestation (including tropical eosinophilia), bronchopulmonary aspergillosis, polyarteritis nodosa (including Churg-Strauss syndrome), eosinophilic granuloma and eosinophil-related disorders affecting the airways occasioned by drug-reaction.

Agents of the invention are also useful in the treatment of inflammatory or allergic conditions of the skin, for example psoriasis, contact dermatitis, atopic dermatitis, alopecia areata, erythema multiforma, dermatitis herpetiformis, scleroderma, vitiligo, hypersensitivity angiitis, urticaria, bullous pemphigoid, lupus erythematosus, pemphisus, epidermolysis bullosa acquisita, and other inflammatory or allergic conditions of the skin.

Agents of the invention may also be used for the treatment of other diseases or conditions, in particular diseases or conditions having an inflammatory component, for example, treatment of diseases and conditions of the eye such as conjunctivitis, keratoconjunctivitis sicca, and vernal conjunctivitis, diseases affecting the nose including allergic rhinitis, e.g. atrophic, chronic, or seasonal rhinitis, inflammatory conditions of the gastrointestinal tract, for example inflammatory bowel disease such as ulcerative colitis and Crohn's disease, diseases of the bone and joints including rheumatoid arthritis, psoriatic arthritis, ankylosing spondylitis and systemic sclerosis, and other diseases such as cyctic fibrosis, pulmonary hypertension, atherosclerosis, multiple sclerosis, diabetes (type I), myasthenia gravis, hyper IgE syndrome and acute and chronic allograft rejection, e.g. following transplantation of heart, kidney, liver, lung or bone marrow.

The effectiveness of an agent of the invention in inhibiting inflammatory conditions, for example in inflammatory airways diseases, may be demonstrated in an animal model, e.g. a mouse or rat model, of airways inflammation or other inflammatory conditions, for example as described by Szarka et al, J. Immunol. Methods (1997) 202:49-57; Renzi et al, Am. Rev. Respir. Dis. (1993) 148:932-939; Tsuyuki et al., J. Clin. Invest. (1995) 96:2924-2931; and Cernadas et al (1999) Am. J. Respir. Cell Mol. Biol. 20:1-8.

The agents of the invention are also useful as co-therapeutic agents for use in combination with other drug substances such as anti-inflammatory, bronchodilatory, antihistamine or antitussive drug substances, particularly in the treatment of obstructive or inflammatory airways diseases such as those mentioned hereinbefore, for example as potentiators of therapeutic activity of such drugs or as a means of reducing required dosaging or potential side effects of such drugs. An agent of the invention may be mixed with the other drug substance in a fixed pharmaceutical composition or it may be administered separately, before, simultaneously with or after the other drug substance.

Such anti-inflammatory drugs include steroids, in particular gluco-corticosteroids such as budesonide, beclamethasone, fluticasone, ciclesonide or mometasone, or steroids described in WO 02/88167, WO 02/12266, WO 02/100879, WO 04/039827 or WO 02/00679, especially those of Examples 3, 11, 14, 17, 19, 26, 34, 37, 39, 51, 60, 67, 72, 73, 90, 99 and 101; LTB4 antagonists such as those described in US 5451700, also LY293111, CGS025019C, CP-195543, SC-53228, BIIL 284, ONO 4057 and SB 209247; LTD4 antagonists such as montelukast and zafirlukast; Dopamine receptor agonists such as cabergoline, bromocriptine, ropinirole and 4-hydroxy-7-[2-[[2-[[3-(2-phenylethoxy)propyl]sulfonyl]ethyl]-amino]ethyl]-2(3H)-benzothiazolone and pharmaceutically acceptable salts thereof (the hydrochloride being Viozan^{®} - AstraZeneca); PDE4 inhibitors such as cilomilast (Ariflo® GSK), Roflumilast (Byk Gulden),V-11294A (Napp), BAY19-8004 (Bayer), SCH-351591 (Schering-Plough), Arofylline (Almirall Prodesfarma), PD189659 / PD168787 (Parke-Davis), AWD-12-281 (Asta Medica), CDC-801 (Celgene), SelCID(TM) CC-10004 (Celgene), VM554/UM565 (Vernalis), T-440 (Tanabe), KW-4490 (Kyowa Hakko Kogyo), WO 92/19594, WO 93/19749, WO 93/19750, WO 93/19751, WO 99/16766, WO 01/13953, WO 03/104204, WO 03/104205, WO 04/000814, WO 04/000839 and WO 04/005258, WO 04018450, WO 04/018451, WO 04/018457, WO 04/018465, WO 04/018431, WO 04/018449, WO 04/018450, WO 04/018451, WO 04/018457, WO 04/018465, WO 04/019944, WO 04/019945 and WO 04/045607, WO 04/037805 as well as those described in WO 98/18796 and WO 03/39544; A2a agonists such as those described in EP 409595A2, EP 1052264, EP 1241176, WO 94/17090, WO 96/02543, WO 96/02553, WO 98/28319, WO 99/24449, WO 99/24450, WO 99/24451, WO 99/38877, WO 99/41267, WO 99/67263, WO 99/67264, WO 99/67265, WO 99/67266, WO 00/23457, WO 00/77018, WO 00/78774, WO 01/23399, WO 01/27130, WO 01/27131, WO 01/60835, WO 01/94368, WO 02/00676, WO 02/22630, WO 02/96462, WO 03/086408, WO 04/039762, WO 04/039766, WO 04/045618, WO 04/046083; and A2b antagonists such as those described in WO 02/42298.

Such bronchodilatory drugs include anticholinergic or antimuscarinic agents, in particular ipratropium bromide, oxitropium bromide, tiotropium bromide, CHF 4226 (Chiesi) and glycopyrrolate, but also those described in WO 01/04118, WO 02/51841, WO 02/53564, WO 03/00840, WO 03/87094, WO 04/05285, WO 02/00652, WO 03/53966, EP 424021, US 5171744, US 3714357, US 5171744, WO 03/33495 and WO 04/018422; and beta (β)-2-adrenoceptor agonists such as albuterol (salbutamol), metaproterenol, terbutaline, salmeterol, fenoterol, procaterol, and especially, formoterol and pharmaceutically acceptable salts thereof, and compounds (in free or salt or solvate form) of formula (I) of WO 00/75114, which document is incorporated herein by reference, preferably compounds of the Examples thereof, especially a compound of formula 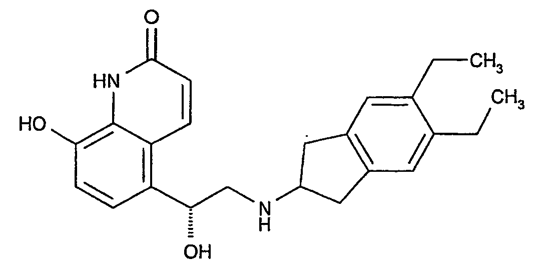 and pharmaceutically acceptable salts thereof, as well as compounds (in free or salt or solvate form) of formula (I) of WO 04/16601. Further suitable β -2-adrenoreceptor agonists include compounds such as those described in JP 05025045, US 2002/0055651, WO 93/18007, WO 99/64035, WO 01/42193, WO 01/83462, WO 02/066422, WO 02/070490, WO 02/076933, WO 03/24439, WO 03/72539, WO 03/42160, WO 03/91204, WO 03/42164, WO 03/99764, WO 04/11416, WO 04/16578, WO 04/22547, WO 04/32921, WO 04/33412, WO 04/37773, WO 04/37807, WO 04/39762, WO 04/39766, WO 04/45618 and WO 04/46083.

Such co-therapeutic antihistamine drug substances include cetirizine hydrochloride, acetaminophen, clemastine fumarate, promethazine, loratidine, desloratidine, diphenhydramine and fexofenadine hydrochloride, activastine, astemizole, azelastine, ebastine, epinastine, mizolastine and tefenadine as well as those disclosed in JP 2004107299, WO 03/99807 and WO 04/26841.

Combinations of agents of the invention and one or more steroids, beta-2 agonists, PDE4 inhibitors or LTD4 antagonists may be used, for example, in the treatment of COPD or, particularly, asthma. Combinations of agents of the invention and anticholinergic or antimuscarinic agents, PDE4 inhibitors, dopamine receptor agonists or LTB4 antagonists may be used, for example, in the treatment of asthma or, particularly, COPD.

Other useful combinations of agents of the invention with anti-inflammatory drugs are those with other antagonists of chemokine receptors, e.g. CCR-1, CCR-2, CCR-4, CCR-5, CCR-6, CCR-7, CCR-8, CCR-9 and CCR10, CXCR1, CXCR2, CXCR3, CXCR4, CXCR5, particularly CCR-5 antagonists such as Schering-Plough antagonists SC-351125, SCH-55700 and SCH-D, Takeda antagonists such as N-[[4-[[[6,7-dihydro-2-(4-methylphenyl)-5H-benzocyclohepten-8-yl]carbonyl]amino]phenyl]-methyl]tetrahydro-N,N-dimethyl-2H-pyran-4-aminium chloride (TAK-770), CCR-5 antagonists described in US 6166037 (particularly claims 18 and 19), WO 00/66558 (particularly claim 8), and WO 00/66559 (particularly claim 9), WO 04/018425 and WO 04/026873.

In accordance with the foregoing, a method is disclosed for the treatment of a condition mediated by CCR-3, for example an inflammatory or allergic condition, particularly an inflammatory or obstructive airways disease, which comprises administering to a subject, particularly a human subject, in need thereof an effective amount of a compound of formula Ia or Ib in a free or pharmaceutically acceptable salt form as hereinbefore described. In another aspect the invention provides the use of a compound of formula Ia or Ib, in free or pharmaceutically acceptable salt form, as hereinbefore described for the manufacture of a medicament for the treatment of a condition mediated by CCR-3, e.g. an inflammatory or allergic condition, particularly an inflammatory or obstructive airways disease.

The agents of the invention may be administered by any appropriate route, e.g. orally, for example in the form of a tablet or capsule; parenterally, for example intravenously; by inhalation, for example in the treatment of inflammatory or obstructive airways disease; intranasally, for example in the treatment of allergic rhinitis; topically to the skin, e.g. in the treatment of atopic dermatitis; or rectally, e.g. in the treatment of inflammatory bowel disease.

In a further aspect, the invention also provides a pharmaceutical composition comprising as active ingredient a compound of formula Ia or Ib in free or pharmaceutically acceptable salt form, optionally together with a pharmaceutically acceptable diluent or carrier therefor. The composition may contain a co-therapeutic agent such as an anti-inflammatory bronchodilatory or antihistamine drug as hereinbefore described. Such compositions may be prepared using conventional diluents or excipients and techniques known in the galenic art. Thus oral dosage forms may include tablets and capsules. Formulations for topical administration may take the form of creams, ointments, gels or transdermal delivery systems, e.g. patches. Compositions for inhalation may comprise aerosol or other atomizable formulations or dry powder formulations.

When the composition comprises an aerosol formulation, it preferably contains, for example, a hydro-fluoro-alkane (HFA) propellant such as HFA134a or HFA227 or a mixture of these, and may contain one or more co-solvents known in the art such as ethanol (up to 20% by weight), and/or one or more surfactants such as oleic acid or sorbitan trioleate, and/or one or more bulking agents such as lactose. When the composition comprises a dry powder formulation, it preferably contains, for example, the compound of formula Ia or Ib having a particle diameter up to 10 microns, optionally together with a diluent or carrier, such as lactose, of the desired particle size distribution and a compound that helps to protect against product performance deterioration due to moisture e.g. magnesium stearate. When the composition comprises a nebulised formulation, it preferably contains, for example, the compound of formula Ia or Ib either dissolved, or suspended, in a vehicle containing water, a co-solvent such as ethanol or propylene glycol and a stabiliser, which may be a surfactant.

The invention includes (A) an agent of the invention in inhalable form, e.g. in an aerosol or other atomisable composition or in inhalable particulate, e.g. micronised form, (B) an inhalable medicament comprising an agent of the invention in inhalable form; (C) a pharmaceutical product comprising such an agent of the invention in inhalable form in association with an inhalation device; and (D) an inhalation device containing an agent of the invention in inhalable form.

Dosages of agents of the invention employed in practising the present invention will of course vary depending, for example, on the particular condition to be treated, the effect desired and the mode of administration. In general, suitable daily dosages for administration by inhalation are of the order of 0.01 to 30 mg/kg while for oral administration suitable daily doses are of the order of 0.01 to 100 mg/kg.

The invention is illustrated by the following Examples.

### EXAMPLES

Compounds of formula Ia that are also formula XX 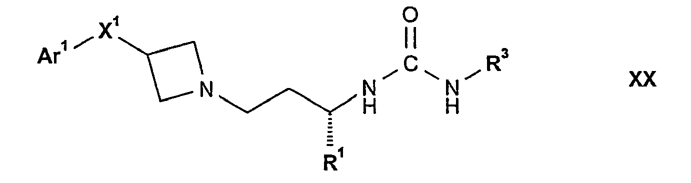 where Ar, X¹ and R³ are as shown in Table 1 below, the methods of preparation being described hereinafter. The table also shows characterising mass spectrometry data. The compounds are all in free form.

**TABLE 1**

| Ex. | Ar | X | R¹ | R³ | MS [M+H] |
|---|---|---|---|---|---|
| 1 | 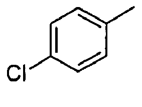 | 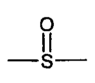 | 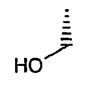 | 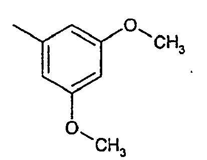 | 484.32 |
| 2 | 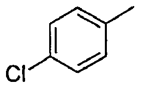 | 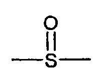 | 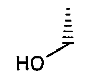 | 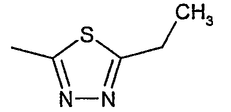 | 458.08 |
| 3 | 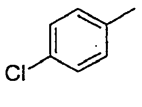 | 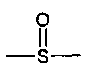 | 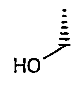 | 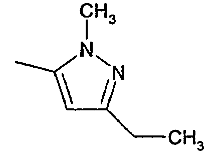 | - |
| 4 | 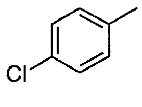 | 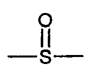 | 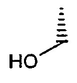 | 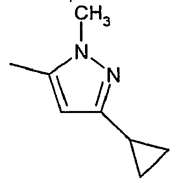 | 466.18 |
| 5 | 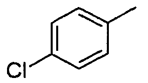 | 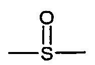 | 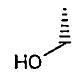 | 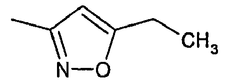 | - |
| 6 | 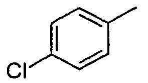 | 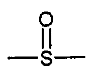 | 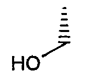 | 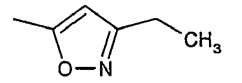 | - |
| 7 | 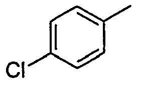 | 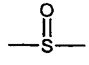 | 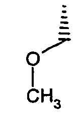 | 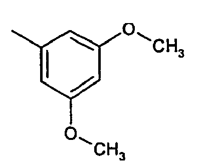 | - |
| 8 | 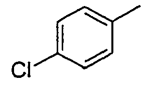 | 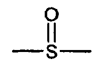 | 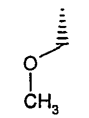 | 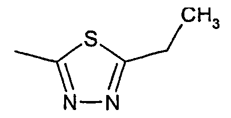 | - |
| 9 | 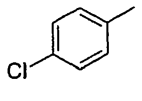 | 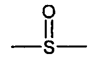 | 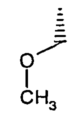 | 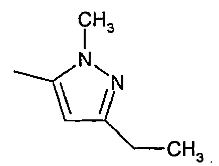 | - |
| 10 | 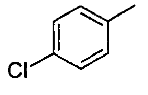 | 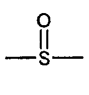 | 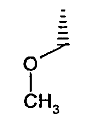 | 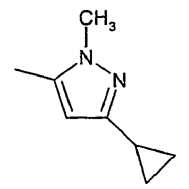 | - |
| 11 | 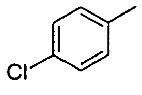 | 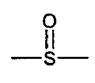 | 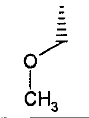 | 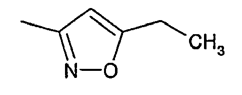 | - |
| 12 | 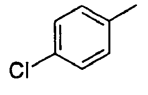 | 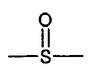 | 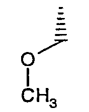 | 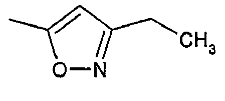 | - |
| 13 | 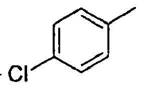 | 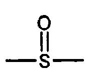 | 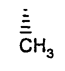 | 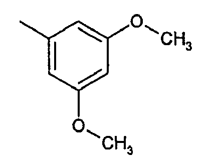 | - |
| 14 | 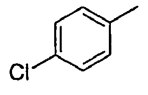 | 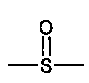 | 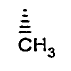 | 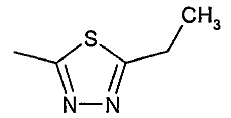 | - |
| 15 | 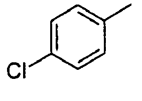 | 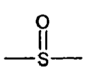 | 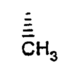 | 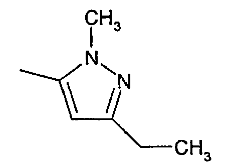 | - |
| 16 | 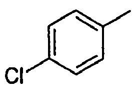 | 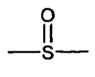 | 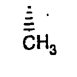 | 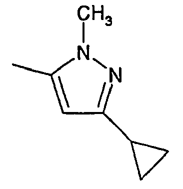 | - |
| 17 | 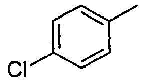 | 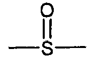 | 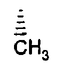 | 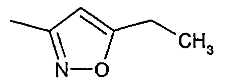 | - |
| 18 | 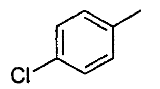 | 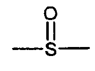 | 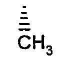 | 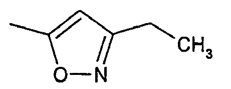 | - |
| 19 | 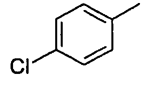 | -s- | 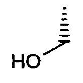 | 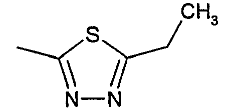 | 442.07 |
| 20 | 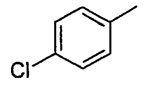 | -s- | 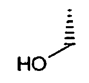 | 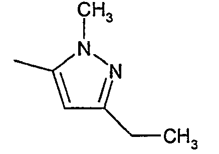 | - |
| 21 | 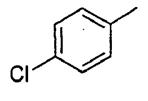 | -s- | 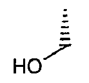 | 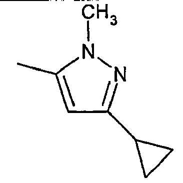 | - |
| 22 | 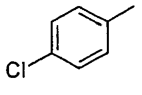 | -s- | 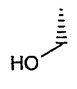 | 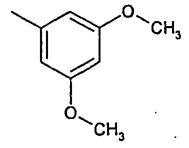 | - |
| 23 | 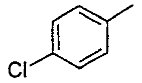 | -s- | 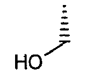 | 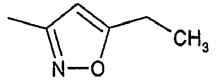 | - |
| 24 | 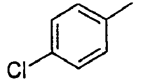 | -s- | 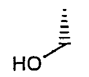 | 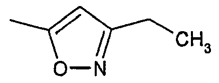 | - |
| 25 | 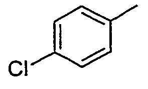 | 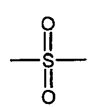 | 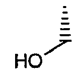 | 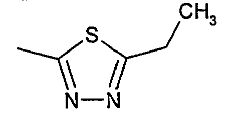 | 474.11 |
| 26 | 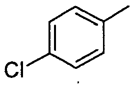 | 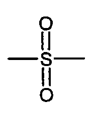 | 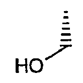 | 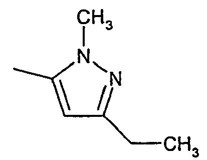 | - |
| 27 | 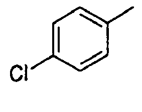 | 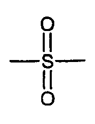 | 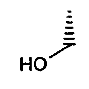 | 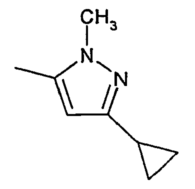 | - |
| 28 | 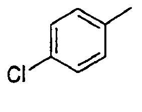 | 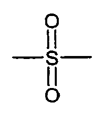 | 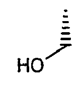 | 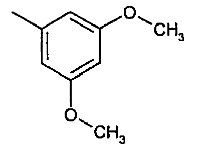 | - |
| 29 | 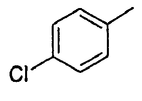 | 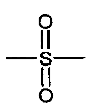 | 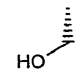 | 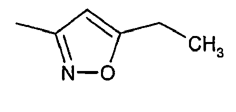 | - |
| 30 | 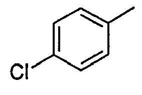 | 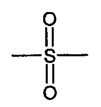 | 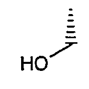 | 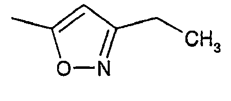 | - |

### Preparation of Intermediates

### [(S)-1-(tert-butyl-diphenyl-silanyloxymethyl)-3-iodo-propyl]-carbamic acid tert-butyl ester:

### (a) (S)-3-tert-Butoxycarbonylamino-4-(tert-butyl-diphenyl-silanyloxy)-butyric acid benzyl ester

A solution of (S)-3-tert-butoxycarbonylamino-4-hydroxy-butyric acid benzyl ester (1.34 g, 4.37 mmol) (prepared using the method of Rodriguez, Marc; Linares, Muriel; Doulut, Sylvie; Heitz, Annie; Martinez, Jean; Tetrahedron Lett. (1991), 32(7), 923-6.) and imidazole (0.88 g, 13.01 mmol in dimethylformamide (7 ml) is treated with tertbutyldiphenylsilyl chloride (1.69 ml, 6.5 mmol). The reaction mixture is stirred together at room temperature for 1 hour, then diluted with water and extracted into ethyl acetate. The ethyl acetate phase is dried over MgSO₄ and evaporated. The crude product is purified by flash silica chromatography (elution with 1:1 ethyl acetate/hexane) to afford (S)-3-tert-butoxycarbonylamino-4-(tert-butyl-diphenyl-silanyloxy)-butyric acid benzyl ester. [M-BOC] 448.0.

### (b) [(S)-1-(tert-Butyl-diphenyl-silanyloxymethyl)-3-hydroxy-propyl]-carbamic acid tert-butyl ester

A solution of (S)-3-tert-butoxycarbonylamino-4-(tert-butyl-diphenyl-silanyloxy)-butyric acid benzyl ester in (2.37 g, 4.33 mmol) in dry diethylether (25 ml) at 0°C is treated with a 2 M solution of lithium borohydride in THF (4.33 ml). The reaction mixture is allowed to warm to ambient temperature and stirred for 3 hours under argon, then quenched by addition of water (10 ml) and 0.5 M aqueous citric acid solution (20 ml). The ether is separated, and the aqueous phase extracted with more ether. The combined ether phases are dried over MgSO₄ and evaporated. The crude product is purified by flash silica chromatography on a biotage column (90 g) (elution with a 1:3 ethyl acetate/hexane then methanol) to afford [(S)-1-(tert-butyldiphenyl-silanyloxymethyl)-3-hydroxy-propyl]-carbamic acid tert-butyl ester [M-BOC] 344.1.

### (c) [(S)-1-(tert-Butyl-dilphenyl-silanyloxymethyl)-3-iodo-propyl]-carbamic acid tert-butyl ester

A suspension of polystyrene resin-bound triphenylphosphine (2.33 g, 3 mmol/g) in dry dichloromethane (25 ml) is treated with iodine (1.56 g, 6.16 mmol) and stirred for 15 minutes under argon. Imidazole (0.477 g, 7.0 mmol) is added and the reaction mixture stirred at room temperature for a further 15 minutes. The reaction mixture is then treated with a solution of [(S)-1-(tert-butyl-diphenyl-silanyloxymethyl)-3-hydroxy-propyl]-carbamic acid tert-butyl ester (1.24 g, 2.8 mmol) in dichloromethane (5 ml). The reaction mixture is refluxed for 2 hours under argon, then filtered through a Celite^{™} filter pad, washing with dichloromethane. The filtrate is washed with 5% aqueous sodium thiosulphate solution and water, dried over MgSO₄ and evaporated. The crude product is purified by flash silica chromatography (elution with 1:99 methanol/dichloromethane) to afford [(S)-1-(tert-butyl-diphenyl-silanyloxymethyl)-3-iodo-propyl]-carbamic acid tert-butyl ester. [M-BOC] 453.9.

### ((S)-3-Iodo-1-methoxymethyl-propyl)-carbamic acid tert-butyl ester:

### (a) (S)-3-tert-Butoxycarbonylamino-4-methoxy-butyric acid benzyl ester

A solution of (S)-3-tert-butoxycarbonylamino-4-hydroxy-butyric acid benzyl ester (31.4 g, 101 mmol) (prepared using the method of Rodriguez, Marc; Linares, Muriel; Doulut, Sylvie; Heitz, Annie; Martinez, Jean ;Tetrahedron Lett. (1991), 32(7), 923-6.) in dichloromethane (280 ml) is cooled to -20°C and a 48 % aqueous solution of tetrafluoroboric acid (13.3 ml, 101 mmol) added. With vigorous stirring is added dropwise a 2.0 M solution of trimethylsilyl-diazomethane (50.8 ml, 101 mmol)) in hexane over 35 minutes. After stirring for a further 30 minutes, a second aliquot of trimethylsilyldiazomethane is added (12.7 ml, 25 mmol) slowly over 10 minutes. After stirring for a further 30 minutes at -20°C a further aliquot of trimethylsilyl-diazomethane (12.7 ml, 25 mmol) is added over 10 minutes. This pattern is continued until a total of 127 ml of trimethylsilyldiazomethane solution (254 mmol) is added. After the last addition the reaction mixture is left to stir for 1.5 hours at -20°C. The reaction mixture is then quenched with water and extracted into dichloromethane. The organic phase is dried over magnesium sulphate and evaporated. The crude product is purified by flash silica chromatography (elution with 2:8 ethylacetate / hexane) to afford (S)-3-tert-Butoxycarbonylamino-4-methoxy-butyric acid benzyl ester as a clear oil. [M-BOC] 224.19.

### (b) ((S)-3-Hydroxy-1-methoxvmethyl-propyl)-carbamic acid tert-butyl ester

A solution of (S)-3-tert-Butoxycarbonylamino-4-methoxy-butyric acid benzyl ester (10.4 g, 32.3 mmol) in dry diethylether (70 ml) cooled to 0°C is treated slowly with a 2.0 M solution of LiBH₄ in THF (32.2 ml, 64.4 mmol). The reaction mixture is allowed to warm to ambient temperature and then left to stir. After 6 hours the reaction mixture is quenched slowly with 0.5 M aqueous solution of citric acid and extracted with ether. The ether phase is dried over magnesium sulphate and evaporated. The crude product is purified by flash silica chromatography (elution with a gradient 3:7 to 6:4 ethylacetate/hexane) to afford ((S)-3-Hydroxy-1-methoxymethyl-propyl)-carbamic acid tert-butyl ester as a clear oil. [M-BOC] 120.13

### (c) ((S)-3-Iodo-1-methoxymethyl-propyl)-carbamic acid tert-butyl ester

A suspension of polystyrene resin-bound triphenylphosphine (18.645 g, 3 mmol/g) in dry dichloromethane (250 ml) is treated with iodine (12.5 g, 49.22 mmol) and stirred for 15 minutes under argon . Imidazole (3.87 g, 55.94 mmol) is added and the reaction mixture stirred at room temperature for a further 15 minutes. The reaction mixture is then treated with a solution of ((S)-3-Hydroxy-1-methoxymethyl-propyl)-carbamic acid tert-butyl ester (4.9 g, 22.37 mmol) in dichloromethane (30 ml), and refluxed for 1.5 hours under argon. The resin is removed by filtration through a Celite^{™} filter material pad, washing with dichloromethane. The filtrate is washed with 5% aqueous sodium thiosulphate solution and water, dried over MgSO₄ and evaporated to afford ((S)-3-Iodo-1-methoxymethyl-propyl)-carbamic acid tert-butyl ester as a crude oil. [M-BOC] 230.06.

### ((R)-3-iodo-1-methyl-propyl)-carbamic acid tert-butyl ester:

### (a) (S)-3-tert-Butoxycarbonylamino-4-iodo-butyric acid benzyl ester

A suspension of polymer bound triphenyl phosphine (18.25 g, 54.76 mmol) in DCM (100 ml) is treated with iodine (12.2 g, 48.1 mmol) and the reaction mixture stirred at ambient temperature for 15 minutes. Imidazole (3.72 g, 54.7 mmol) is added and the reaction mixture stirred for a further 15 minutes. A solution of (S)-3-tert-butoxycarbonylamino-4-hydroxybutyric acid benzyl ester (6.76 g, 21.9 mmol) in DCM (100 ml) is added. The suspension is stirred at reflux for 1.5 hours, then filtered through Celite^{™} filter material, washing through with DCM. The combined organic phase is washed with an aqueous solution of 10% sodium thiosulphate, water and brine, then dried over magnesium sulphate and evaporated. The crude product is chromatographed over flash silica using 8% ethylacetate in iso-hexane as eluent to afford (S)-3-tert-butoxycarbonylamino-4-iodo-butyric acid benzyl ester. [M-BOC] 320.12

### (b) ((R)-3-Hydroxy-1-methyl-propyl)-carbamic acid tert-butyl ester

A solution of (S)-3-tert-butoxycarbonylamino-4-iodo-butyric acid benzyl ester (0.2 g, 0.477 mmol) in dry diethylether (3 ml) cooled to 0°C under argon, is treated with a 2.0 M solution of lithium borohydride in THF (0.95 ml, 1.9 mmol). The reaction mixture is allowed to warm to ambient temperature with stirring for 18 hours. The reaction is quenched by addition of water and partitioned between ethylacetate and 10% citric acid solution. The organic phase is washed with brine, dried over magnesium sulphate and evaporated. The crude product is purified by flash silica chromatography using 1 % methanol in DCM as eluent to afford ((R)-3-hydroxy-1-methyl-propyl)-carbamic acid tert-butyl ester.

### (c) ((R)-3-Iodo-1-methyl-propyl)-carbamic acid tert-butyl ester

A suspension of polymer bound triphenyl phosphine (0.200 g, 0.595 mmol) in DCM (2 ml) is treated with iodine (0.133 g, 0.523 mmol) and the reaction mixture stirred at ambient temperature for 15 minutes. Imidazole (39 mg, 0.57 mmol) is added and the reaction mixture stirred for a further 15 minutes. A solution of ((R)-3-Hydroxy-1-methyl-propyl)-carbamic acid tert-butyl ester (0.045 g, 0.238 mmol) in DCM (2 ml) is added. The suspension is stirred at reflux for 2.5 hours, then filtered through Celite^{™} filter material, washing through with DCM. The combined organic phase is washed with an aqueous solution of 10% sodium thiosulphate, water and brine, then dried over magnesium sulphate and evaporated to afford ((R)-3-iodo-1-methyl-propyl)-carbamic acid tert-butyl ester. [M+H] 285.05

### 5-Ethyl-isoxazol-3-ylamine:

### (a) (2-Ethyl-[1,3]dioxolan-2-yl)-acetonitrile

A solution of 3-oxo-pentanenitrile (1.582 g, 16.49mmol), ethylene glycol (1.026 ml, 84.59 mmol) and a catalytic amount of p-Toluene sulphonic acid (8 mg) in Toluene (10 ml) is refluxed at 150°C for 2 days using Dean-Stark apparatus. The reaction mixture is diluted with ethyl acetate and washed with saturated sodium bicarbonate solution. The organic phase is dried over MgSO₄, filtered, and the solvent evaporated to yield (2-ethyl-[1,3]dioxolan-2-yl)-acetonitrile. 1H NMR (400MHz, CDCl3) d 4.15(2H,m), 4.05 (2H, m), 2.65 (2H, s), 1.80 (2H, q), 0.95 (3H, t)

### (b) 2-(2-Ethyl [1,3]dioxolan-2-yl)-N-hydroxy-acetamidine.

A solution of NaOH (1.17 g, 29.3 mmol) in water/methanol (1:1) (1 8ml), cooled to 0°C in an ice bath, is treated with hydroxylamine hydrochloride (1.58 g, 22.78 mmol), with stirring for 5 minutes. (2-Ethyl-[1,3]dioxolan-2-yl)-acetonitrile (1.42 g, 10.125 mmol) is added and the reaction mixture stirred at room temperature for 18 hours, then refluxed for a further 2 hours. The reaction mixture is cooled and partitioned between ethyl acetate and water. The organic phase is dried over MgSO₄, filtered, and the solvent evaporated to yield 2-(2-Ethyl [1,3]dioxolan-2-yl)-N-hydroxy-acetamidine. [M+H] 175.21

### (c) 5-Ethyl-isoxazol-3-ylamine

A solution of 2-(2-ethyl [1,3]dioxolan-2-yl)-N-hydroxy-acetamidine (1.49 g 8.564 mmol) in ethanol (49 ml), acidified to pH 1 with concentrated hydrochloric acid solution, is refluxed at 50°C for 3 days. The solvent is evaporated and the crude product dissolved in water and extracted into ethyl acetate (x2). The organic phase is dried over MgSO₄ and evaporated to afford 5-ethyl-isoxazol-3-ylamine. [M + H] 113.02

### (5-Ethyl-[1,3,4]thiadiazol-2-yl)-carbamic acid phenyl ester:

A solution of 5-Ethyl-[1,3,4]thiadiazol-2-ylamine (2.5g, 19.4mmol) and pyridine (1.72 ml, 21.3mmol) in dichloromethane (70 ml) is cooled to -70°C and treated with a solution of phenylchloroformate (2.45 ml, 19.6 mmol) in dichloromethane (10 ml) dropwise. The reaction mixture is allowed to warm to ambient temperature and stirred for 3 hours during which a precipitate forms. The precipitate is collected by filtration, and dried under vacuum to afford (5-Ethyl-[1,3,4]thiadiazol-2-yl)-carbamic acid phenyl ester as white solid. [M+H] 250.15

### (5-Cyclopropyl-2-methyl-2H-pyrazol-3-yl)-carbamic acid phenyl ester:

A solution of 5-Cyclopropyl-2-methyl-2H-pyrazol-3-ylamine (3.0 g, 22 mmol) (65 ml) and sodium bicarbonate (2 g, 24 mmol) in THF is cooled to 0°C. Phenylchloroformate (3.4 g, 22 mmol) is added dropwise over 15 minutes. The reaction mixture is allowed to warm to ambient temperature and stirred for 5 hours, then filtered and the filtrate partitioned between ethyl acetate and water. The organic phase is washed with water, 5% aqueous citric acid solution and brine, dried over MgSO₄ and evaporated to afford (5-Cyclopropyl-2-methyl-2H-pyrazol-3-yl)-carbamic acid phenyl ester. [M+H] 258.17.

### (5-Ethyl-2-methyl-2H-pyrazol-3-yl)-carbamic acid phenyl ester:

### (a) 5-Ethyl-2-methyl-2H-pyrazol-3-ylamine

A solution of 3-Oxo-pentanenitrile (0.5 g, 5.15 mmol) and methylhydrazine (0.24 g, 5.15 mmol) in ethanol (5ml) is heated to reflux for 1.5 hours. The solvent is evaporated and the residue partitioned between ethyl acetate and brine. The organic phase is dried over MgSO4 and evaporated to afford 5-Ethyl-2-methyl-2H-pyrazol-3-ylamine. [M+H]

### (b) (5-Ethyl-2-methyl-2H-pyrazol-3-yl)-carbamic acid phenyl ester

A solution of 5-Ethyl-2-methyl-2H-pyrazol-3-ylamine (10 g, 79.8 mmol) (500 ml) and potassium carbonate in THF (12.14 g, 87.8 mmol) is cooled to 0°C. Phenylchloroformate (10.15 ml, 80.6 mmol) is added dropwise over 20 minutes. The reaction mixture is stirred for 40 minutes at 0°C then allowed to warm to ambient temperature and stirred for a further 2.5 hours. The reaction mixture is partitioned between ethyl acetate and water. The organic phase is washed with 5% aqueous citric acid solution and brine. The organic phase is treated with MgS04 and charcoal then filtered and evaporated to afford (5-Cyclopropyl-2-methyl-2H-pyrazol-3-yl)-carbamic acid phenyl ester. [M+H] 246.21

### 5-Cyclobutyl-2-methyl-2H-pyrazol-3-yl)-carbamic acid phenyl ester:

### (a) 5-Cyclobutyl-2-methyl-2H-pyrazol-3-ylamine

This is synthesized in an analogous manner to 5-ethyl-2-methyl-2H-pyrazol-3-ylamine except using 3-cyclobutyl-3-oxopropionitrile instead of 3-oxo-pentanenitrile.

### (b) 5-Cyclobutyl-2-methyl-2H-pyrazol-3-yl)-carbamic acid phenyl ester

A solution of 5-Cyclobutyl-2-methyl-2H-pyrazol-3-ylamine (0.156 g, 1.03 mmol) in dimethyl-formamide (3 ml) cooled to 0°C, is treated with phenylchloroformate (0.13 ml, 1.03 mmol) dropwise and left to stir at 0°C for 1 hour. The reaction mixture is partitioned between ethyl acetate and 1.0 M hydrochloric acid solution, and the organic phase washed with water, dried over magnesium sulphate and evaporated to yield (5-Cyclobutyl-2-methyl-2H-pyrazol-3-yl)-carbamic acid phenyl ester. [M+H] 272.22

### (2-Ethyl-2H-tetrazol-5-yl)-carbamic acid phenyl ester:

A solution of 2-ethyl-2H-tetrazol-5-ylamine (0.1 g, 0.88 mmol) in dry THF (2 ml) is treated with pyridine (0.09 ml, 1.10 mmol) followed by a solution of phenylchloroformate (0.11 ml, 0.911 mmol) in THF (1 ml). The reaction mixture is stirred at ambient temperature for 1.5 hours, then partitioned between ethyl acetate and water. The ethyl acetate phase is dried over magnesium sulphate and evaporated to afford (2-Ethyl-2H-tetrazol-5-yl)-carbamic acid phenyl ester as a white solid. 1H NMR (CDCl3, 400 MHz) δ, 7.90 (1H, brs), 7.30 (2H, m), 7.15, (3H, m), 4.60 (2H, q) 1.60 (3H, t).

(5-Ethyl-isoxazol-3-yl)-carbamic acid phenyl ester and (3-Ethyl-isoxazol-5yl)-carbamic acid phenyl ester are prepared analogously using 5-Ethyl-isoxazol-3-ylamine and 5-Ethyl-isoxazol-5-ylamine respectively in place of 2-ethyl-2H-tetrazol-5-ylamine.

### Preparation of final compounds

### Example 1

### 1-{(S)-3-[3-(4-Chloro-benzenesulfinyl)-azetidin-1-yl]-1-hydroxymethyl-propyl}-3-(3,5-dimethoxy-phenyl)-urea:

### (a) 1-Benzhydryl-3-(4-chloro-benzenesulfinyl)-azetidine

A solution of 1-Benzhydryl-3-(4-chloro-phenylsulfanyl)-azetidine (3.16 g, 8.6 mmol) in chloroform (30 ml) cooled to -15°C is treated slowly with a solution of meta chloro perbenzoic acid (2.17 g, 8.8 mmol) in chloroform over 2 hours. The reaction mixture is washed with water, aqueous saturated sodium bisulphate solution, sodium bicarbonate solution and brine. The organic phase is dries over magnesium sulphate and evaporated. The crude product is purified by flash silica chromatography (elution with iso-hexane : ether, 7:3 to 1:1) to afford 1-Benzhydryl-3-(4-chlorobenzenesulfinyl)-azetidine, [MH]+ 384.12.

### (b) 3 -(4-Chloro-benzenesulfinyl)-azetidine

A solution of 1-Benzhydryl-3-(4-chloro-phenylsulfinyl)-azetidine (2.6g, 6.8 mmol) in dichloromethane (35 ml) cooled to -4°C, is treated with 1-chloroethoxycarbonyl chloride (1.25 ml, 11.54 mmol) and the reaction mixture allowed to warm to room temperature with stirring for 18 hours. The solvent is evaporated and the residue taken up in methanol (15 ml) and stirred at room temperature for a further 18 hours. The solvent is evaporated and the residue crystallized from methanol/diethylether to afford 3-(4-Chloro-phenylsulfinyl)-azetidine. [MH]+ 218.06

### (c) {(S)-1-(tert-Butyl-diphenyl-silanyloxymethyl)-3-[3-(4-chloro-benzenesulfinyl)-azetidin-1-yl]-propyl}-carbamic acid tert-butyl ester

A solution of 3-(4-Chloro-phenylsulfinyl)-azetidine (1.17 g, 4.6 mmol), [(S)-1-(tert-Butyldiphenyl-silanyloxymethyl)-3-iodo-propyl]-carbamic acid tert-butyl ester (2.8 g, 5.1 mmol) and triethylamine (2.6 ml, 18.4 mmol) in dimethylformamide (35 ml) is stirred at ambient temperature for 18 hours. The reaction mixture is partitioned between ethyl acetate and saturated sodium bicarbonate solution. The organic phase is washed with water and brine, dried over magnesium sulphate and evaporated. The crude product is purified by flash silica chromatography (elution with dichloromethane: methanol, 95:5) to afford {(S)-1-(tert-Butyldiphenyl-silanyloxymethyl)-3-[3-(4-chloro-phenylsulfinyl)-azetidin-1-yl]-propyl}-carbamic acid tert-butyl ester. [MH] + 641.29.

### (d) {(S)-3-[3-(4-Chloro-benzenesulfinyl)-azetidin-1-yl]-1-hydroxymethyl-propyl}-carbamic acid tert-butyl ester

A solution of {(S)-1-(tert-Butyl-diphenyl-silanyloxymethyl)-3-[3-(4-chloro-phenylsulfinyl)-azetidin-1-yl]-propyl}-carbamic acid tert-butyl ester (2.7 g, 4.21 mmol) in THF (30 ml) is treated with a 1.0 M solution of tetrabutylammonium fluoride in THF (4.21 ml, 4.21 mmol) and the reaction mixture stirred at ambient temperature for 18 hours. The solvent is evaporated and the residue partitioned between ethyl acetate and saturated sodium bicarbonate solution. The organic phase is washed with water and brine, dried over magnesium sulphate and evaporated. The crude product is purified by flash silica chromatography (elution with dichloromethane: methanol, 95:5) to afford {(S)-3-[3-(4-Chloro-phenylsulfinyl)-azetidin-1-yl]-1-hydroxymethyl-propyl}-carbamic acid tert-butyl ester. [MH]+ 403.18

### (e) (S)-2-Amino-4-[3-(4-chloro-benzenesulfinyl)-azetidin-1-yl]-butan-1-ol

A solution of {(S)-3-[3-(4-Chloro-phenylsulfinyl)-azetidin-1-yl]-1-hydroxymethyl-propyl}-carbamic acid tert-butyl ester (1.06 g, 2.63 mmol) in dichloromethane (30 ml) is treated with trifluoroacetic acid (7 ml) and stirred at ambient temperature for 2 hours. The solvent is evaporated and the residue partitioned between dichloromethane and sodium hydroxide solution. The DCM phase is dried over magnesium sulphate and evaporated to afford (S)-2-Amino-4-[3-(4-chloro-phenylsulfinyl)-azetidin-1-yl]-butan-l-ol. [M +H] 303.8

### (f) 1-{(S)-3-[3-(4-Chloro-benzenesulfinyl)-azetidin-1-yl]-1-hydroxymethyl-propyl}-3-(3,5-dimethoxy-phenyl)-urea

A solution of (S)-2-Amino-4-[3-(4-chloro-phenylsulfinyl)-azetidin-1-yl]-butan-1-ol (0.1 g, 0.264 mmol) and dimethoxyphenylisocyanate (0.048 g, 0.264 mmol) in dichloromethane is stirred at ambient temperature for 2 hours. Te solvent is evaporated and the crude product is purified by flash silica chromatography (elution with dichloromethane: methanol, 99:1 to 95:5) to afford 1-{(S)-3-[3-(4-Chloro-benzenesulfinyl)-azetidin-1-yl]-1-hydroxymethyl-propyl}-3-(3,5-dimethoxy-phenyl)-urea [MH]+ 484.32

### Examples 2 to 6

The compounds of these Examples, namely 1-{(S)-3-[3-(4-Chloro-benzenesulfinyl)-azetidin-1-yl]-1-hydroxymethyl-propyl)-3-(5-ethyl-[1,3,4]thiadiazol-2)-urea, 1-{(S)-3-[3-(4-Chlorobenzenesulfinyl)-azetidin-1-yl]-1-hydroxymethyl-propyl}-3-(5-ethyl-2-methyl-2H-pyrazol-3-yl)-urea, 1-{(S)-3-[3-(4-Chloro-benzenesulfinyl)-azetidin-1-yl]-1-hydroxymethyl-propyl}-3-(5-cyclopropyl-2-methyl-2H-pyrazol-3-yl)-urea, 1-{(S)-3-[3-(4-Chloro-benzenesulfinyl)-azetidin-1-yl]-1-hydroxymethyl-propyl}-3-(5-ethyl-isoxazol-3-yl)-urea and 1-{(S)-3-[3-(4-Chlorobenzenesulfinyl)-azetidin-1-yl]-1-hydroxymethyl-propyl}-3-(3-ethyl-isoxazol-5-yl)-urea are prepared in a manner that is analogous to that described in Example 1.

### Examples 7 to 12

The compounds of these Examples are prepared in a manner that is analogous to that described in Examples 1 to 6 but using ((S)-3-Iodo-1-methoxymethyl-propyl)-carbamic acid tert-butyl ester in place of [(S)-1-(tert-butyl-diphenyl-silanyloxymethyl)-3-iodo-propyl]-carbamic acid tert-butyl ester.

### Examples 13 to 18

The compounds of these Examples are prepared in a manner that is analogous to that described in Examples 1 to 6 but using ((R)-3-iodo-1-methyl-propyl)-carbamic acid tert-butyl ester in place of [(S)-1-(tert-butyl-diphenyl-silanyloxymethyl)-3-iodo-propyl]-carbamic acid tert-butyl ester.

### Example 19

### 1-{(S)-3-[3-(4-Chloro-phenylsulfanyl)-azetidin-1-yl]-1-hydroxymethyl-propyl}-3-(5-ethyl-[1,3,4]thiadiazol-2-yl)-urea:

### (a) 1-Benzhydryl-3-(4-chloro-phenylsulfanyl)-azetidine

A solution of 4-chlorothiophenol (3.0 g, 20.7 mmol) in dimethylformamide (30 ml) is treated with 60% sodium hydride dispersion in mineral oil (1.2 g, 30 mmol), stirred for 10 minutes at ambient temperature, then treated with a solution of methanesulfonic acid 1-benzhydrylazetidin-3-yl ester (5.98 g, 18.8 mmol) in dimethylformamide (40 ml). The reaction mixture is heated at 60°C for 5 hours, then cooled to ambient temperature and partitioned between ethyl acetate and water. The organic phase is dried over magnesium sulphate and evaporated. The crude product is purified by flash silica chromatography (elution with iso-hexane: ether, 8:2) to afford 1-Benzhydryl-3-(4-chloro-phenylsulfanyl)-azetidine as a pale yellow solid.

### (b) 3-(4-Chloro-phenylsulfanyl)-azetidine

A solution of 1-Benzhydryl-3-(4-chloro-phenylsulfanyl)-azetidine (0.62 g, 1.7 mmol) in dichloromethane (8 ml) cooled to -4°C, is treated with 1-chloroethoxycarbonyl chloride (0.4 ml, 2.68 mmol) and the reaction mixture allowed to warm to room temperature with stirring for 18 hours. The solvent is evaporated and the residue taken up in methanol (15 ml) and stirred at room temperature for a further 18 hours. The solvent is evaporated and the residue crystallized from methanol/diethylether to afford 3-(4-Chloro-phenylsulfanyl)-azetidine as a white solid.

### (c) {(S)-1-(tert-Butyl-diphenyl-silanyloxymethyl)-3-[3-(4-chloro-phenylsulfanyl)-azetidin-1-yl]-pronyl}-carbamic acid tert-butyl ester

A solution of 3-(4-Chloro-phenylsulfanyl)-azetidine (0.26 g, 1.1 mmol), [(S)-1-(tert-Butyldiphenyl-silanyloxymethyl)-3-iodo-propyl]-carbamic acid tert-butyl ester (0.67 g, 1.21 mmol) and triethylamine (0.62 ml, 4.4 mmol) in dimethylformamide (12 ml) is stirred at ambient temperature for 18 hours. The reaction mixture is partitioned between ethyl acetate and saturated sodium bicarbonate solution. The organic phase is washed with water and brine, dried over magnesium sulphate and evaporated. The crude product is purified by flash silica chromatography (elution with dichloromethane: methanol, 95:5) to afford {(S)-1-(tert-Butyldiphenyl-silanyloxymethyl)-3-[3-(4-chloro-phenylsulfanyl)-azetidin-1-yl]-propyl}-carbamic acid tert-butyl ester. [MH] + 625.26.

### (d) {(S)-3-[3-(4-Chloro-phenylsulfanyl)-azetidin-1-yl]-1-hydroxymethyl-propyl}-carbamic acid tert-butyl ester

A solution of {(S)-1-(tert-Butyl-diphenyl-silanyloxymethyl)-3-[3-(4-chloro-phenylsulfanyl)-azetidin-1-yl]-propyl}-carbamic acid tert-butyl ester (0.612 g, 0.978 mmol) in THF (7 ml) is treated with a 1.0 M solution of tetrabutylammonium fluoride in THF (0.98 ml, 0.98 mmol) and the reaction mixture stirred at ambient temperature for 18 hours. The solvent is evaporated and the residue partitioned between ethyl acetate and saturated sodium bicarbonate solution. The organic phase is washed with water and brine, dried over magnesium sulphate and evaporated. The crude product is purified by flash silica chromatography (elution with dichloromethane: methanol, 95:5) to afford {(S)-3-[3-(4-Chloro-phenylsulfanyl)-azetidin-1-yl]-1-hydroxymethyl-propyl}-carbamic acid tert-butyl ester. [M]+ 387.15

### (e) (S)-2-Amino-4-[3-(4-chloro-phenylsulfanyl)-azetidin-1-yl]-butan-1-ol

A solution of {(S)-3-[3-(4-Chloro-phenylsulfanyl)-azetidin-1-yl]-1-hydroxymethyl-propyl}-carbamic acid tert-butyl ester (0.22 g, 0.568 mmol) in dichloromethane (7 ml) is treated with trifluoroacetic acid (3 ml) and stirred at ambient temperature for 3 hours. The solvent is evaporated and the residue partitioned between dichloromethane and sodium hydroxide solution. The DCM phase is dried over magnesium sulphate and evaporated to afford (S)-2-Amino-4-[3-(4-chloro-phenylsulfanyl)-azetidin-1-yl]-butan-1-ol.

### (f) 1-{(S)-3-[3-(4-Chloro-phenylsulfanyl)-azetidin-1-yl]-1-hydroxymethyl-propyl}-3-(5-ethyl-[1,3,4]thiadiazol-2-yl)-urea

A solution of (S)-2-Amino-4-[3-(4-chloro-phenylsulfanyl)-azetidin-1-yl]-butan-1-ol (0.12 g, 0.418 mmol) and (5-Ethyl-[1,3,4]thiadiazol-2-yl)-carbamic acid phenyl ester (0.104 g, 0.418 mmol) in DMSO (2 ml) is stirred at ambient temperature for 4 hours. The reaction mixture is partitioned between ethyl acetate and water. The organic phase is dried over magnesium sulphate and evaporated. The crude product is purified by flash silica chromatography (elution with DCM: methanol, 95:5 to 90:10) to afford 1-{(S)-3-[3-(4-Chloro-phenyl-sulfanyl)-azetidin-1-yl]-1-hydroxymethyl-propyl}-3-(5-ethyl-[1,3,4]thiadiazol-2-yl)-urea. [MH]+ 442.07.

### Examples 20 to 24

The compounds of these Examples, namely 1-{(S)-3-[3-(4-Chloro-phenylsulfanyl)-azetidin-1-yl]-1-hydroxymethyl-propyl}-3-(5-ethyl-2-methyl-2H-pyrazol-3-yl)-urea, 1-{(S)-3-[3-(4-Chlorophenylsulfanyl)-azetidin-1-yl]-1-hydroxymethyl-propyl}-3-(5-cyclopropyl-2-methyl-2H-pyrazol-3-yl)-urea, 1-{(S)-3-[3-(4-Chloro-phenylsulfanyl)-azetidin-1-yl]-1-hydroxymethyl-propyl}-3-(3,5-dimethoxy-phenyl)-urea and 1-{(S)-3-[3-(4-Chloro-phenylsulfanyl)-azetidin-1-yl]-1-hydroxymethyl-propyl}-3-(5-ethyl-isoxazol-3-yl)-urea and 1-{(S)-3-[3-(4-Chlorophenylsulfanyl)-azetidin-1-yl]-1-hydroxymethyl-propyl}-3-(3-ethyl-isoxazol-5-yl)-urea are prepared in a manner that is analogous to that described in Example 19.

### Example 25

### 1-{(S)-3-[3-(4-Chloro-benzenesulfonyl)-azetidin-1-yl]-1-hydroxymethyl-propyl}-3-(5-ethyl-[1,3,4]thiadiazol-2-yl)-urea:

A solution of 1-{(S)-3-[3-(4-Chloro-phenylsulfanyl)-azetidin-1-yl]-1-hydroxymethyl-propyl}-3-(5-ethyl-[1,3,4]thiadiazol-2-yl)-urea (0.055 g, 0.124 mmol) in dichloromethane (9 ml) cooled to 15°C, is slowly treated with a solution of *meta* chloroperbenzoic acid (0.052 mg, 0.3 mmol) in dichloromethane (2 ml) and stirred for 3 hours. The reaction mixture is diluted with dichloromethane and washed with saturated sodium bicarbonate solution and brine. The organic phase is dries over magnesium sulphate and evaporated. The crude product is purified by solid phase extraction using a 2 g Isolute SCX-3 cartridge (elution with methanol, then 5% ammonia in methanol) to afford 1-{(S)-3-[3-(4-Chloro-benzenesulfonyl)-azetidin-1-yl]-1-hydroxymethyl-propyl}-3-(5-ethyl-[1,3,4]thiadiazol-2-yl)-urea. [MH] + 474.11

### Examples 26 to 30

The compounds of these Examples, namely 1-{(S)-3-[3-(4-Chloro-benzenesulfonyl)-azetidin-1-yl]-1-hydroxymethyl-propyl}-3-(5-ethyl-2-methyl-2H-pyrazol-3-yl)-urea, 1-{(S)-3-[3-(4-Chlorobenzenesulfonyl)-azetidin-1-yl]-1-hydroxymethyl-propyl}-3-(5-cyclopropyl-2-methyl-2H-pyrazol-3-yl)-urea and 1-{(S)-3-[3-(4-Chloro- benzenesulfonyl)-azetidin-1-yl]-1-hydroxymethylpropyl}-3-(3,S-dimethoxy-phenyl)-urea, 1-{(S)-3-[3-(4-Chloro-benzenesulfonyl)-azetidin-1-yl]-1-hydroxymethyl-propyl}-3-(5-ethyl-isoxazol-3-yl)-urea and 1-{(S)-3-[3-(4-Chloro-benzenesulfonyl)-azetidin-1-yl]-1-hydroxymethyl-propyl}-3-(3-ethyl-isoxazol-5-yl)-urea are prepared in a manner that is analogous to that described in Example 25.

Compounds of formula Ib that are also formula XXI 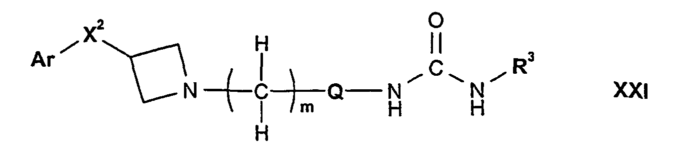 where Ar, X², m, Q and R³ are as shown in Table 2 below, the methods of preparation being described hereinafter. The table also shows characterising mass spectrometry data. The compounds are all in free form.

**TABLE 2**

| Ex. | Ar | X | m | -Q- | R³ | MS [M+H] |
|---|---|---|---|---|---|---|
| 31 | 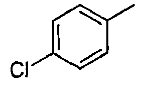 | -O- | 1 | 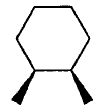 | 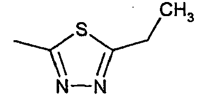 | 450.21 |
| 32 | 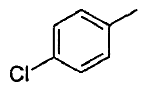 | -O- | 1 | 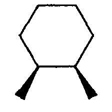 | 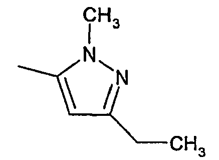 | 446.22 |
| 33 | 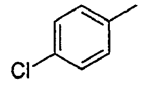 | -O- | 1 | 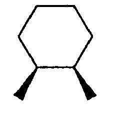 | 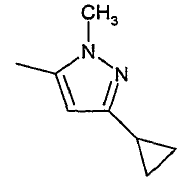 | 458.27 |
| 34 | 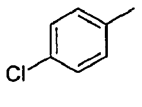 | -O- | 1 | 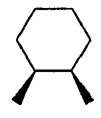 | 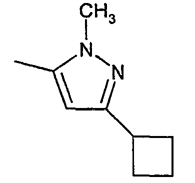 | - |
| 35 | 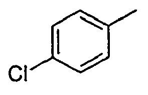 | -O- | 1 | 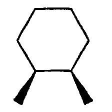 | 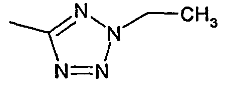 | - |
| 36 | 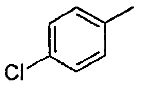 | -O- | 1 | 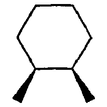 | 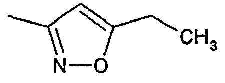 | - |
| 37 | 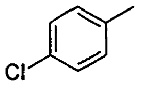 | -O- | | 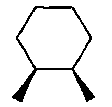 | 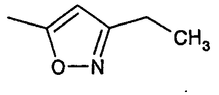 | - |
| 38 | 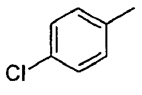 | 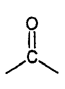 | | 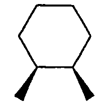 | 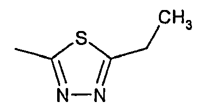 | - |
| 39 | 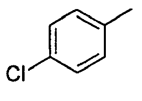 | 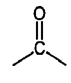 | | 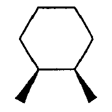 | 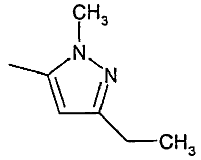 | - |
| 40 | 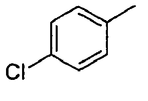 | 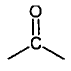 | | 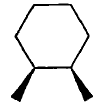 | 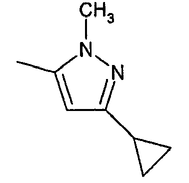 | - |
| 41 | 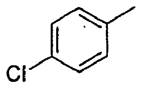 | 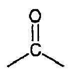 | | 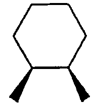 | 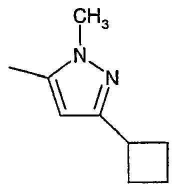 | - |
| 42 | 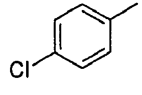 | 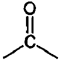 | | 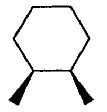 | 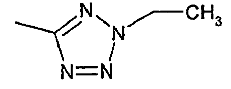 | - |
| 43 | 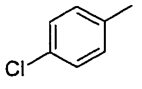 | 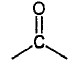 | | 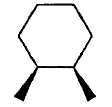 | 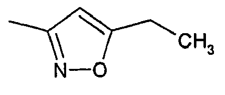 | - |
| 44 | 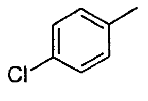 | 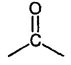 | | 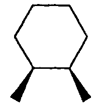 | 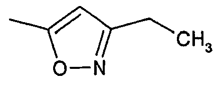 | - |
| 45 | 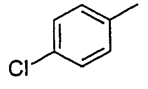 | 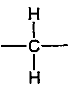 | | 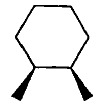 | 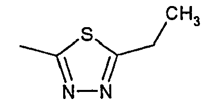 | - |
| 46 | 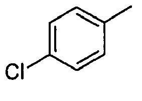 | 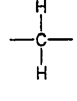 | | 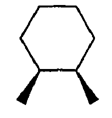 | 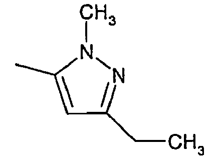 | - |
| 47 | 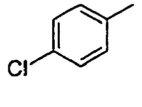 | 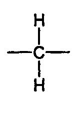 | 1 | 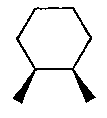 | 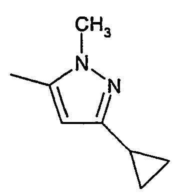 | - |
| 48 | 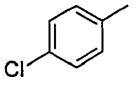 | 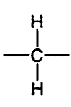 | 1 | 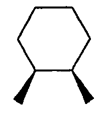 | 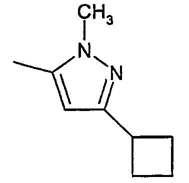 | - |
| 49 | 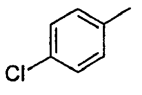 | 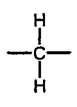 | 1 | 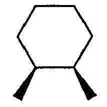 | 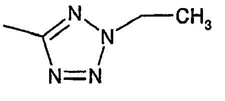 | - |
| 50 | 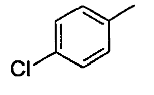 | 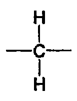 | 1 | 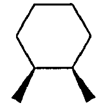 | 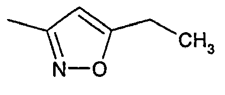 | - |
| 51 | 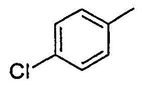 | 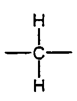 | 1 | 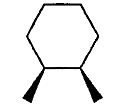 | 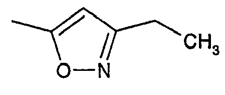 | - |
| 52 | 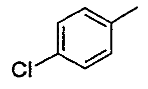 | -O- | 1 | 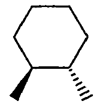 | 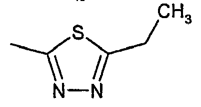 | 451.65 |
| 53 | 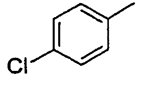 | -O- | 1 | 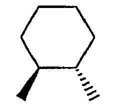 | 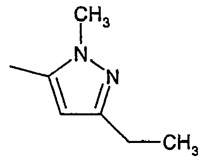 | 446.24 |
| 54 | 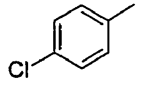 | -O- | 1 | 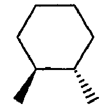 | 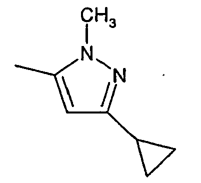 | 458.21 |
| 55 | 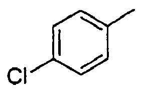 | -O- | 1 | 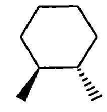 | 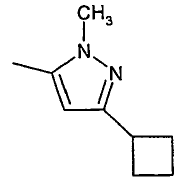 | - |
| 56 | 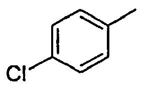 | -O- | 1 | 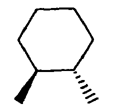 | 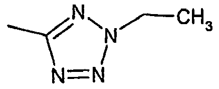 | - |
| 57 | 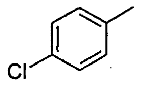 | -O- | 1 | 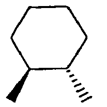 | 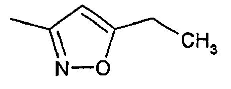 | - |
| 58 | 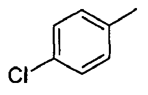 | -0- | 1 | 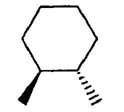 | 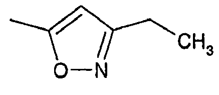 | - |
| 59 | 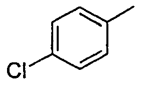 | 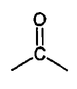 | 1 | 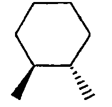 | 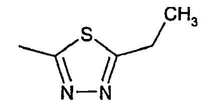 | - |
| 60 | 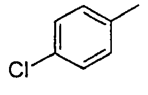 | 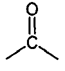 | 1 | 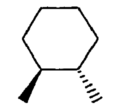 | 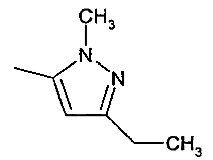 | - |
| 61 | 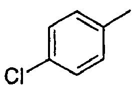 | 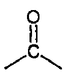 | 1 | 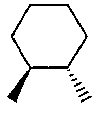 | 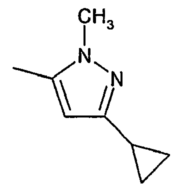 | - |
| 62 | 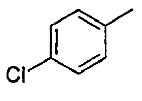 | 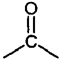 | 1 | 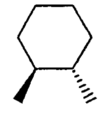 | 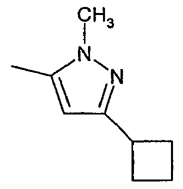 | - |
| 63 | 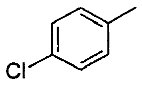 | 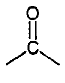 | 1 | 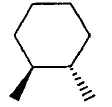 | 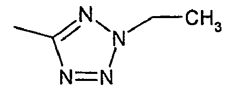 | - |
| 64 | 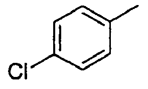 | 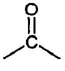 | 1 | 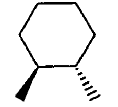 | 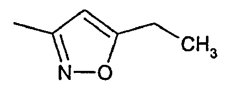 | - |
| 65 | 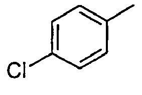 | 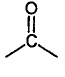 | 1 | 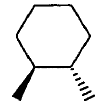 | 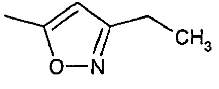 | - |
| 66 | 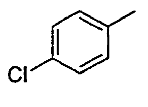 | 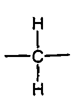 | 1 | 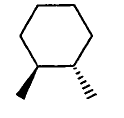 | 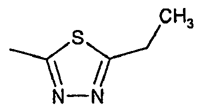 | - |
| 67 | 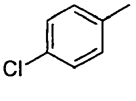 | 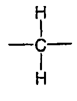 | 1 | 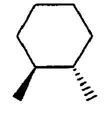 | 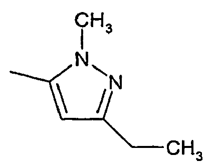 | - |
| 68 | 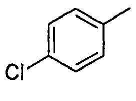 | 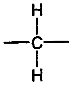 | 1 | 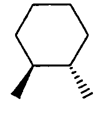 | 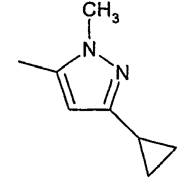 | - |
| 69 | 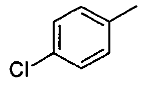 | 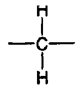 | 1 | 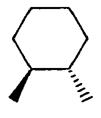 | 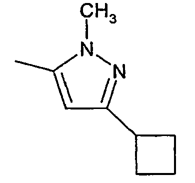 | - |
| 70 | 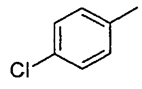 | 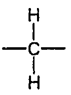 | 1 | 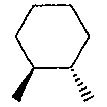 | 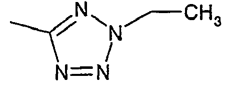 | - |
| 71 | 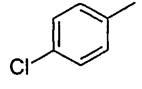 | 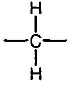 | 1 | 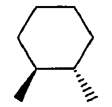 | 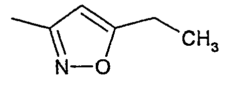 | - |
| 72 | 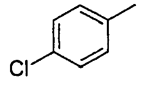 | 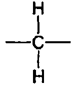 | 1 | 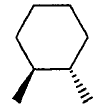 | 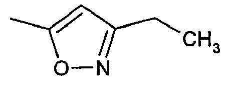 | - |
| 73 | 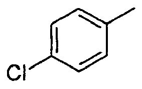 | -0- | 2 | 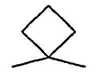 | 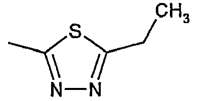 | 436.12 |
| 74 | 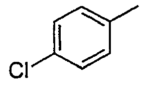 | -0- | 2 | 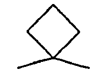 | 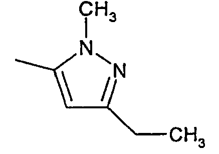 | - |
| 745 | 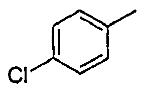 | -O- | 2 | 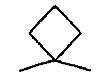 | 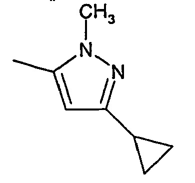 | - |
| 76 | 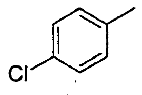 | -O- | 2 | 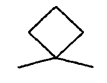 | 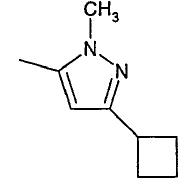 | - |
| 77 | 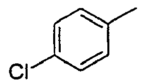 | -O- | 2 | 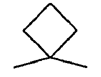 | 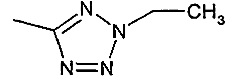 | - |
| 78 | 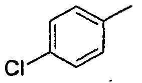 | -O- | 2 | 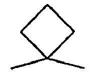 | 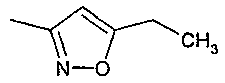 | - |
| 79 | 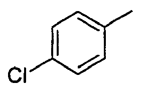 | -O- | 2 | 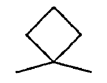 | 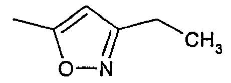 | - |
| 80 | 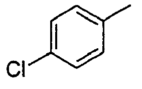 | 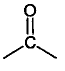 | 2 | 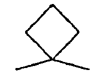 | 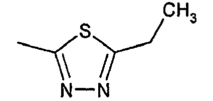 | - |
| 81 | 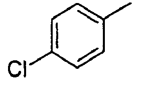 | 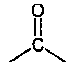 | 2 | 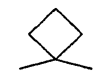 | 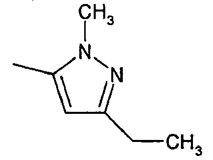 | - |
| 82 | 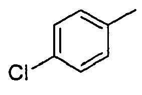 | 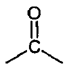 | 2 | 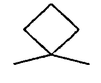 | 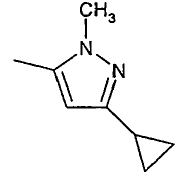 | - |
| 83 | 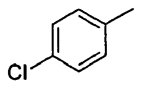 | 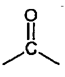 | 2 | 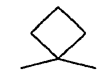 | 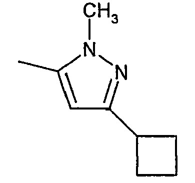 | - |
| 84 | 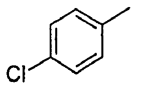 | 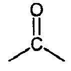 | 2 | 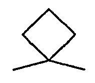 | 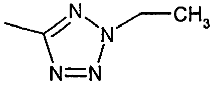 | - |
| 85 | 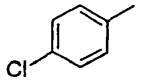 | 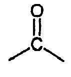 | 2 | 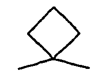 | 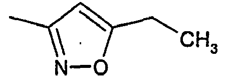 | - |
| 86 | 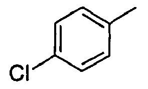 | 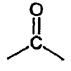 | 2 | 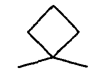 | 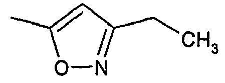 | - |
| 87 | 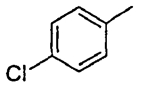 | 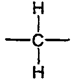 | 2 | 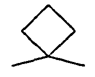 | 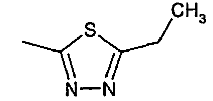 | - |
| 88 | 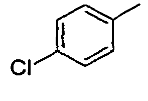 | 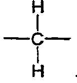 | 2 | 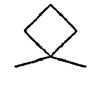 | 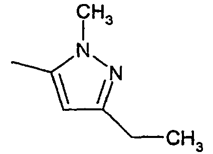 | - |
| 89 | 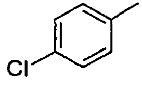 | 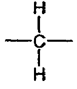 | 2 | 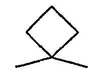 | 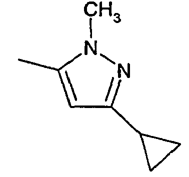 | - |
| 90 | 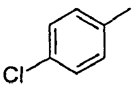 | 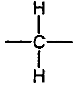 | 2 | 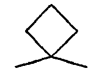 | 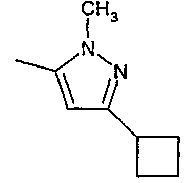 | - |
| 91 | 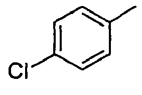 | 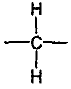 | 2 | 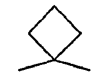 | 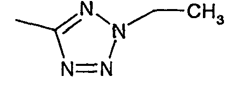 | - |
| 92 | 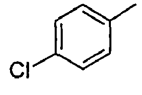 | 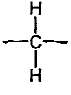 | 2 | 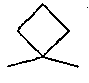 | 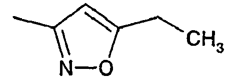 | - |
| 93 | 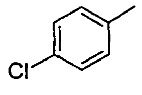 | 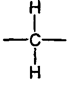 | 2 | 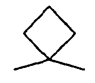 | 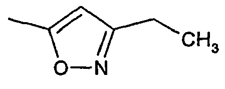 | - |
| 94 | 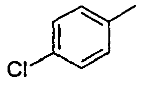 | -O- | 2 | 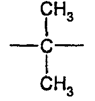 | 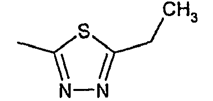 | 424.13 |
| 95 | 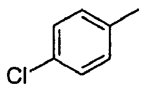 | -O- | 2 | 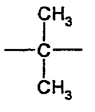 | 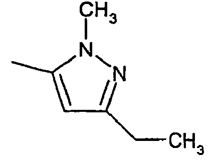 | - |
| 96 | 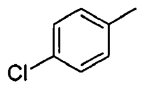 | -O- | 2 | 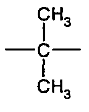 | 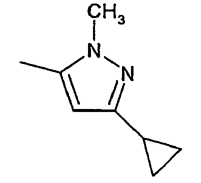 | - |
| 97 | 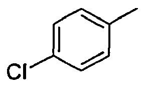 | -O- | 2 | 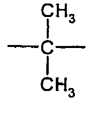 | 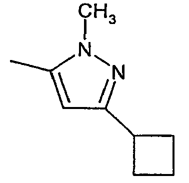 | - |
| 98 | 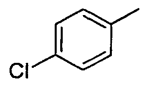 | -O- | 2 | 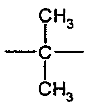 | 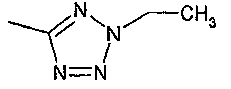 | - |
| 99 | 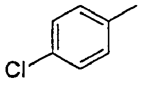 | -O- | 2 | 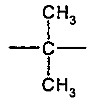 | 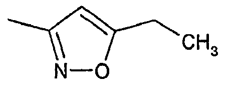 | - |
| 100 | 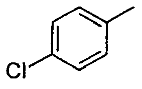 | -O- | 2 | 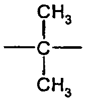 | 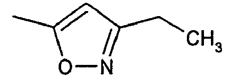 | - |
| 101 | 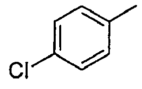 | 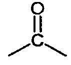 | 2 | 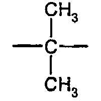 | 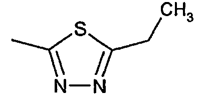 | - |
| 102 | 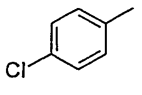 | 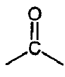 | 2 | 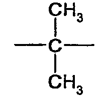 | 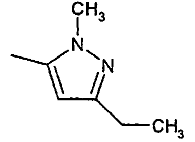 | - |
| 103 | 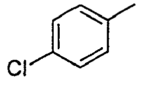 | 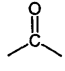 | 2 | 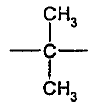 | 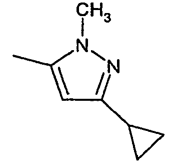 | - |
| 104 | 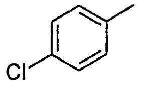 | 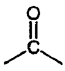 | 2 | 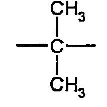 | 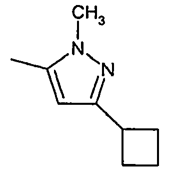 | - |
| 105 | 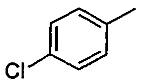 | 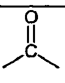 | 2 | 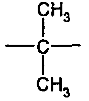 | 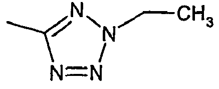 | - |
| 106 | 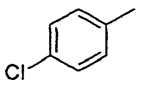 | 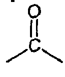 | 2 | 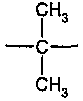 | 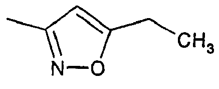 | - |
| 107 | 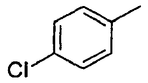 | 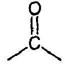 | 2 | 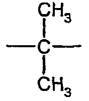 | 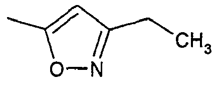 | - |
| 108 | 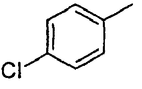 | 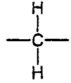 | 2 | 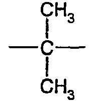 | 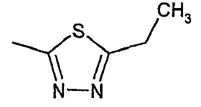 | - |
| 109 | 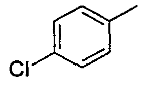 | 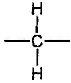 | 2 | 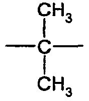 | 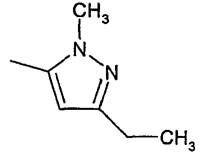 | - |
| 110 | 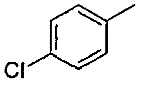 | 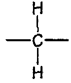 | 2 | 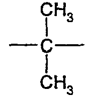 | 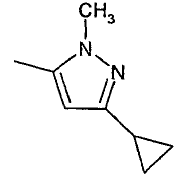 | - |
| 111 | 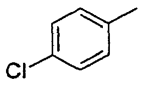 | 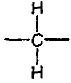 | 2 | 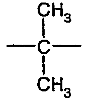 | 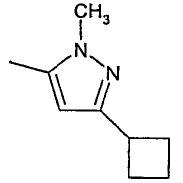 | - |
| 112 | 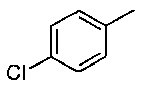 | 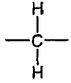 | 2 | 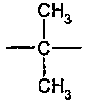 | 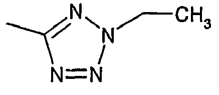 | - |
| 113 | 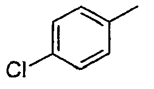 | 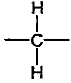 | 2 | 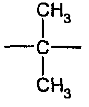 | 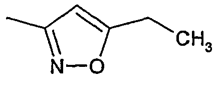 | - |
| 114 | 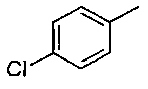 | 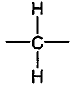 | 2 | 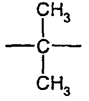 | 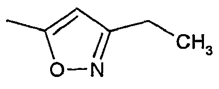 | - |
| 115 | 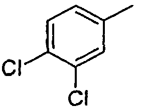 | -O- | 1 | 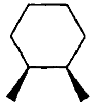 | 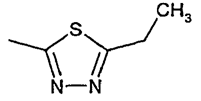 | - |
| 116 | 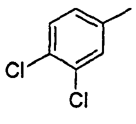 | -O- | 1 | 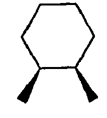 | 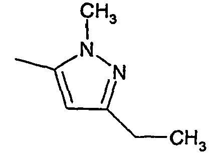 | - |
| 117 | 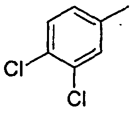 | -O- | 1 | 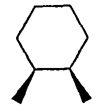 | 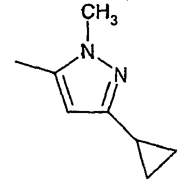 | 432.25 |
| 118 | 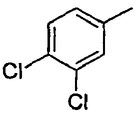 | -O- | 1 | 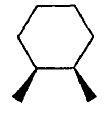 | 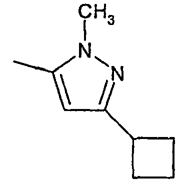 | - |
| 119 | 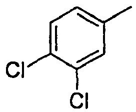 | -O- | 1 | 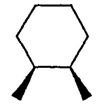 | 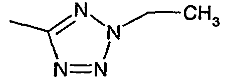 | - |
| 120 | 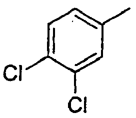 | -O- | 1 | 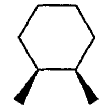 | 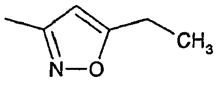 | - |
| 121 | 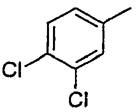 | -O- | 1 | 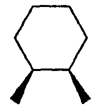 | 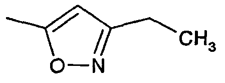 | - |
| 122 | 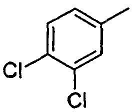 | -O- | 1 | 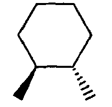 | 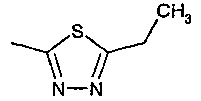 | - |
| 123 | 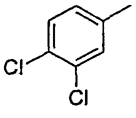 | -O- | 1 | 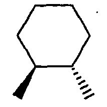 | 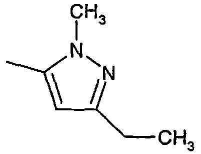 | - |
| 124 | 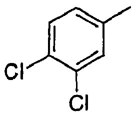 | -O- | 1 | 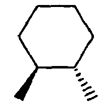 | 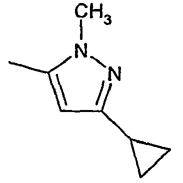 | - |
| 125 | 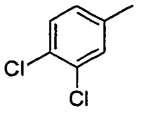 | -O- | 1 | 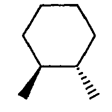 | 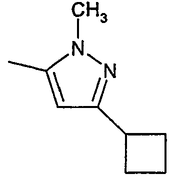 | - |
| 126 | 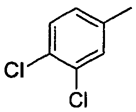 | -O- | 1 | 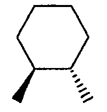 | 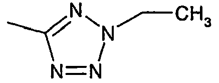 | - |
| 127 | 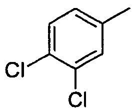 | -O- | 1 | 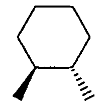 | 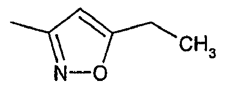 | - |
| 128 | 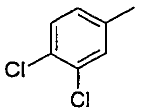 | -O- | 1 | 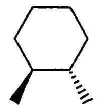 | 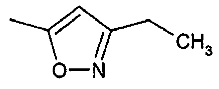 | - |
| 129 | 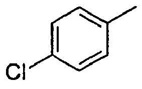 | -O- | 1 | 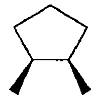 | 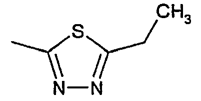 | - |
| 130 | 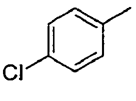 | -O- | 1 | 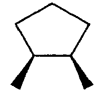 | 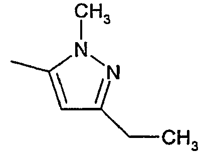 | - |
| 131 | 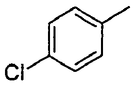 | -O- | 1 | 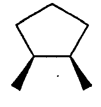 | 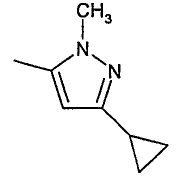 | 432.25 |
| 132 | 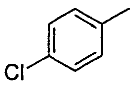 | -O- | 1 | 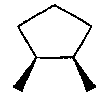 | 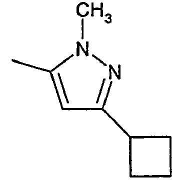 | - |
| 133 | 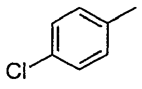 | -O- | 1 | 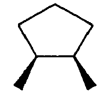 | 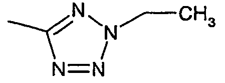 | - |
| 134 | 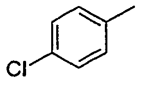 | -O- | 1 | 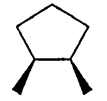 | 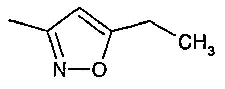 | - |
| 135 | 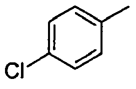 | -O- | 1 | 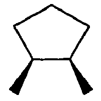 | 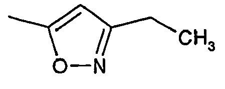 | - |
| 136 | 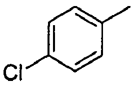 | -O- | 1 | 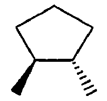 | 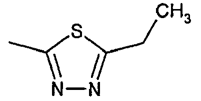 | - |
| 137 | 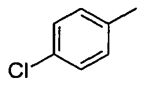 | -O- | 1 | 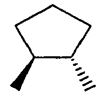 | 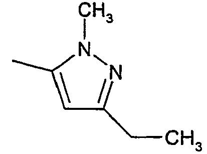 | - |
| 138 | 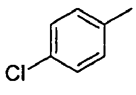 | -O- | 1 | 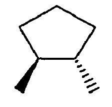 | 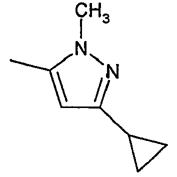 | - |
| 139 | 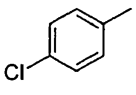 | -O- | 1 | 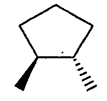 | 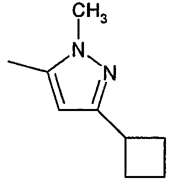 | - |
| 140 | 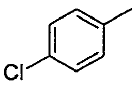 | -O- | 1 | 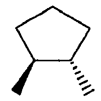 | 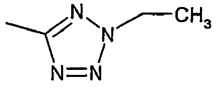 | - |
| 141 | 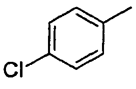 | -O- | 1 | 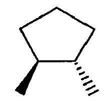 | 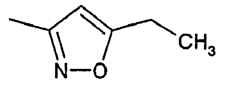 | - |
| 142 | 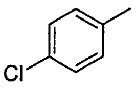 | -O- | 1 | 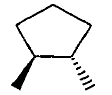 | 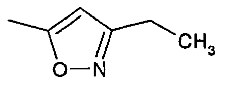 | - |

### Preparation of intermediates

### 3-(3,4-Dichloro-phenoxy)-azetidine hydrochloride:

### (a) 1-Benzhydryl-3-(3,4-dichloro-phenoxy)-azetidine

A solution of 3,4 dichlorophenol (4.12 g, 25.3 mmol) in DMF (150 ml) under argon is treated with a 60% dispersion of sodium hydride in mineral oil (40.4 mmol) and the reaction mixture stirred for 10 minutes. A solution of Methanesulfonic acid 1-benzhydryl-azetidin-3-yl ester (7.27 g, 22.96 mmol) in DMF (50 ml) is added and the reaction mixture left to stir at 60° C for 20 hours. The reaction mixture is portioned between ethyl acetate and water. The organic phase is washed with water (x2), dried over magnesium sulphate and evaporated. The crude product is purified by flash silica chromatography (elution with 1:4 ethylacetate/isohexane) to afford 1-Benzhydryl-3-(3,4-dichloro-phenoxy)-azetidine. [M+H] 383.8

### (b) 3-(3,4-Dichloro-phenoxy)-azetidine hydrochloride

A solution of 1-Benzhydryl-3-(3,4-dichloro-phenoxy)-azetidine (2.14 g, 6.4 mmol) in dry dichloromethane (20 ml) is treated with 1-chloroethylchloroformate (0.832 ml, 7.7 mmol) with stirring for 4 hours. The solvent is evaporated and the residue dissolved in methanol and refluxed for 18 hrs. The methanol is evaporated to a saturated solution, and then treated with diethylether. The resulting precipitate is filtered and dried under vacuum to afford 3-(3,4-Dichloro-phenoxy)-azetidine hydrochloride. 1H NMR (D6 DMSO, 400Mhz) δ 9.5 (2H, brS), 7.6 (1H, d), 7.2 (1H, s), 6.9 (1H, d), 5.1 (1H, m), 4.4 (2H, m), 3.95 (2H, m).

All other substituted phenoxy azetidine hydrochloride compounds, including 3-(4-chlorophenoxy)-azetidine hydrochloride, are made analogously if they are not readily available from commercial sources.

### Azetidin-3=yl-(4-chloro-phenyl)-methanone hydrochloride:

### (a) (1-Benzhydryl-azetidin-3-yl)-(4-chloro-phenyl)-methanone

A solution of 1-Benzhydryl-azetidine-3-carbonitrile (23.6 g, 95 mmol) in chlorobenzene (250 ml) under nitrogen is treated with a 1.0 M solution of 4-chlorophenylmagnesium bromide in diethylether (100 ml, 100 mmol) over one hour, ensuring the temperature does not exceed 30 °C. The stirred reaction mixture is heated to 60°C for 1 hour, then cooled back to ambient temperature and quenched with a saturated aqueous solution of ammonium chloride (250 ml). The organic phase is washed with brine, dried over magnesium sulphate, and evaporated to a yellow oil. The oil is dissolved in methanol (300 ml), treated with concentrated hydrochloric acid (25ml), and stirred at ambient temperature for 18 hours. The solvent is evaporated and the residue partitioned between ethyl acetate (250 ml) and saturated sodium bicarbonate solution (250 ml). The aqueous phase is extracted with more ethyl acetate and the combined organic phases, treated with magnesium sulphate and charcoal, filtered and evaporated to afford (1-Benzhydryl-azetidin-3-yl)-(4-chloro-phenyl)-methanone. [MH]+ 361.99

### (b) Azetidin-3-yl-(4-chloro-phenyl)-methanone hydrochloride

A solution of (1-Benzhydryl-azetidin-3-yl)-(4-chloro-phenyl)-methanone (19.8 g, 54.8 mmol) in dichloromethane (250 ml), cooled to -4°C, is treated with 1-chloroethylchloroformate (8.0 ml, 73.8 mmol) and allowed to warm to ambient temperature. The reaction mixture is stirred for 18 hours and then evaporated. The residue is dissolved in methanol (220 ml) and stirred at ambient temperature for 3.5 hours. The methanol solution is concentrated and the product precipitated by addition of diethylether. The precipitate is collected by filtration and dries under high vacuum to afford Azetidin-3-yl-(4-chloro-phenyl)-methanone hydrochloride. [MH]+ 195.95.

Azetidin-3-yl-(4-chloro-benzoyl)-methanone hydrochloride and all other substituted benzoyl azetidine compounds are made analogously.

### 3-(4-Chloro-benzyl)-azetidinium trifluoroacetate salt:

### (a) 3-(4-Chloro-benzoyl)-azetidine-1-carboxylic acid tert-butyl ester

A solution of azetidin-3-yl-(4-chloro-phenyl)-methanone hydrochloride (50 g, 210 mmol) in dioxan:water 1:1 (800 ml) is added powdered sodium bicarbonate (61.7 g, 730 mmol) and the reaction mixture cooled to 10°C. Di-tbutyl-dicarbonate (52.6 g, 240 mmol) is added portion wise and the reaction mixture allowed to warm to room temperature with stirring for 1.5 hours. The reaction mixture is poured into water (1500 ml) and the resulting white precipitate filtered off and dried under vacuum to afford 3-(4-Chloro-benzoyl)-azetidine-1-carboxylic acid tert-butyl ester. ¹H NMR 400 MHz, CDCl₃, δ 1.45 (9H), 4.10 (1H), 4.20 (4H), 7.47 (2H), 7.80 (2H)

### (b) 3-[(4-Chloro-phenyl)-hydroxy-methyl]-azetidine-1-carboxylic acid tert-butyl ester

A solution of 3-(4-Chloro-benzoyl)-azetidine-1-carboxylic acid tert-butyl ester (62.5 g, 210 mmol) in ethanol (1000 ml) cooled to 10°C is treated with sodium borohydride (9.5 g, 250 mmol). The reaction mixture is allowed to warm to room temperature and stirred for 2 hours. The reaction mixture is added to water and the precipitate collected by filtration, and dried under vacuum to afford 3-[(4-Chloro-phenyl)-hydroxy-methyl]-azetidine-1-carboxylic acid tert-butyl ester. Mpt 123-125°C.

### (c) 3-[(4-Chloro-phenyl)-iodo-methyl]-azetidine-1-carboxylic acid tert-butyl ester

Polymer supported triphenylphosphine (125 g, 370 mmol) is suspended in tetrahydrofuran: acetonitrile 9:1 (1000 ml) and treated with Iodine (95.2 g, 370 mmol) followed by stirring for 15 minutes. Imidazole (25.5 g, 370 mmol) is added followed by a solution of 3-[(4-Chlorophenyl)-hydroxy-methyl]-azetidine-1-carboxylic acid tert-butyl ester (44.7 g, 150 mmol) in tetrahydrofuran (150 ml) and the reaction mixture stirred at ambient temperature for 20 hours. The reaction mixture is filtered through Celite^{™} filter material and the filtrate evaporated. The residue is taken up in chloroform and washed with sodium thiosulphate solution, water and brine. The solution is dried over magnesium sulphate and evaporated to provide 3-[(4-Chlorophenyl)-iodo-methyl]-azetidine-1-carboxylic acid tert-butyl ester. ¹H NMR 400 MHz, CDCl₃, δ 1.35 (9H), 3.21 (1H), 3.37 (1H), 3.60 (2H), 4.05 (1H), 5.12 (1H), 7.20 (4H).

### (d) 3-(4-Chloro-benzyl)-azetidine-1-carboxylic acid tert-butyl ester

A solution of 3-[(4-Chloro-phenyl)-iodo-methyl]-azetidine-1-carboxylic acid tert-butyl ester (58 g, 140 mmol) in dimethylsulphoxide (450 ml) is treated with sodium borohydride with cooling. The reaction mixture was stirred at room temperature for 20 hours, then quenched by the slow addition of water (1000 ml). The aqueous mixture is extracted into ethyl acetate, and the ethyl acetate phase washed with saturated brine, dried over magnesium sulphate and evaporated. The crude product is purified by flash chromatography using a biotage 75 column (eluant gradient isohexane:ethylacetate 9:1 to 85:15) to afford 3-(4-Chloro-benzyl)-azetidine-1-carboxylic acid tert-butyl ester. ¹H NMR 400 MHz, CDCl₃, δ 1.35 (9H), 2.70 (1H), 2.80 ( 2H), 3.55 (2H), 3.90 (2H), 6.97 (2H), 7.17 (2H).

### (e) 3-(4-Chloro-benzyl)-azetidinium trifluoroacetate salt

A solution of 3-(4-Chloro-benzyl)-azetidine-1-carboxylic acid tert-butyl ester (1.61 g, 5.71 mmol) in dichloromethane (20 ml) is treated with trifluoroacetic acid (20 ml) and stirred at ambient temperature for 1 hour. The reaction mixture is evaporated and then re-suspended in toluene and evaporated to dryness to afford 3-(4-Chloro-benzyl)-azetidinium trifluoroacetate salt. [MH]+ of free base 182.12.

All other substituted benzyl azetidine compounds are made analogously.

### (5-Cyclopropyl-2-methyl-2H-pyrazol-3-yl)-carbamic acid phenyl ester:

A solution of 5-Cyclopropyl-2-methyl-2H-pyrazol-3-ylamine (3.0 g, 22 mmol) (65 ml) and sodium bicarbonate (2 g, 24 mmol) in THF is cooled to 0°C. Phenylchloroformate (3.4 g, 22 mmol) is added dropwise over 15 minutes. The reaction mixture is allowed to warm to ambient temperature and stirred for 5 hours, then filtered and the filtrate partitioned between ethyl acetate and water. The organic phase is washed with water, 5% aqueous citric acid solution and brine, dried over MgSO₄ and evaporated to afford (5-Cyclopropyl-2-methyl-2H-pyrazol-3-yl)-carbamic acid phenyl ester. [M+H] 258.17.

### (+/-)(1R,2R)-2-[3-(4-Chloro-phenoxy)-azetidin-1-ylmethyl]-cyclohexylamine;

### (a) (+/-){(1R,2R)-2-[3-(4-Chloro-phenoxy)-azetidine-1-carbonyl]-cyclohexyl}-carbamic acid tert-butyl ester

A solution of BOC-Cis-2amino-cyclohexylcarboxylic acid (1.0 g, 4.11mmol) and diisopropylethylamine (2.14ml, 12/33mmol) in dichloromethane is treated with [dimethylamino-([1,2,3]triazolo[4,5-b]pyridin-3-yloxy)-methylene]-dimethyl-ammonium hexafluoro phosphate (1.56g, 4.11mmol) and stirred at ambient temperature for 5 minutes. 3-(4-Chloro-phenoxy)-azetidine hydrochloride (0.984g, 4.32mmol) is added and the reaction mixture stirred for 18hours. The reaction mixture is diluted with dichloromethane and washed with saturated sodium bicarbonate solution and brine. The organic phase is dried over magnesium sulphate and evaporated. The crude product is purified by flash chromatography (eluant gradient 30% ethyl acetate in isohexane) to afford {(1R,2R)-2-[3-(4-Chloro-phenoxy)-azetidine-1-carbonyl]-cyclohexyl}-carbamic acid tert-butyl ester as a racemic mixture of optical isomers [M-BOC] 309.16

### (b) (+/-){(1R,2R)-2-[3-(4-Chloro-phenoxy)-azetidin-1-ylmethyl]-cyclohexyl}-carbamic acid tert-butyl ester

A solution of {(1R,2R)-2-[3-(4-Chloro-phenoxy)-azetidine-1-carbonyl]-cyclohexyl}-carbamic acid tert-butyl ester (0.215 g, 0.52 mmol) in dry THF (5ml) cooled to -5°C is treated with a 1.0 M solution of LiA1H4 in THF (1.26 ml, 1.26 mmol) and the reaction mixture stirred at -5°C for 20 minutes and then for a further 1 hour at ambient temperature. The reaction mixture is quenched with saturated sodium sulphate solution and filtered through a Celite^{™} filter pad washing through with ethyl acetate. The filtrate is dried over magnesium sulphate and evaporated. The crude product is purified by flash chromatography (eluant 40% Ethyl acetate in isohexane) to afford {(1R,2R)-2-[3-(4-Chloro-phenoxy)-azetidin-1-ylmethyl]-cyclohexyl}-carbamic acid tert-butyl ester as a racemic mixture of optical isomers [MH]+ 395.23

### (c) (+/-)(1R,2R)-2-[3-(4-Chloro-phenoxyl-azetidin-1-ylmethyl]-cyclohexylamine

A solution of {(1R,2R)-2-[3-(4-Chloro-phenoxy)-azetidin-1-ylmethyl]-cyclohexyl}-carbamic acid tert-butyl ester (0.078 g, 0.199 mmol) in dichloromethane (1 ml) is treated with trifluoroacetic acid (0.3ml) and the reaction mixture stirred at ambient temperature for 30 minutes. The solvent is evaporated and the residue taken up in dichloromethane and washed with 1 M NaOH solution. The organic phase is dried over magnesium sulphate and evaporated to afford (1R,2R)-2-[3-(4-Chloro-phenoxy)-azetidin-1-ylmethyl]-cyclohexylamine as a racemic mixture of optical isomers. [MH]+ 295.19

### 1-{2-[3-(4-Chloro-phenoxy)-azetidin-1-yl]-ethyl}-cyclobutylamine:

### (a) (1-{2-[3-(4-Chloro-phenoxy)-azetidin-1-yl]-2-oxo-ethyl}-cyclobutyl)-carbamic acid tert-butyl ester

A solution of (1-tert-Butoxycarbonylamino-cyclobutyl)-acetic acid (0.13 g, 0.567 mmol) ( synthesized by the method described in European Journal of Medicinal Chemistry (1999), 34(5), 363-380), and diisopropylethylamine (0.197 ml, 1.134 mmol) in dichloromethane is treated with [Dimethylamino-([1,2,3]triazolo[4,5-b]pyridin-3-yloxy)-methylene]-dimethylammonium hexafluoro phosphate (0.216 g, 0.567 mmol) and stirred at ambient temperature for 5 minutes. 3-(4-Chloro-phenoxy)-azetidine hydrochloride is added and the reaction mixture stirred for 18 hours. The reaction mixture is diluted with dichloromethane and washed with saturated sodium bicarbonate solution and brine. The organic phase is dried over magnesium sulphate and evaporated. The crude product is purified by flash chromatography (eluant gradient isohexane:ethylacetate 3:7 to 2:8) to afford (1-{2-[3-(4-Chloro-phenoxy)-azetidin-1-yl]-2-oxo-ethyl}-cyclobutyl)-carbamic acid tert-butyl ester. [MH]+ 395.16.

### (b) (1-{2-[3-(4-Chloro-phenoxy)-azetidin-1-yl]-ethyl}-cyclobutyl)-carbamic acid tert-butyl ester

A solution of (1-{2-[3-(4-Chloro-phenoxy)-azetidin-1-yl]-2-oxo-ethyl}-cyclobutyl)-carbamic acid tert-butyl ester (0.16 g, 0.405 mmol) in dry THF (5 ml) cooled to 0°C is treated with a 1.0M solution of LiAIH₄ in THF (1.0 ml, 1.0 mmol) and the reaction mixture stirred at 0°C for 20 minutes and then for a further 1 hour at ambient temperature. The reaction mixture is quenched with saturated sodium sulphate solution and filtered through a Celite^{™} filter pad washing through with ethylacetate. The filtrate is dried over magnesium sulphate and evaporated. The crude product is purified by flash chromatography (eluant gradient dichloromethane:methanol 1:0 to 9:1) to afford (1-[2-[3-(4-Chloro-phenoxy)-azetidin-1-yl]-ethyl}-cyclobutyl)-carbamic acid tert-butyl ester. [MH]+ 381.20.

### (c) 1-{2-[3-(4-Chloro-phenoxy)-azetidin-1-yl]-ethyl}-cyclobutylamine

A solution of (1-{2-[3-(4-Chloro-phenoxy)-azetidin-1-yl]-ethyl}-cyclobutyl)-carbamic acid tert-butyl ester (0.04g, 0.105mmol) in dichloromethane (3ml) is treated with trifluoroacetic acid ( 0.5ml) and the reaction mixture stirred at ambient temperature for 2 hours. The solvent is evaporated and the residue taken up in dichloromethane and washed with 1M NaOH solution. The organic phase is dried over magnesium sulphate and evaporated to afford 1-{2-[3-(4-Chloro-phenoxy)-azetidin-1-yl]-ethyl}-cyclobutylamine. [MH]+ 281.11

### 3-[3-(4-Chloro-phenoxy)-azetidin-1-yl]-1,1-dimethyl-propylamine and {3-[3-(4-Chloro-phenoxy)-azetidin-1-yl]-1,1-dimethyl-propyl}-methyl-amine:

### (a) {3-[3-(4-Chloro-phenoxy)-azetidin-1-yl]-1,1-dimethyl-3-oxo-propyl}-carbamic acid tert-butyl ester

A solution of 3-tert-Butoxycarbonylamino-3-methyl-butyric acid (0.58g, 2.67mmol) ( synthesized by the method described in Journal of Medicinal Chemistry (1991), 34(2), 633-42.), and diisopropylethylamine (1.39ml, 8.01mmol) in dichloromethane is treated with [dimethylamino-([1,2,3]triazolo[4,5-b]pyridin-3-yloxy)-methylene]-dimethyl-ammonium hexafluoro phosphate (1.01g, 2.67mmol) and stirred at ambient temperature for 5 minutes. 3-(4-Chloro-phenoxy)-azetidine hydrochloride (0.68g, 2.67mmol) is added and the reaction mixture stirred for 18hours. The reaction mixture is diluted with dichloromethane and washed with saturated sodium bicarbonate solution and brine. The organic phase is dried over magnesium sulphate and evaporated. The crude product is purified by flash chromatography (eluant gradient 25% to 50% ethylacetate: isohexane) to afford {3-[3-(4-Chloro-phenoxy)-azetidin-1-yl]-1,1-dimethyl-3-oxo-propyl}-carbamic acid tert-butyl ester [M-BOC] 283.08.

### (b) 3-[3-(4-Chloro-phenoxy)-azetidin-1-yl]-1,1-dimethyl-propylamine

A solution of {3-[3-(4-Chloro-phenoxy)-azetidin-1-yl]-1,1-dimethyl-3-oxo-propyl}-carbamic acid tert-butyl ester (0.587g, 1.53mmol) in dry THF (5ml) cooled to -5oC is treated with a 1.0M solution of LiAlH₄ in THF (3.83ml, 3.83mmol) and the reaction mixture stirred at OoC for 30 minutes and then for a further 1 hour at ambient temperature. The reaction mixture is quenched with saturated sodium sulphate solution and filtered through a Celite^{™} filter pad washing through with ethyl acetate. The filtrate is dried over magnesium sulphate and evaporated. The crude product is purified by flash chromatography (eluant gradient dichloromethane:methanol 9:1 to 9:3) to afford as a 1:1 mixture of 3-[3-(4-Chloro-phenoxy)-azetidin-1-yl]-1,1-dimethyl-propylamine. [MN]+ 269.1 and {3-[3-(4-Chloro-phenoxy)-azetidin-1-yl]-1,1-dimethyl-propyl}-methyl-amine [MH]+ 283.12.

### Preparation of final compounds

### Example 31

### (+/-)1-{(1R,2R)-2-[3-(4-Chloro-1-phenoxy)-azetidin-1-yl-methyl]-cyclohexyl}-3-(5-ethyl-[1,3,4]thiadiazol-2-yl)-urea:

A solution of (1R,2R)-2-[3-(4-Chloro-phenoxy)-azetidin-1-ylmethyl]-cyclohexylamine (0.111 mg, 0.382 mmol) and (5-Ethyl-[1,3,4]thiadiazol-2-yl)-carbamic acid phenyl ester (0.100 g, 0.40 mmol) in dimethylsulphoxide (2 ml) is stirred at ambient temperature form 18 hours. The reaction mixture is partitioned between ethyl acetate and water. The organic phase is dried over magnesium sulphate and evaporated. The crude product is purified by flash chromatography (eluant ethyl acetate) to afford 1-{(1R,2R)-2-[3-(4-Chloro-phenoxy)-azetidin-1-ylmethyl]-cyclohexyl}-3-(5-ethyl-[1,3,4]thiadiazol-2-yl)-urea as a racemic mixture of optical isomers. [MH]+ 450.213

### Examples 32 to 37

The compounds of these Examples, namely 1-{(1R,2R)-2-[3-(4-Chloro-phenoxy)-azetidin-1-yl-methyl]-cyclohexyl}-3-(5-ethyl-2-methyl-2H-pyrazol-3-yl)-urea, 1-{(1R,2R)-2-[3-(4-Chloro-phenoxy)-azetidin-1-yl-methyl]-cyclohexyl}-3-(5-cyclopropyl-2-methyl-2H-pyrazol-3-yl)-urea, 1-{(1R,2R)-2-[3-(4-Chloro-phenoxy)-azetidin-1-yl-methyl]-cyclohexyl}-3-(5-cyclobutyl-2-methyl-2H-pyrazol-3-yl)-urea, 1-{(1R,2R)-2-[3-(4-Chloro-phenoxy)-azetidin-1-yl-methyl]-cyclohexyl}-3-(2-ethyl-2H-tetrazol-5-yl)-urea, 1-{(1R,2R)-2-[3-(4-Chloro-phenoxy)-azetidin-1-yl-methyl)-cyclohexyl}-3-(5-ethyl-isoxazol-3-yl)-urea and 1-{(1R,2R)-2-[3-(4-Chloro-phenoxy)-azetidin-1-yl-methyl]-cyclohexyl}-3-(3-ethyl-isoxazol-5-yl)-urea are prepared using procedures that are analogous to those used in Example 31.

### Examples 38 to 51

The compounds of these Examples are prepared using procedures that are analogous to those used to prepare the compounds of Examples 31 to 37 but using the appropriate amine.

### Examples 52 to 72

The compounds of these Examples are trans analogues of the compounds of Examples 31 to 51 respectively. They are prepared analogously but using the intermediate BOC-trans-2-amino-cyclohexylcarboxylic acid instead of BOC-cis-2-amino-cyclohexylcarboxylic acid.

### Example 73

### 1-(1-{2-[3-(4-Chloro-phenoxy)-azetidin-1-yl]-ethyl}-cyclobutyl)-3-(5-ethyl-[1,3,4]thiadiazol-2-yl)-urea :

A solution of 1-{2-[3-(4-Chloro-phenoxy)-azetidin-1-y1]-ethyl}-cyclobutylamine (0.028 mg, 0.1 mmol) and (5-Ethyl-[1,3,4]thiadiazol-2-yl)-carbamic acid phenyl ester (0.028 g, 0.112 mmol) in dimethylsulphoxide (1.5 ml) is stirred at ambient temperature form 18 hours. The reaction mixture is partitioned between ethyl acetate and water. The organic phase is dried over magnesium sulphate and evaporated. The crude product is purified by flash chromatography (eluant gradient dichloromethane:methanol 98:2 to 93:7) to afford 1-(1-12-[3-(4-Chloro-phenoxy)-azetidin-1-yl]-ethyl)-cyclobutyl)-3-(5-ethyl-[1,3,4]thiadiazol-2-yl)-urea. [MH]+ 436.12.

### Examples 74 to 79

The compounds of these Examples, namely 1-(1-{2-[3-(4-Chloro-phenoxy)-azetidin-1-yl]-ethyl}-cyclobutyl)-3-(5-ethyl-2-methyl-2H-pyrazol-3-yl)-urea, 1-(1-{2-[3-(4-Chloro-phenoxy)-azetidin-1-yl]-ethyl}-cyclobutyl)-3-(5-cyclopropyl-2-methyl-2H-pyrazol-3-yl)-urea, 1-(1-{2-[3-(4-Chloro-phenoxy)-azetidin-1-yl]-ethyl}-cyclobutyl)-3-(5-cyclobutyl-2-methyl-2H-pyrazol-3= yl)-urea, 1-(1-{2-[3-(4-Chloro-phenoxy)-azetidin-1-yl]-ethyl}-cyclobutyl)-3-(2-ethyl-2H-tetrazol-5-yl)-urea, 1-(1-{2-[3-(4-Chloro-phenoxy)-azetidin-1-yl]-ethyl}-cyclobutyl)-3-(5-ethyl-isoxazol-3-yl)-urea and 1-(1-{2-[3-(4-Chloro-phenoxy)-azetidin-1-yl]-ethyl}-cyclobutyl)-3-(3-ethyl-isoxazol-5-yl)-urea are prepared using procedures to those used in Example 73.

### Examples 80 to 93

The compounds of these Examples are prepared using procedures that are analogous to those used to prepare the compounds of Examples 73 to 79 but using the appropriate amine.

### Example 94

### 1-{3-[3-(4-Chloro-phenoxy)-azetidin-1-yl]-1,1-dimethyl-propyl}-3-(5-ethyl-[1,3,4]thiadiazol-2-yl)-urea:

A solution of a 1:1 mixture of 3-[3-(4-Chloro-phenoxy)-azetidin-1-yl]-1,1-dimethylpropylamine and {3-[3-(4-Chloro-phenoxy)-azetidin-1-yl]-1,1-dimethyl-propyl}-methyl-amine (0.069 mg) and 5-Ethyl-[1,3,4]thiadiazol-2-yl)-carbamic acid phenyl ester (0.067g, 0.26 mmol) in dimethylsulphoxide (2.0 ml) is stirred at ambient temperature form 18 hours. The reaction mixture is partitioned between ethyl acetate and water. The organic phase is dried over magnesium sulphate and evaporated. The crude product is purified by flash chromatography (eluant gradient dichloromethane:methanol 98:2 to 90:10) to afford 1-{3-[3-(4-Chloro-phenoxy)-azetidin-1-yl]-1,1-dimethyl-propyl}-3-(5-ethyl-[1,3,4]thiadiazol-2-yl)-urea. [MH]+ 424.13.

### Examples 95 to 100

The compounds of these Examples, namely 1-{3-[3-(4-Chloro-phenoxy)-azetidin-1-yl]-1,1-dimethyl-propyl}-3-(5-ethyl-2-methyl-2H-pyrazol-3-yl)-urea, 1-{3-[3-(4-Chloro-phenoxy)-azetidin-1-yl]-1,1-dimethyl-propyl}-3-(5-cyclopropyl-2-methyl-2H-pyrazol-3-yl)-urea, 1-{3-[3-(4-Chloro-phenoxy)-azetidin-1-yl]-1,1-dimethyl-propyl}-3-(5-cyclobutyl-2-methyl-2H-pyrazol-3-yl)-urea, 1-{3-[3-(4-Chloro-phenoxy)-azetidin-1-yl]-1,1-dimethyl-propyl}-3-(2-ethyl-2H-tetrazol-5-yl)-urea, 1-{3-[3-(4-Chloro-phenoxy)-azetidin-1-yl]-1,1-dimethyl-propyl}-3-(5-ethyl-isoxazol-3-yl)-urea and 1-{3-[3-(4-Chloro-phenoxy)-azetidin-1-yl]-1,1-dimethyl-propyl}-3-(3-ethyl-isoxazol-5-yl)-urea are prepared using procedures to those used in Example 94.

### Examples 101 to 114

The compounds of these Examples are prepared using procedures that are analogous to those used to prepare the compounds of Examples 94 to 100 but using the appropriate amine.

### Examples 115 to 98

The compounds of these Examples are prepared using procedures that are analogous to those used to prepare the compounds of Examples 31 to 37 and 52 to 58 respectively but using the appropriate starting materials.

### Examples 129 to 142

The compounds of these Examples are prepared using procedures that are analogous to those used to prepare the compounds of Examples 31 to 37 and 52 to 58 respectively but using the appropriate starting materials.

## Claims

1. A compound of formula Ia or Ib 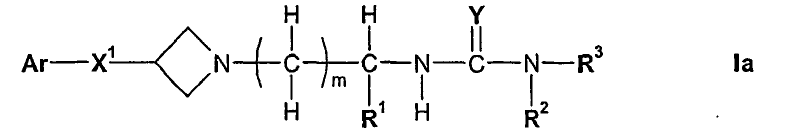 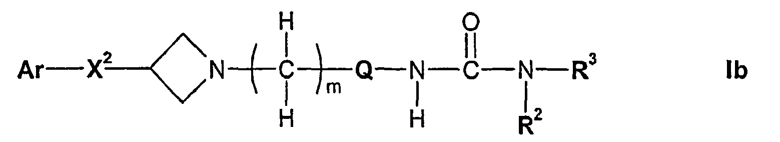
in free or salt form, where
Ar is phenyl optionally substituted by one or more substituents selected from halogen,
C₁-C₈-alkyl, cyano or nitro;
X¹ is -S-, -S(=O)- or -S(=O)₂-;
X² is -C(=O)-, -O-, -CH₂-, -S-, -S(=O)- or -S(=O)₂-;
m is 1, 2, 3 or 4;
R¹ is hydrogen or C₁-C₈-alkyl optionally substituted by hydroxy, C₁-C₈-alkoxy, acyloxy, halogen, carboxy, C₁-C₈-alkoxycarbonyl, -N(R⁴)R⁵, -CON(R⁶)R⁷ or by a monovalent cyclic organic group having 3 to 15 atoms in the ring system;
Q has the formula 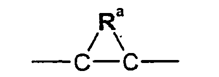
where Rₐ is C₁-C₈-alkylene,
or Q is -C(R^{b})(R^{c})- where R^{b} and R^{c} are independently C₁-C₈-alkyl
or R^{b} and R^{c} together form a C₃-C₁₀-cycloalkyl;
Y is oxygen or sulfur;
R² is hydrogen, C₁-C₈-alkyl or C₃-C₁₀-cycloalkyl and R³ is C₁-C₈-alkyl substituted by phenyl, phenoxy, acyloxy or naphthyl, or R³ is C₃-C₁₀-cycloalkyl optionally having a benzo group fused thereto, a heterocyclic group having 5 to 11 ring atoms of which 1 to 4 are hetero atoms, phenyl or naphthyl, said phenyl, phenoxy or naphthyl groups being optionally substituted by one or more substituents selected from halogen, cyano, hydroxy, acyl, nitro, -SO₂NH₂, C₁-C₈-alkyl optionally substituted by C₁-C₈-alkoxy, C₁-C₈-haloalkyl, C₁-C₈-alkoxy, C₁-C₈-haloalkoxy, C₁-C₈-alkylthio, -SO₂-C₁-C₈-alkyl, C₁-C₈-alkoxycarbonyl, C₁-C₈-acylamino optionally substituted on the nitrogen atom by C₁-C₈-alkyl, C₁-C₈-alkylamino, aminocarbonyl,
C₁-C₈-alkylamino-carbonyl, di(C₁-C₈-alkyl)amino, di(C₁-C₈-alkyl)aminocarbonyl, di(C₁-C₈-alkyl)aminocarbonyl-methoxy,
or R² and R³ together with the nitrogen atom to which they are attached denote a heterocyclic group having 5 to 10 ring atoms of which 1, 2 or 3 are hetero atoms;
R⁴ and R⁵ are each independently hydrogen or C₁-C₈-alkyl, or R⁴ is hydrogen and R⁵ is hydroxy-C₁-C₈-alkyl, acyl, -SO₂R⁸ or -CON(R⁶)R⁷, or R⁴ and R⁵ together with the nitrogen atom to which they are attached denote a 5-or 6-membered heterocyclic group;
R⁶ and R⁷ are each independently hydrogen or C₁-C₈-alkyl, or R⁶ and R⁷ together with the nitrogen atom to which they are attached denote a 5- or 6-membered heterocyclic group; and
R⁸ is C₁-C₈alkyl, C₁-C₈-haloalkyl, or phenyl optionally substituted by C₁-C₈-alkyl.

2. A compound according to claim 1, which is
(i) a compound of formula Ia in free or salt form, wherein
Ar is phenyl substituted by halo;
X¹ is -S-, -S(=O)- or -S(=O)₂-;
m is 2;
R¹ is C₁-C₈-alkyl optionally substituted by hydroxy or C₁-C₈-alkoxy;
Y is oxygen;
R² is hydrogen; and
R³ is a heterocyclic group having 5 to 11 ring atoms of which 1 to 4 are hetero atoms; or
(ii) a compound of formula Ib in free or salt form, wherein
Ar is phenyl substituted by halo;
X² is -O-, -C(=O)- or -CH₂-;
m is 1 or 2;
Q has the formula 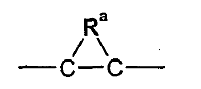 where R^{a} is C₁-C₈-alkylene,
or Q is -C(R^{b}) (R^{c})- where R^{b} and R^{c} are independently C₁-C₈-alkyl
or R^{b} and R^{c} together form a C₃-C₁₀-cycloalkyl;
R² is hydrogen; and
R³ is a heterocyclic group having 5 to 11 ring atoms of which 1 to 4 are hetero atoms.

3. A compound according to claim 1, which is
(i) a compound of formula Ia in free or salt form, wherein
Ar is phenyl substituted by halo, preferably chloro;
X¹ is -S-, -S(=O)- or-S(=O)₂-;
m is 2;
R¹ is C₁-C₄-alkyl optionally substituted by hydroxy or C₁-C₄-alkoxy;
Y is oxygen;
R² is hydrogen; and
R³ is a heterocyclic group having 5, 6 or 7 ring atoms of which one, two, three or four, are hetero atoms selected from nitrogen, oxygen and sulphur, said heterocyclic group being optionally substituted by C₁-C₄-alky, C₁-C₄-alkoxy or C₃-C₆-cycloalkyl; or
(ii) a compound of formula Ib in free or salt form, wherein
Ar is phenyl substituted by halo, preferably chloro;
X² is -O-, -C(=O)- or -CH₂-;
m is 1 or 2;
Q has the formula 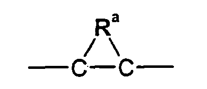 where R^{a} is C₁-C₈-alkylene,
or Q is -C(R^{b})(R^{c})- where R^{b} and R^{c} are independently C₁-C₄-alkyl
or R^{b} and R^{c} together form a C₃-C₆-cycloalkyl;
R² is hydrogen; and
R³ is a heterocyclic group having 5, 6 or 7 ring atoms of which one, two, three or four, are hetero atoms selected from nitrogen, oxygen and sulphur, said heterocyclic group being optionally substituted by C₁-C₄-alkyl or C₃-C₆-Cycloalkyl.

4. A compound according to claim 1 that is also either a compound of formula XX 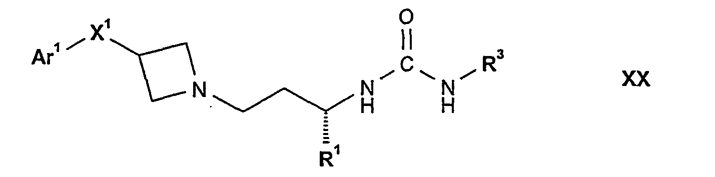 where Ar, X¹ and R³ are as shown in the following table:
| Ar | X | R¹ | R³ |
|---|---|---|---|
| 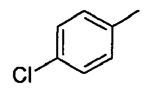 | 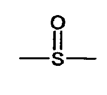 | 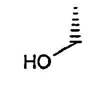 | 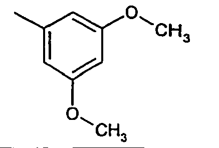 |
| 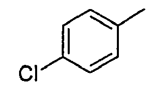 | 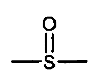 | 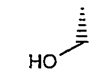 | 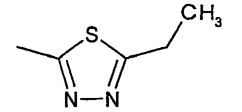 |
| 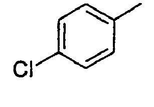 | 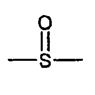 | 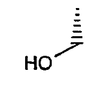 | 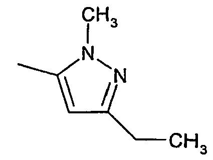 |
| 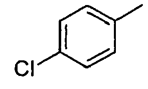 | 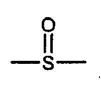 | 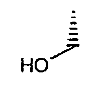 | 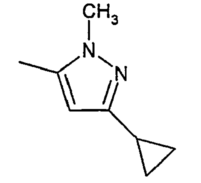 |
| 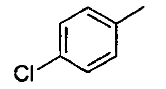 | 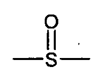 | 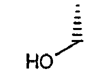 | 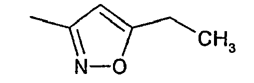 |
| 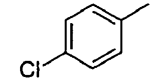 | 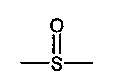 | 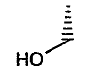 | 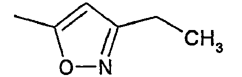 |
| 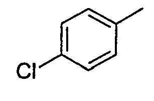 | 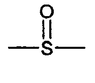 | 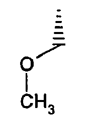 | 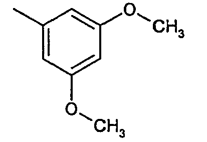 |
| 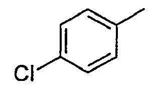 | 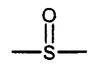 | 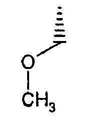 | 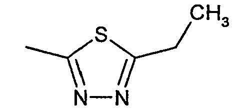 |
| 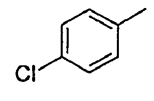 | 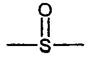 | 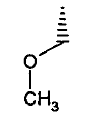 | 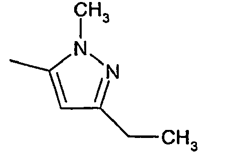 |
| 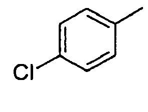 | 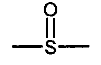 | 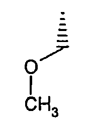 | 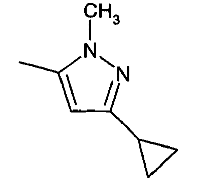 |
| 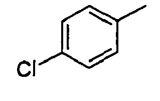 | 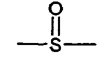 | 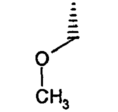 | 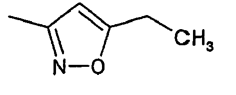 |
| 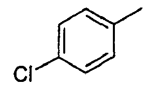 | 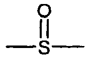 | 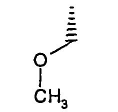 | 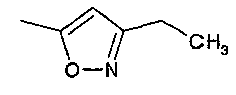 |
| 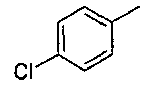 | 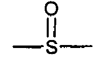 | 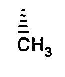 | 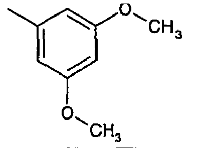 |
| 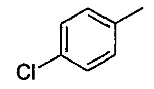 | 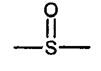 | 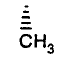 | 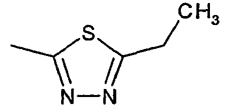 |
| 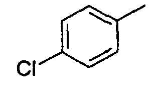 | 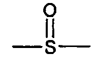 | 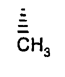 | 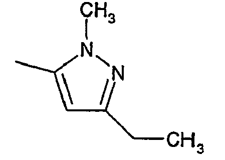 |
| 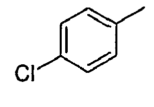 | 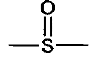 | 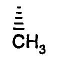 | 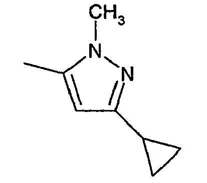 |
| 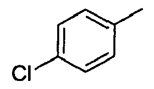 | 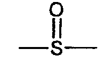 | 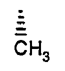 | 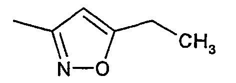 |
| 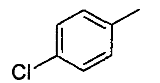 | 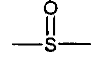 | 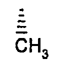 | 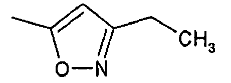 |
| 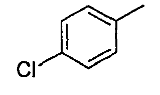 | -s- | 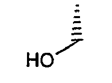 | 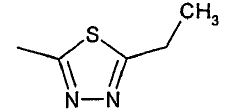 |
| 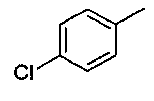 | -s- | 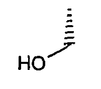 | 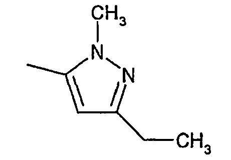 |
| 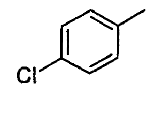 | -s- | 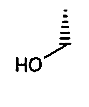 | 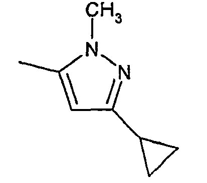 |
| 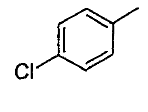 | -s- | 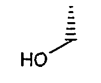 | 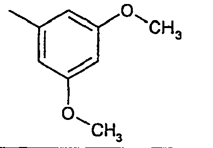 |
| 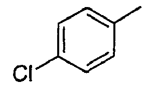 | -s- | 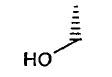 | 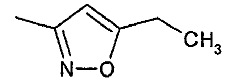 |
| 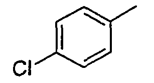 | -s- | 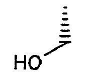 | 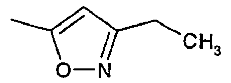 |
| 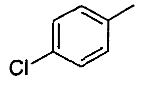 | 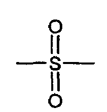 | 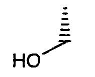 | 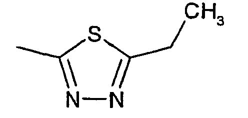 |
| 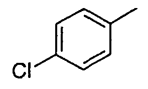 | 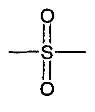 | 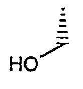 | 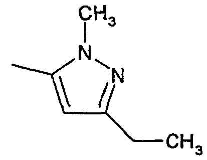 |
| 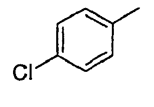 | 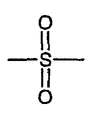 | 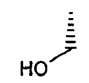 | 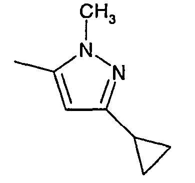 |
| 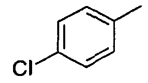 | 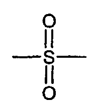 | 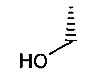 | 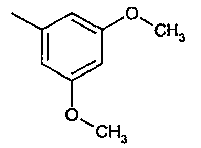 |
| 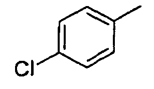 | 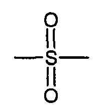 | 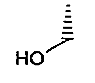 | 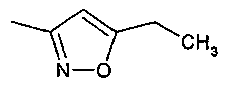 |
| 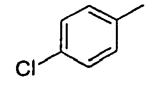 | 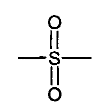 | 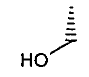 | 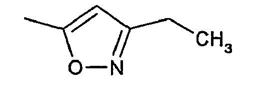 |
or a compound of formula XXI 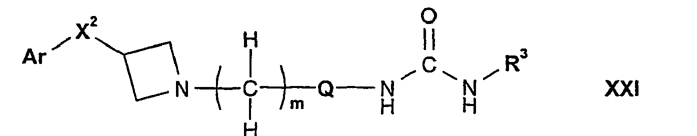 where Ar, X², m, Q and R³ are as shown in the following table:
| Ar | X | m | -Q- | R³ |
|---|---|---|---|---|
| 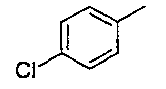 | -O- | 1 | 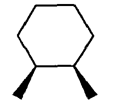 | 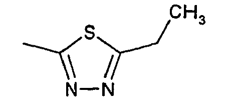 |
| 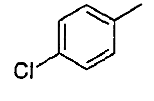 | -O- | 1 | 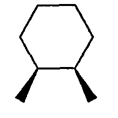 | 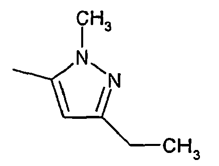 |
| 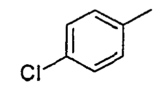 | -O- | 1 | 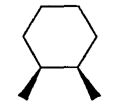 | 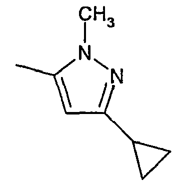 |
| 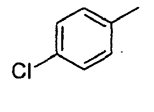 | -O- | 1 | 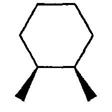 | 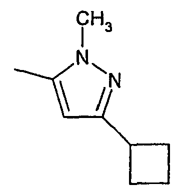 |
| 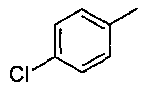 | -O- | 1 | 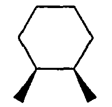 | 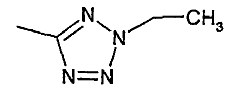 |
| 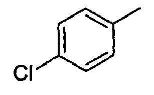 | -O- | 1 | 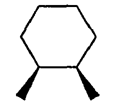 | 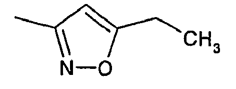 |
| 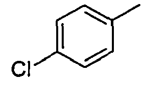 | -O- | 1 | 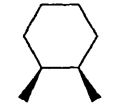 | 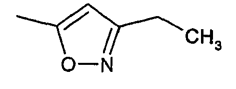 |
| 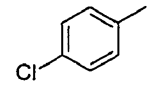 | 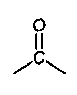 | 1 | 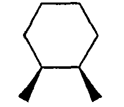 | 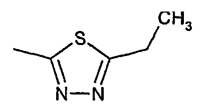 |
| 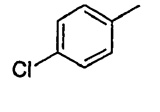 | 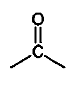 | 1 | 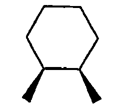 | 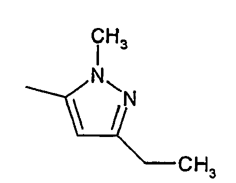 |
| 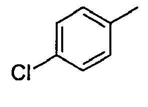 | 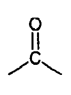 | 1 | 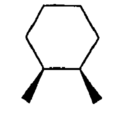 | 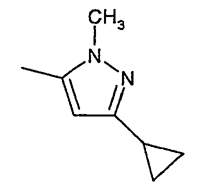 |
| 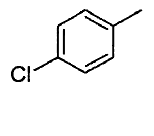 | 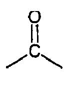 | 1 | 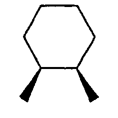 | 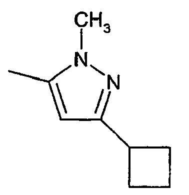 |
| 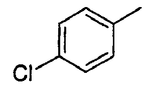 | 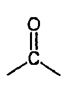 | 1 | 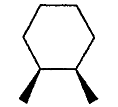 | 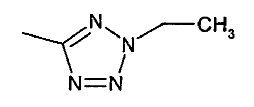 |
| 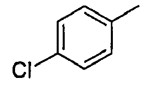 | 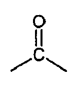 | 1 | 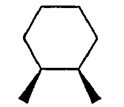 | 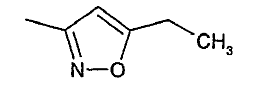 |
| 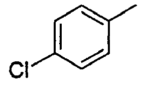 | 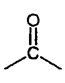 | 1 | 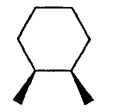 | 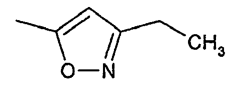 |
| 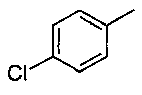 | 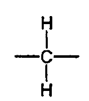 | 1 | 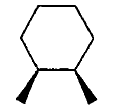 | 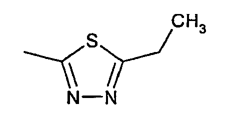 |
| 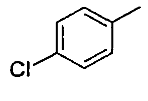 | 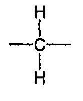 | 1 | 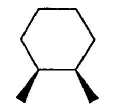 | 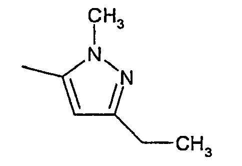 |
| 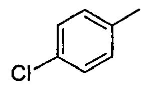 | 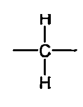 | 1 | 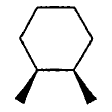 | 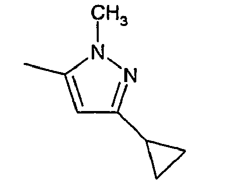 |
| 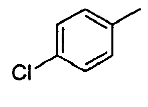 | 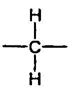 | 1 | 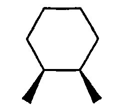 | 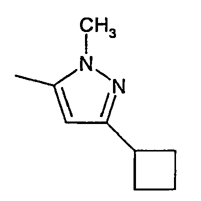 |
| 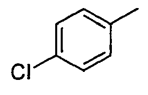 | 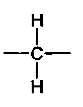 | 1 | 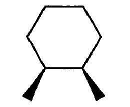 | 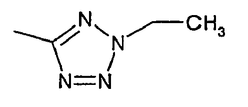 |
| 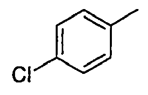 | 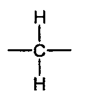 | 1 | 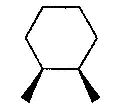 | 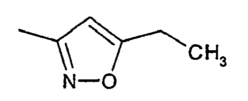 |
| 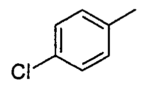 | 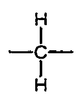 | 1 | 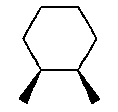 | 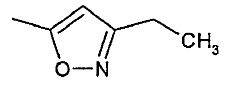 |
| 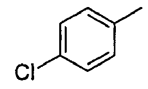 | -O- | 1 | 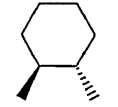 | 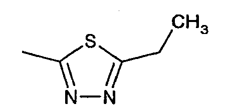 |
| 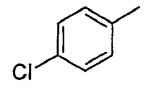 | -O- | 1 | 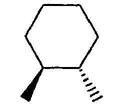 | 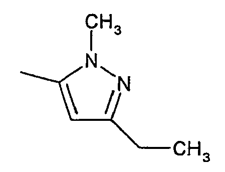 |
| 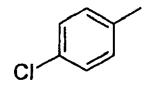 | -O- | 1 | 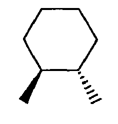 | 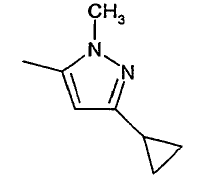 |
| 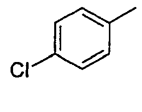 | -O- | 1 | 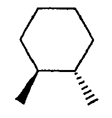 | 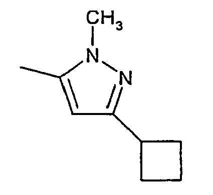 |
| 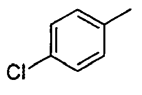 | -O- | 1 | 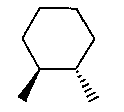 | 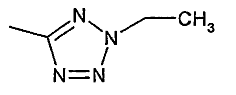 |
| 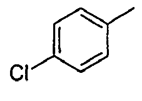 | -O- | 1 | 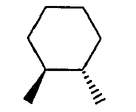 | 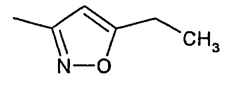 |
| 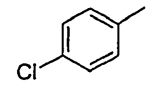 | -O- | 1 | 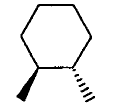 | 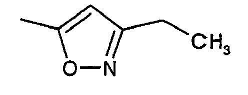 |
| 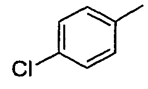 | 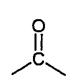 | 1 | 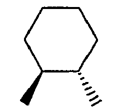 | 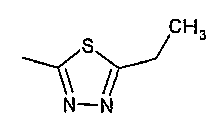 |
| 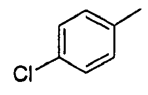 | 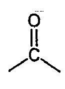 | 1 | 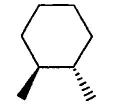 | 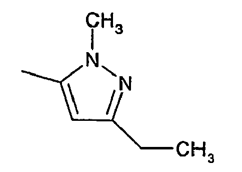 |
| 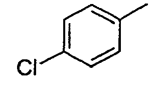 | 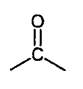 | 1 | 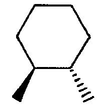 | 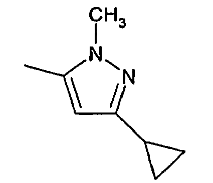 |
| 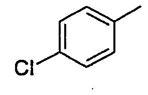 | 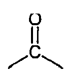 | 1 | 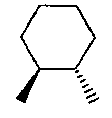 | 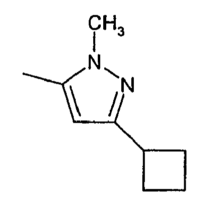 |
| 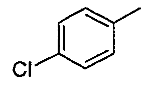 | 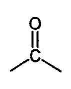 | 1 | 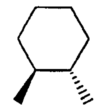 | 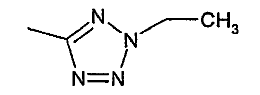 |
| 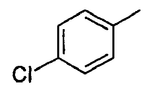 | 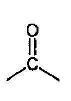 | 1 | 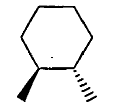 | 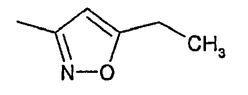 |
| 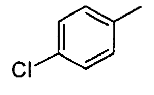 | 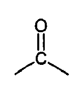 | 1 | 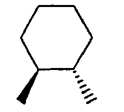 | 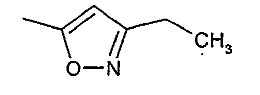 |
| 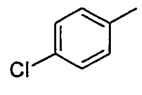 | 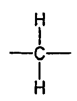 | 1 | 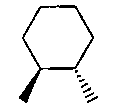 | 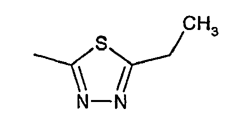 |
| 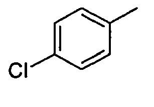 | 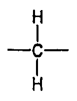 | 1 | 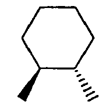 | 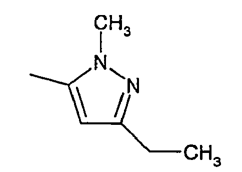 |
| 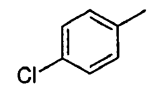 | 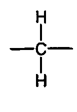 | 1 | 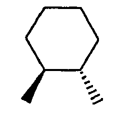 | 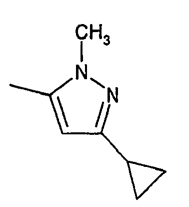 |
| 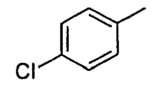 | 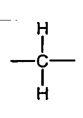 | 1 | 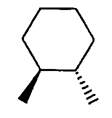 | 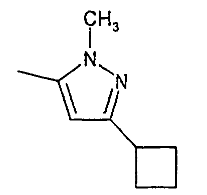 |
| 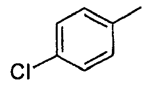 | 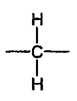 | 1 | 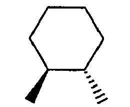 | 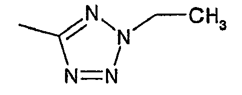 |
| 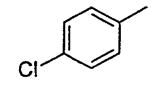 | 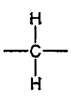 | 1 | 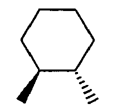 | 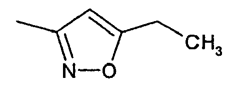 |
| 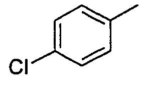 | 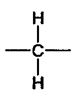 | 1 | 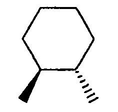 | 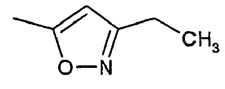 |
| 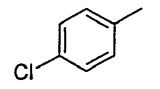 | -O- | 2 | 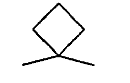 | 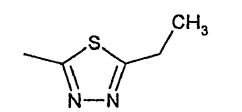 |
| 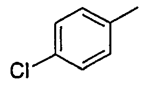 | -O- | 2 | 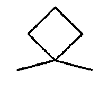 | 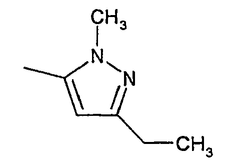 |
| 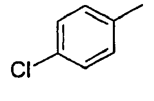 | -O- | 2 | 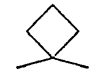 | 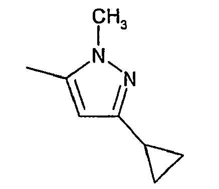 |
| 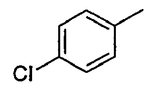 | -O- | 2 | 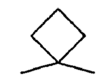 | 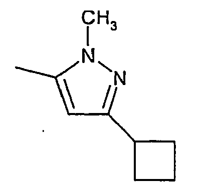 |
| 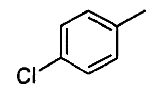 | -O- | 2 | 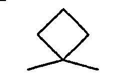 | 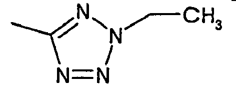 |
| 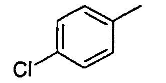 | -O- | 2 | 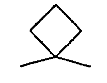 | 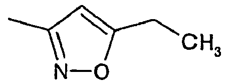 |
| 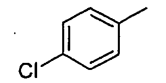 | -O- | 2 | 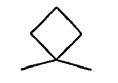 | 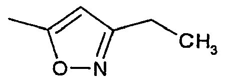 |
| 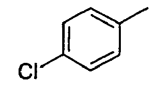 | 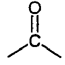 | 2 | 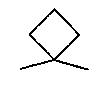 | 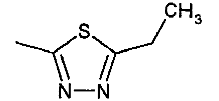 |
| 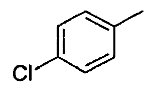 | 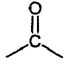 | 2 | 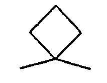 | 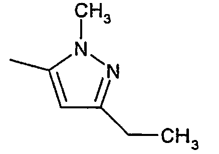 |
| 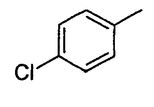 | 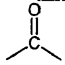 | 2 | 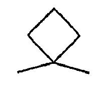 | 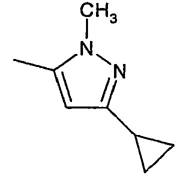 |
| 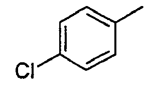 | 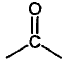 | 2 | 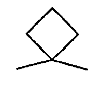 | 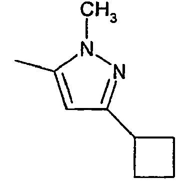 |
| 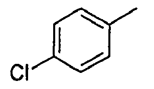 | 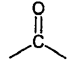 | 2 | 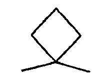 | 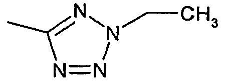 |
| 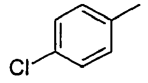 | 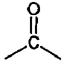 | 2 | 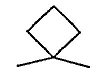 | 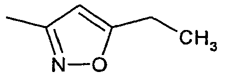 |
| 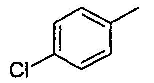 | 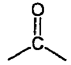 | 2 | 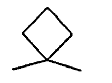 | 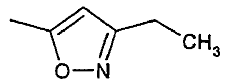 |
| 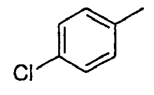 | 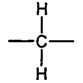 | 2 | 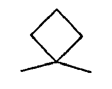 | 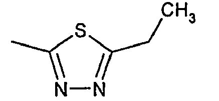 |
| 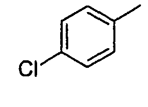 | 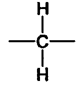 | 2 | 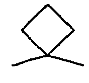 | 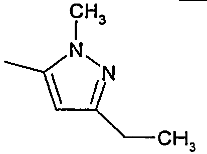 |
| 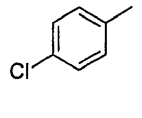 | 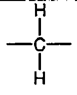 | 2 | 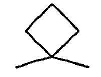 | 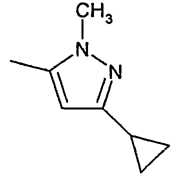 |
| 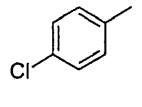 | 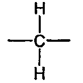 | 2 | 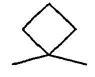 | 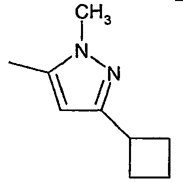 |
| 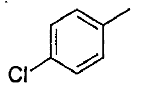 | 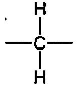 | 2 | 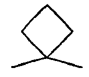 | 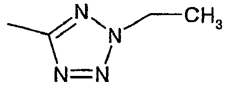 |
| 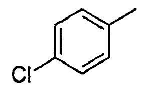 | 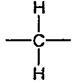 | 2 | 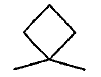 | 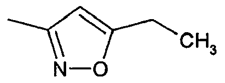 |
| 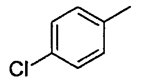 | 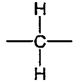 | 2 | 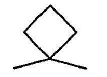 | 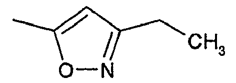 |
| 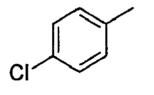 | -O- | 2 | 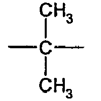 | 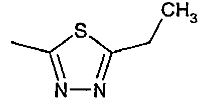 |
| 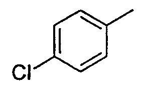 | -O- | 2 | 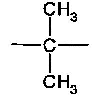 | 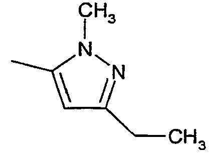 |
| 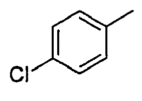 | -O- | 2 | 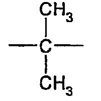 | 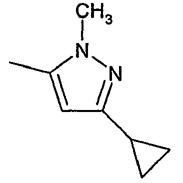 |
| 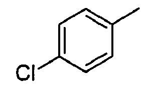 | -O- | 2 | 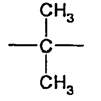 | 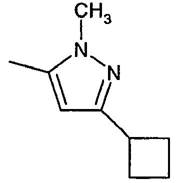 |
| 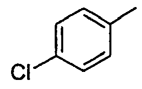 | -O- | 2 | 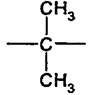 | 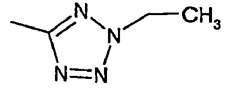 |
| 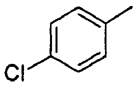 | -O- | 2 | 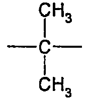 | 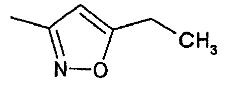 |
| 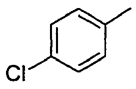 | -O- | 2 | 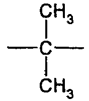 | 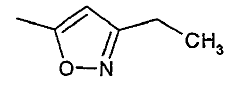 |
| 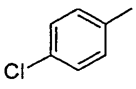 | 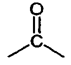 | 2 | 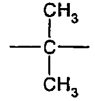 | 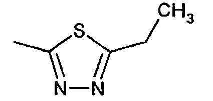 |
| 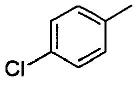 | 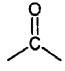 | 2 | 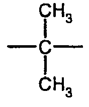 | 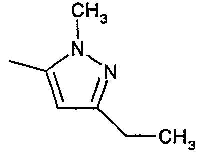 |
| 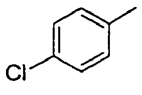 | 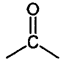 | 2 | 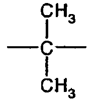 | 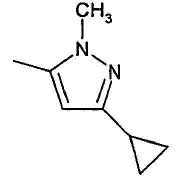 |
| 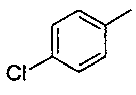 | 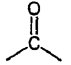 | 2 | 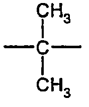 | 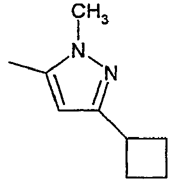 |
| 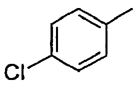 | 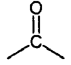 | 2 | 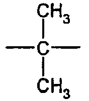 | 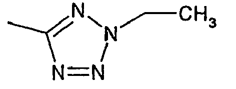 |
| 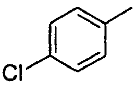 | 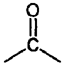 | 2 | 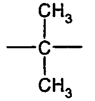 | 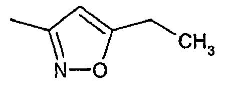 |
| 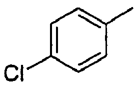 | 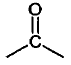 | 2 | 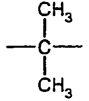 | 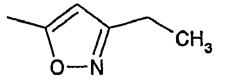 |
| 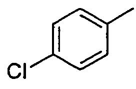 | 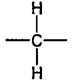 | 2 | 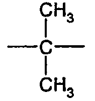 | 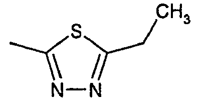 |
| 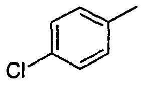 | 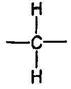 | 2 | 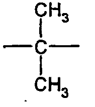 | 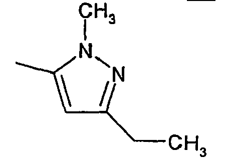 |
| 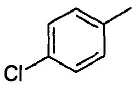 | 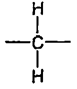 | 2 | 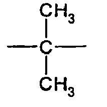 | 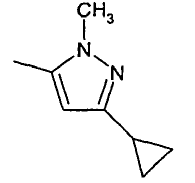 |
| 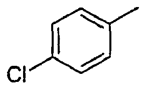 | 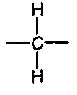 | 2 | 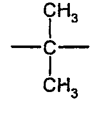 | 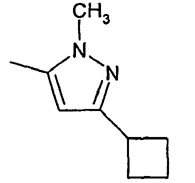 |
| 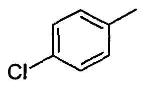 | 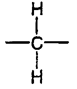 | 2 | 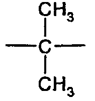 | 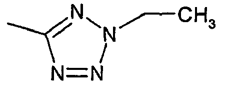 |
| 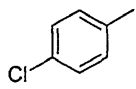 | 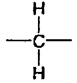 | 2 | 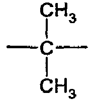 | 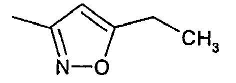 |
| 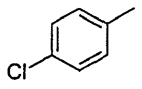 | 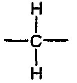 | 2 | 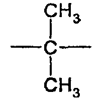 | 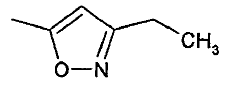 |
| 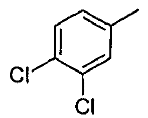 | -O- | 1 | 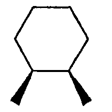 | 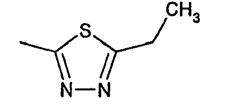 |
| 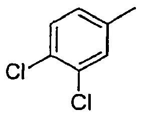 | -O- | 1 | 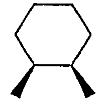 | 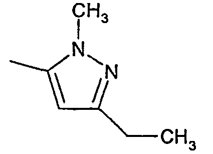 |
| 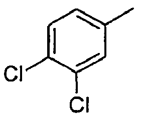 | -O- | 1 | 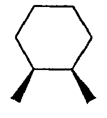 | 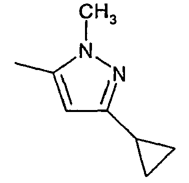 |
| 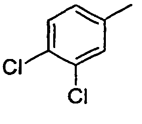 | -O- | 1 | 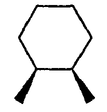 | 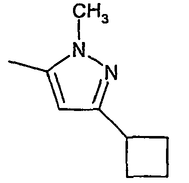 |
| 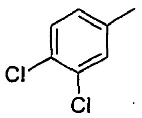 | -O- | 1 | 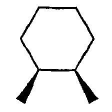 | 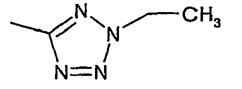 |
| 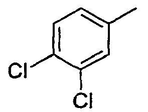 | -O- | 1 | 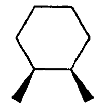 | 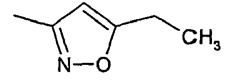 |
| 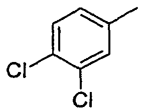 | -O- | 1 | 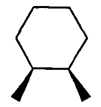 | 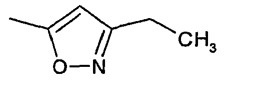 |
| 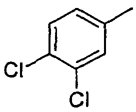 | -O- | 1 | 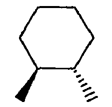 | 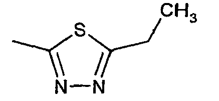 |
| 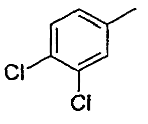 | -O- | 1 | 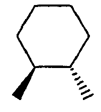 | 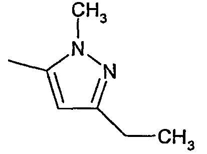 |
| 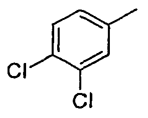 | -O- | 1 | 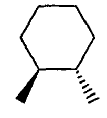 | 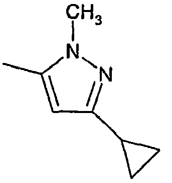 |
| 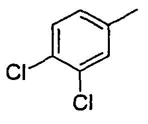 | -O- | 1 | 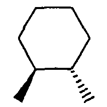 | 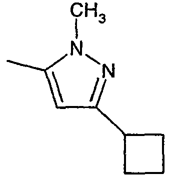 |
| 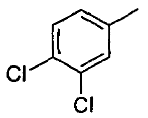 | -O- | 1 | 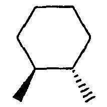 | 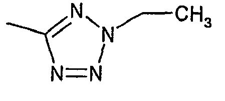 |
| 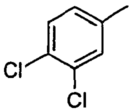 | -O- | 1 | 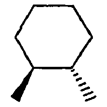 | 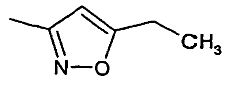 |
| 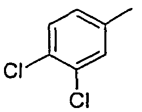 | -O- | 1 | 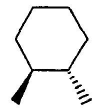 | 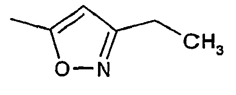 |
| 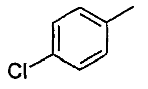 | -O- | 1 | 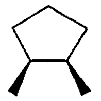 | 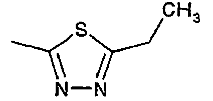 |
| 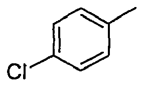 | -O- | 1 | 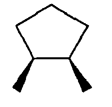 | 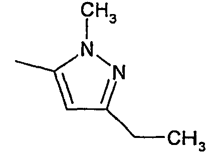 |
| 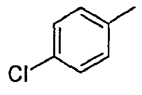 | -O- | 1 | 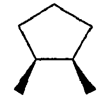 | 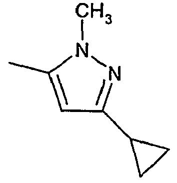 |
| 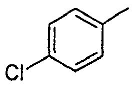 | -O- | 1 | 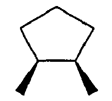 | 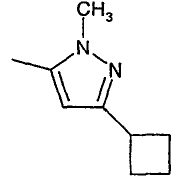 |
| 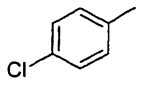 | -O- | 1 | 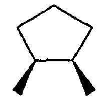 | 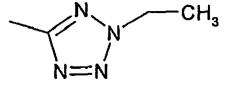 |
| 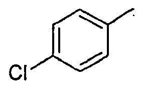 | -O- | 1 | 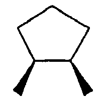 | 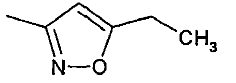 |
| 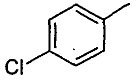 | -O- | 1 | 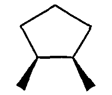 | 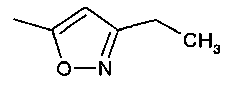 |
| 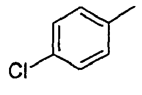 | -O- | 1 | 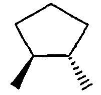 | 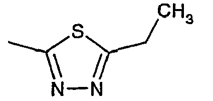 |
| 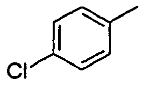 | -O- | 1 | 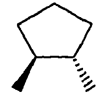 | 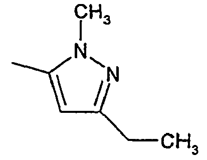 |
| 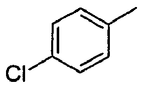 | -O- | 1 | 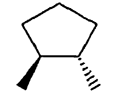 | 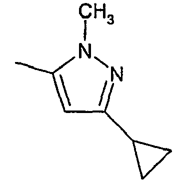 |
| 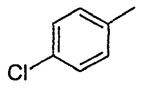 | -O- | 1 | 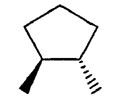 | 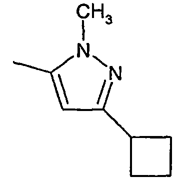 |
| 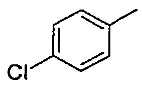 | -O- | 1 | 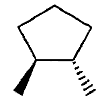 | 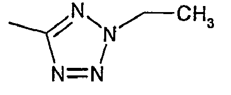 |
| 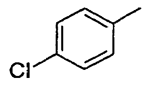 | -O- | 1 | 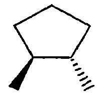 | 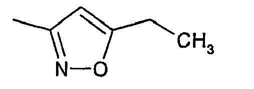 |
| 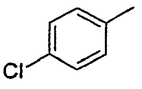 | -O- | 1 | 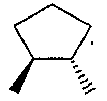 | 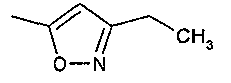 |

5. A compound according to any one of the preceding claims in combination with another drug substance which is an anti-inflammatory, a bronchodilator, an antihistamine or an antitussive substance.

6. A compound according to any one of the preceding claims for use as a pharmaceutical.

7. A pharmaceutical composition comprising as active ingredient a compound according to any one of claims 1 to 4.

8. The use of a compound according to any one of claims 1 to 4 for the manufacture of a medicament for the treatment of a condition mediated by CCR-3.

9. The use of a compound according to any one of claims 1 to 4 for the manufacture of a medicament for the treatment of an inflammatory or allergic condition, particularly an inflammatory or obstructive airways disease.

10. A process for the preparation of a compound of formula Ia or Ib as claimed in claim 1 which comprises
(i) (A) for the preparation of compounds of formula Ia where R² is hydrogen, reacting a compound of formula IIa 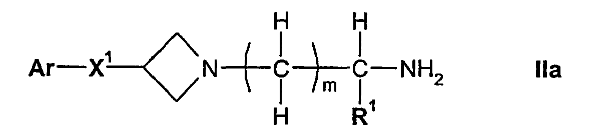 or a protected form thereof, where Ar, X¹, m and R¹ are as defined in claim 1, with a compound of formula III
Y=C=N-R³ **III**
where Y and R³ are as defined in claim 1; or
(B) for the preparation of compounds of formula Ia where Y is oxygen, reacting a compound of formula IIa where Ar, X¹, m and R¹ are as defined in claim 1, with a compound of formula IV 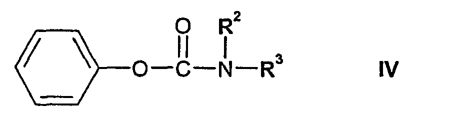 where R² and R³ are as defined in claim 1; or
(C) for the preparation of compounds of formula Ia where X¹ is -S(=O)₂-, oxidising a compound of formula Ia in protected form where X¹ is -S- and Ar, m, R¹, Y, R² and R³ are as defined in claim 1;
(D) for the preparation of compounds of formula Ib, reacting a compound of formula IIb 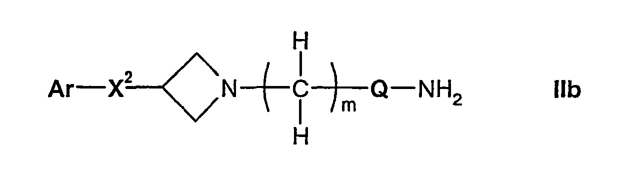 where Ar, X², m and Q are as defined in claim 1, with a compound of formula IV where R² and R³ are as defined in claim 1;
(E) for the preparation of compounds of formula Ib where R² is hydrogen, reacting a compound of formula IIb where Ar, X², m and Q are as defined in claim 1, with a compound of formula V
O=C=N-**R³** **V**
where R³ is as defined in claim 1; or
(F) for the preparation of compounds of formula Ib where X is -S(=O)₂-, oxidising a compound of formula Ib in protected form where X² is -S- and Ar, m, Q, R² and R³ are as defined in claim 1; and
(ii) recovering the product in free or salt form.

## Patentansprüche

1. Verbindungen der Formel Ia oder Ib 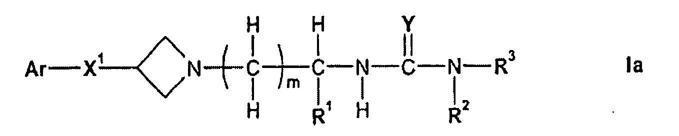 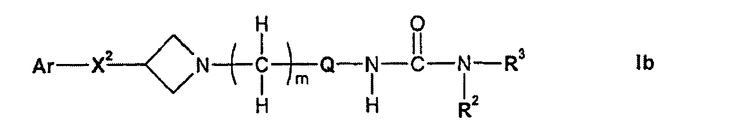 in freier Form oder in Form eines Salzes, worin
Ar für Phenyl steht, das optional substituiert ist durch einen oder mehrere Substituenten, die ausgewählt sind aus Halogen, C₁-C₈-Alkyl, Cyano oder Nitro,
X¹ für -S-, -S(=O)- oder -S(=O)₂- steht,
X² für -C(=O)-, -O-, -CH₂-, -S-, -S(=O)- oder -S(=O)₂- steht,
m für 1, 2, 3 oder 4 steht,
R¹ für Wasserstoff oder C₁-C₈-Alkyl steht, das optional substituiert ist durch Hydroxy, C₁-C₈-Alkoxy, Acyloxy, Halogen, Carboxy, C₁-C₈-Alkoxycarbonyl, -N(R⁴)R⁵, -CON(R⁶)R⁷ oder durch eine monovalente cyclische organische Gruppe, die 3 bis 15 Atome im Ringsystem enthält,
Q die folgende Formel hat 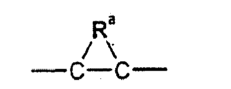
worin R^{a} für C₁-C₈-Alkylen steht, oder
Q für -C(R^{b})(R^{c})- steht, worin R^{b} und R^{c} unabhängig für C₁-C₈-Alkyl stehen oder R^{b} und R^{c} zusammen ein C₃-C₁₀-Cycloalkyl bilden,
Y für Sauerstoff oder Schwefel steht,
R² für Wasserstoff, C₁-C₈-Alkyl oder C₃-C₁₀-Cycloalkyl steht und
R³ für C₁-C₈-Alkyl steht, das durch Phenyl, Phenoxy, Acyloxy oder Naphthyl substituiert ist, oder
R³ steht für C₃-C₁₀-Cycloalkyl, an das optional eine Benzogruppe fusioniert ist, für eine Heterocyclylgruppe mit 5 bis 11 Ringatomen, wovon 1 bis 4 Atome Heteroatome sind, für Phenyl oder Naphthyl, wobei die Gruppen Phenyl, Phenoxy oder Naphthyl optional substituiert sind durch einen oder mehrere Substituenten, die ausgewählt sind aus Halogen, Cyano, Hydroxy, Acyl, Nitro, -SO₂NH₂, C₁-C₈-Alkyl, das optional substituiert ist durch C₁-C₈-Alkoxy, aus C₁-C₈-Halogenalkyl, C₁-C₈-Alkoxy, C₁-C₈-Halogenalkoxy, C₁-C₈-Alkylthio, -SO₂-C₁-C₈-Alkyl, C₁-C₈-Alkoxycarbonyl, C₁-C₈-Acylamino, das optional am Stickstoffatom substituiert ist durch C₁-C₈-Alkyl, aus C₁-C₈-Alkylamino, Aminocarbonyl, C₁-C₈-Alkylaminocarbonyl, Di(C₁-C₈-alkyl)amino, Di(C₁-C₈-alkyl)aminocarbonyl, Di(C₁-C₈-alkyl)aminocarbonylmethoxy, oder
R² und R³ zusammen mit dem Stickstoffatom, an das sie gebunden sind, für eine Heterocyclylgruppe stehen, die 5 bis 10 Ringatome hat, wovon 1, 2 oder 3 dieser Atome Heteroatome sind,
R⁴ und R⁵ jeweils unabhängig für Wasserstoff oder C₁-C₈-Alkyl stehen, oder
R⁴ für Wasserstoff steht und
R⁵ für Hydroxy-C₁-C₈-alkyl, Acyl, -SO₂R⁸ oder-CON(R⁶)R⁷ steht, oder
R⁴ und R⁵ zusammen mit dem Stickstoffatom, an das sie gebunden sind, für eine 5- oder 6-gliedrige Heterocyclylgruppe stehen,
R⁶ und R⁷ jeweils unabhängig für Wasserstoff oder C₁-C₈-Alkyl stehen, oder
R⁶ und R⁷ zusammen mit dem Stickstoffatom, an das sie gebunden sind, für eine 5- oder 6-gliedrige Heterocyclylgruppe stehen, und
R⁸ für C₁-C₈-Alkyl, C₁-C₈-Halogenalkyl oder Phenyl steht, das optional substituiert ist durch C₁-C₈-Alkyl.

2. Verbindung nach Anspruch 1, die Folgendes ist
(i) eine Verbindung der Formel Ia in freier Form oder in Form eines Salzes, worin
Ar für Phenyl steht, das substituiert ist durch Halogen,
X¹ für -S-, -S(=O)- oder -S(=O)₂- steht,
m für 2 steht,
R¹ für C₁-C₈-Alkyl steht, das optional substituiert ist durch Hydroxy oder C₁-C₈-Alkoxy,
Y für Sauerstoff steht,
R² für Wasserstoff steht, und
R³ für eine Heterocyclylgruppe mit 5 bis 11 Ringatomen steht, wovon 1 bis 4 dieser Ringatome für Heteroatome stehen,
oder
(ii) eine Verbindung der Formel Ib in freier Form oder in Form eines Salzes, worin
Ar für Phenyl steht, das substituiert ist durch Halogen,
X² für -O-, -C(=O)- oder -CH₂- steht,
m für 1 oder 2 steht,
Q die folgende Formel hat 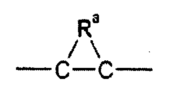 worin R^{a} für C₁-C₈-Alkylen steht, oder
Q für -C(R^{b})(R^{c})- steht, worin R^{b} und R^{c} unabhängig für C₁-C₈-Alkyl stehen oder
R^{b} und R^{c} zusammen ein C₃-C₁₀-Cycloalkyl bilden,
R² für Wasserstoff steht, und
R³ für eine Heterocyclylgruppe mit 5 bis 11 Ringatomen steht, worin 1 bis 4 dieser Atome für Heteroatome stehen.

3. Verbindung nach Anspruch 1, die Folgendes ist
(i) eine Verbindung der Formel Ia in freier Form oder in Form eines Salzes, worin
Ar für Phenyl steht, das substituiert ist durch Halogen, vorzugsweise durch Chlor,
X¹ für -S-, -S(=O)- oder -S(=O)₂- steht,
m für 2 steht,
R¹ für C₁-C₄-Alkyl steht, das optional substituiert ist durch Hydroxy oder C₁-C₄-Alkoxy,
Y für Sauerstoff steht,
R² für Wasserstoff steht, und
R³ für eine Heterocyclylgruppe mit 5, 6 oder 7 Ringatomen steht, wovon ein, zwei, drei oder vier Heteroatome sind, die ausgewählt sind aus Stickstoff, Sauerstoff und Schwefel, wobei diese Heterocyclylgruppe optional substituiert ist durch C₁-C₄-Alkyl, C₁-C₄-Alkoxy oder C₃-C₆-Cycloalkyl, oder
(ii) eine Verbindung der Formel Ib in freier Form oder in Form eines Salzes, worin
Ar für Phenyl steht, das substituiert ist durch Halogen, vorzugsweise durch Chlor,
X² für-O-, -C(=O)- oder -CH₂- steht,
m für 1 oder 2 steht,
Q die folgende Formel hat 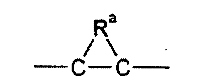
worin R^{a} für C₁-C₈-Alkylen steht, oder
Q für -C(R^{b})(R^{c})- steht, worin R^{b} und R^{c} unabhängig für C₁-C₄-Alkyl stehen oder
R^{b} und R^{c} zusammen ein C₃-C₆-Cycloalkyl bilden,
R² für Wasserstoff steht, und
R³ für eine Heterocyclylgruppe mit 5, 6 oder 7 Ringatomen steht, wovon ein, zwei, drei oder vier Heteroatome sind, die ausgewählt sind aus Stickstoff, Sauerstoff und Schwefel, wobei diese Heterocyclylgruppe optional substituiert ist durch C₁-C₄-Alkyl oder C₃-C₆-Cycloalkyl.

4. Verbindung nach Anspruch 1, die entweder eine Verbindung der folgenden Formel XX ist 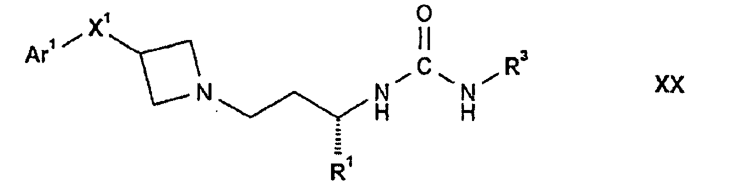 worin Ar, X¹ und R³ die in der folgenden Tabelle angegebenen Bedeutungen haben:
| Ar | X¹ | R¹ | R³ |
|---|---|---|---|
| 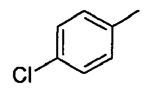 | 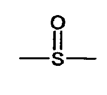 | 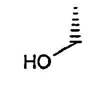 | 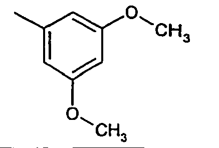 |
| 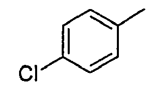 | 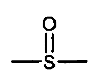 | 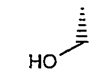 | 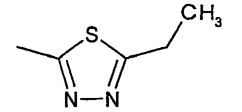 |
| 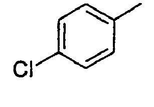 | 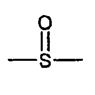 | 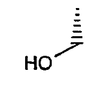 | 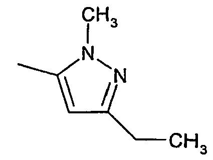 |
| 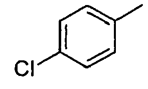 | 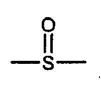 | 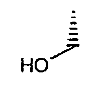 | 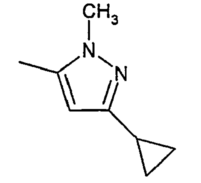 |
| 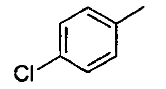 | 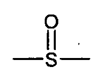 | 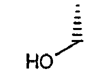 | 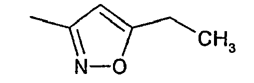 |
| 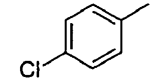 | 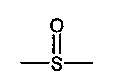 | 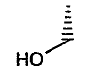 | 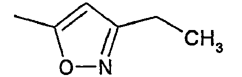 |
| 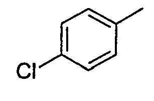 | 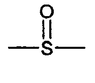 | 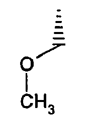 | 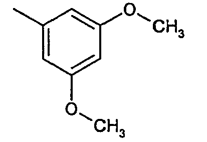 |
| 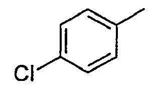 | 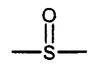 | 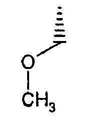 | 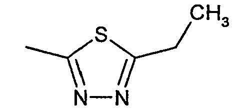 |
| 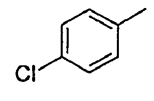 | 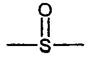 | 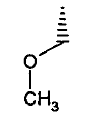 | 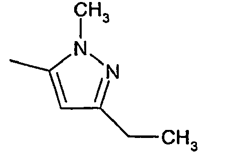 |
| 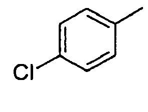 | 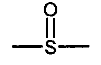 | 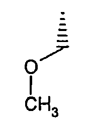 | 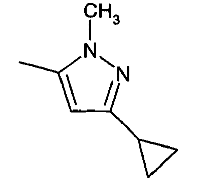 |
| 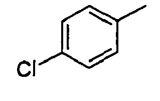 | 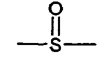 | 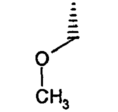 | 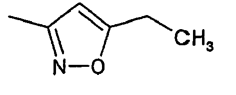 |
| 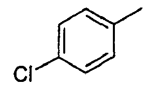 | 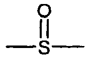 | 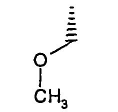 | 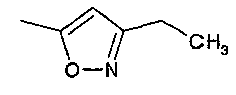 |
| 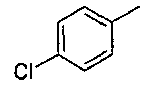 | 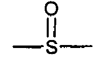 | 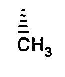 | 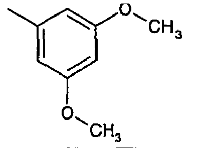 |
| 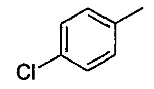 | 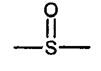 | 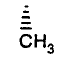 | 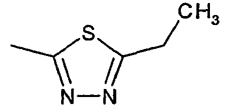 |
| 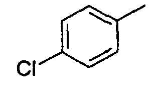 | 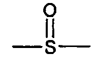 | 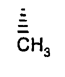 | 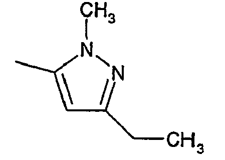 |
| 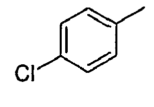 | 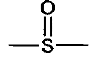 | 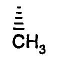 | 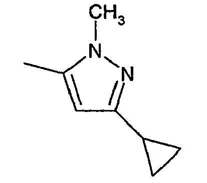 |
| 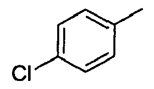 | 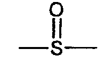 | 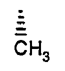 | 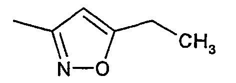 |
| 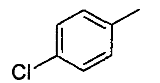 | 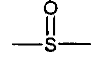 | 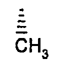 | 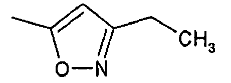 |
| 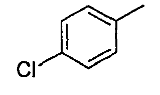 | -s- | 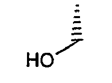 | 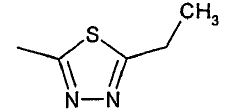 |
| 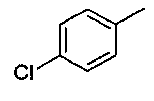 | -s- | 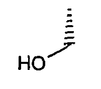 | 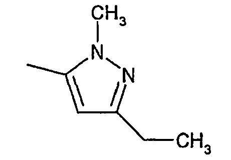 |
| 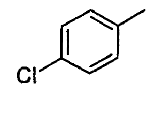 | -s- | 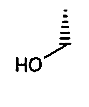 | 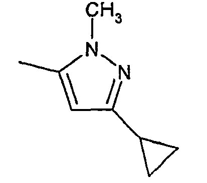 |
| 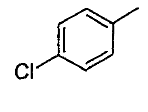 | -s- | 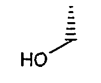 | 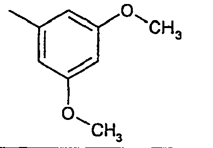 |
| 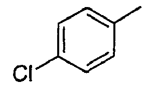 | -s- | 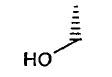 | 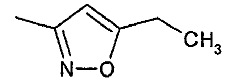 |
| 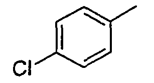 | -s- | 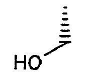 | 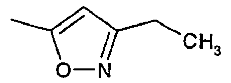 |
| 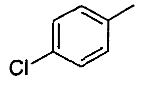 | 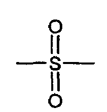 | 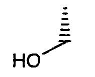 | 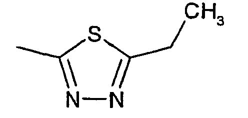 |
| 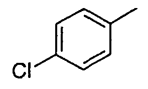 | 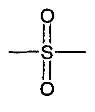 | 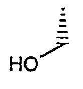 | 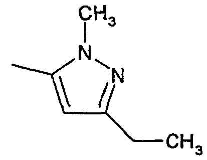 |
| 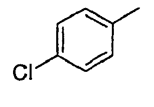 | 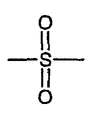 | 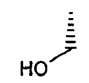 | 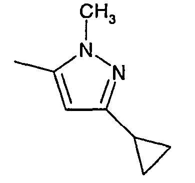 |
| 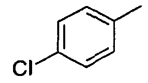 | 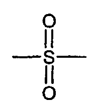 | 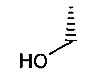 | 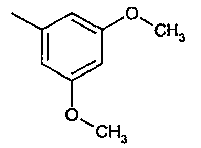 |
| 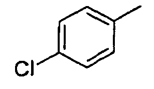 | 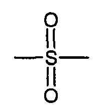 | 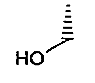 | 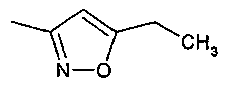 |
| 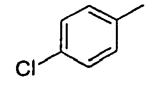 | 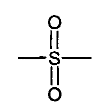 | 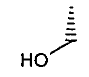 | 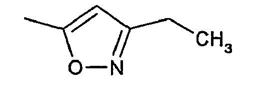 |
oder die eine Verbindung der folgenden Formel XXI ist 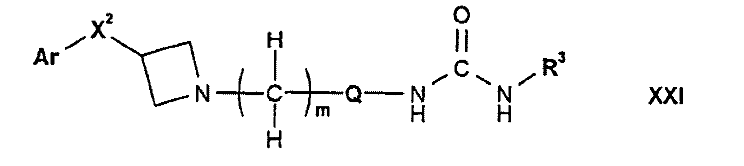 worin Ar, X², m, Q und R³ die in der folgenden Tabelle angegebenen Bedeutungen haben:
| Ar | X² | m | -Q- | R³ |
|---|---|---|---|---|
| 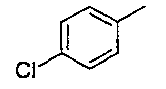 | -O- | 1 | 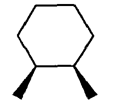 | 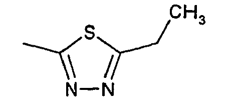 |
| 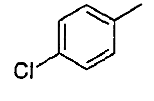 | -O- | 1 | 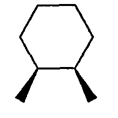 | 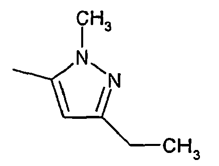 |
| 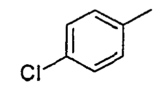 | -O- | 1 | 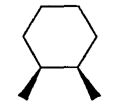 | 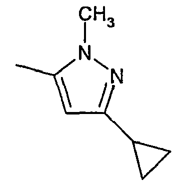 |
| 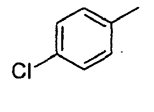 | -O- | 1 | 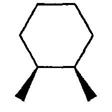 | 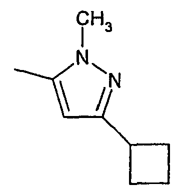 |
| 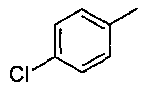 | -O- | 1 | 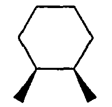 | 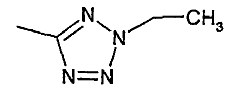 |
| 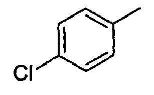 | -O- | 1 | 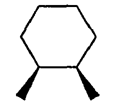 | 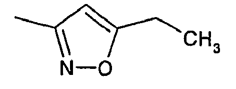 |
| 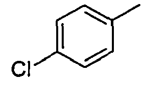 | -O- | 1 | 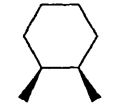 | 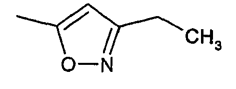 |
| 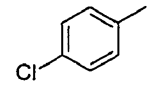 | 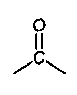 | 1 | 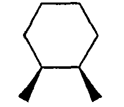 | 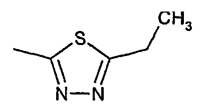 |
| 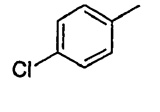 | 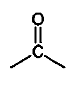 | 1 | 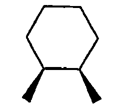 | 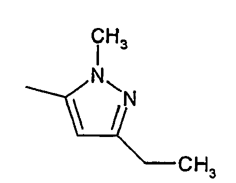 |
| 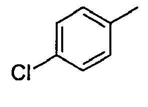 | 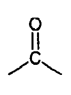 | 1 | 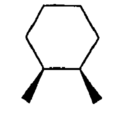 | 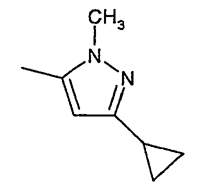 |
| 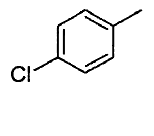 | 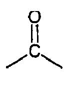 | 1 | 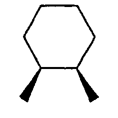 | 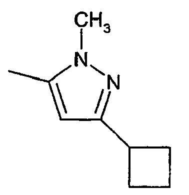 |
| 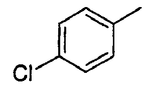 | 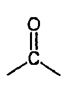 | 1 | 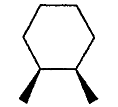 | 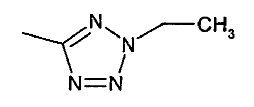 |
| 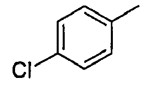 | 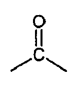 | 1 | 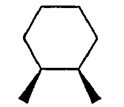 | 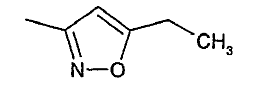 |
| 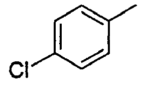 | 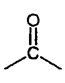 | 1 | 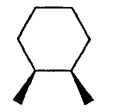 | 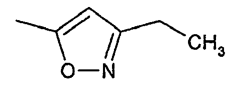 |
| 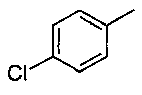 | 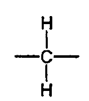 | 1 | 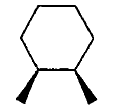 | 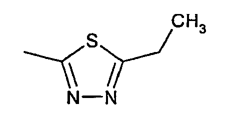 |
| 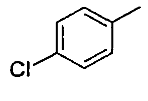 | 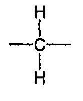 | 1 | 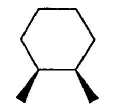 | 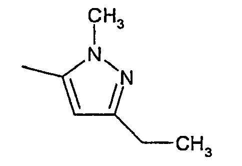 |
| 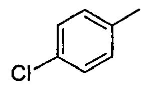 | 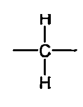 | 1 | 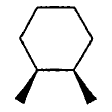 | 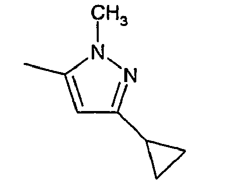 |
| 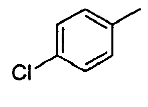 | 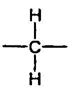 | 1 | 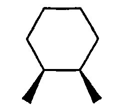 | 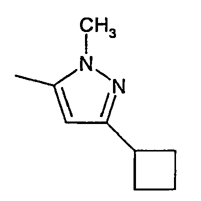 |
| 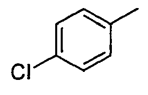 | 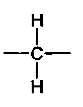 | 1 | 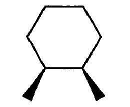 | 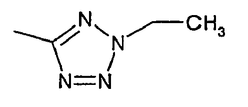 |
| 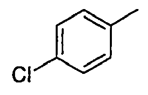 | 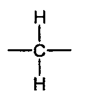 | 1 | 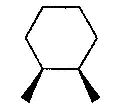 | 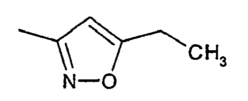 |
| 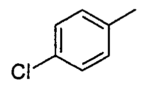 | 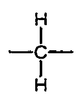 | 1 | 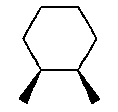 | 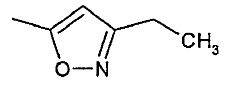 |
| 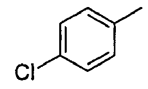 | -O- | 1 | 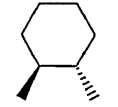 | 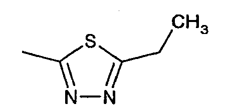 |
| 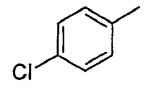 | -O- | 1 | 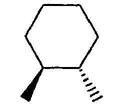 | 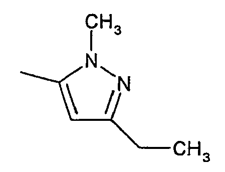 |
| 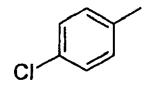 | -O- | 1 | 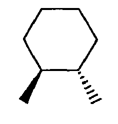 | 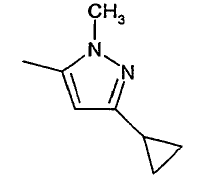 |
| 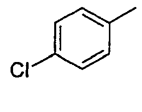 | -O- | 1 | 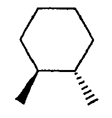 | 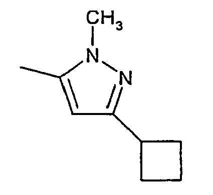 |
| 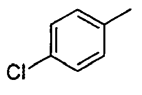 | -O- | 1 | 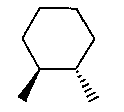 | 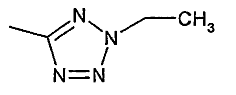 |
| 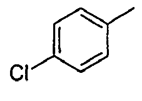 | -O- | 1 | 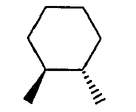 | 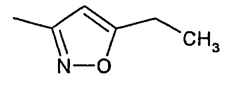 |
| 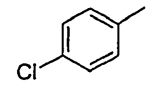 | -O- | 1 | 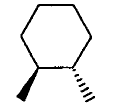 | 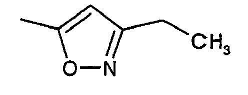 |
| 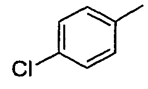 | 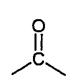 | 1 | 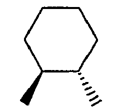 | 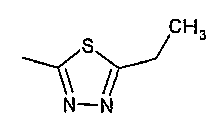 |
| 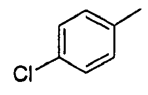 | 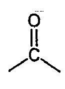 | 1 | 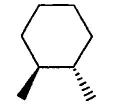 | 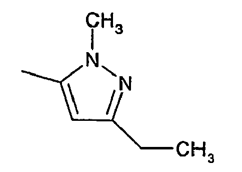 |
| 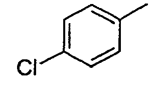 | 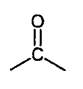 | 1 | 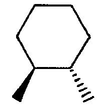 | 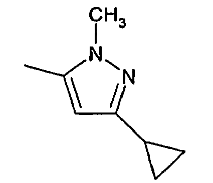 |
| 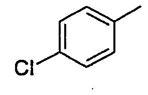 | 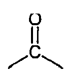 | 1 | 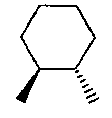 | 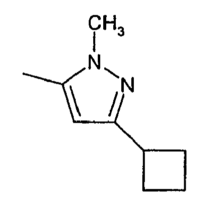 |
| 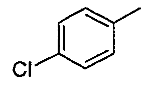 | 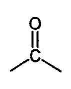 | 1 | 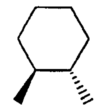 | 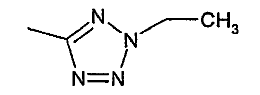 |
| 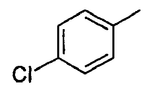 | 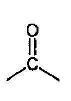 | 1 | 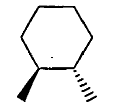 | 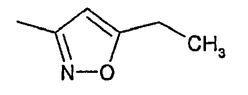 |
| 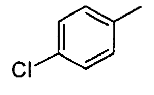 | 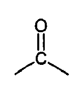 | 1 | 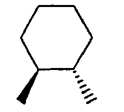 | 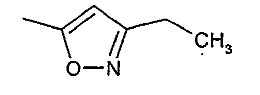 |
| 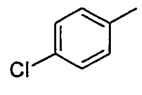 | 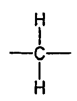 | 1 | 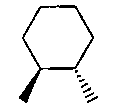 | 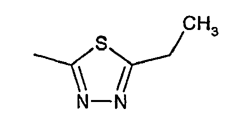 |
| 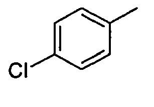 | 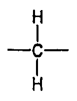 | 1 | 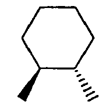 | 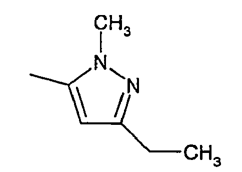 |
| 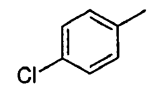 | 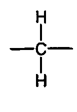 | 1 | 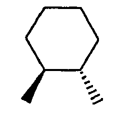 | 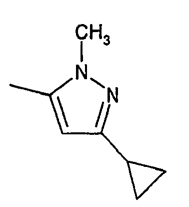 |
| 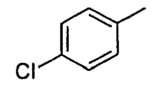 | 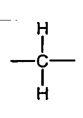 | 1 | 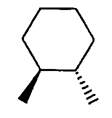 | 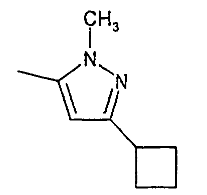 |
| 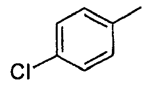 | 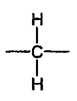 | 1 | 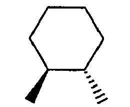 | 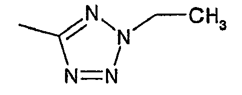 |
| 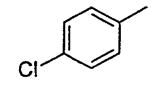 | 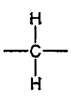 | 1 | 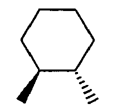 | 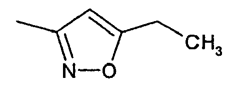 |
| 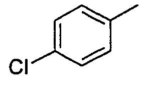 | 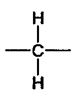 | 1 | 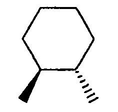 | 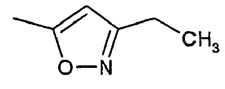 |
| 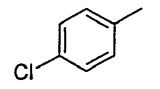 | -O- | 2 | 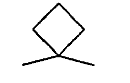 | 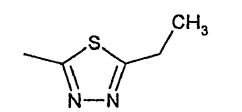 |
| 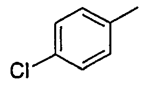 | -O- | 2 | 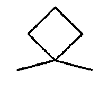 | 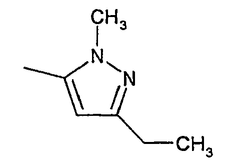 |
| 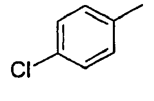 | -O- | 2 | 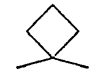 | 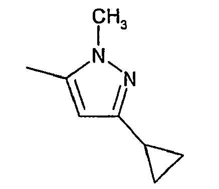 |
| 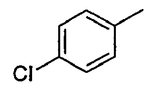 | -O- | 2 | 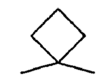 | 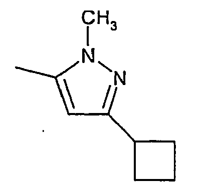 |
| 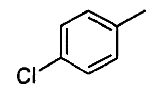 | -O- | 2 | 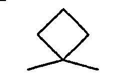 | 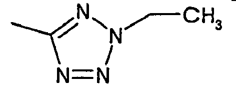 |
| 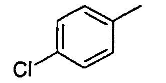 | -O- | 2 | 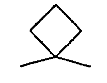 | 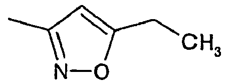 |
| 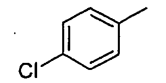 | -O- | 2 | 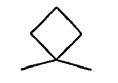 | 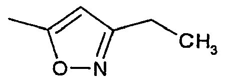 |
| 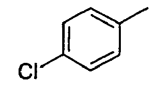 | 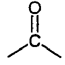 | 2 | 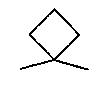 | 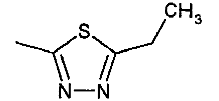 |
| 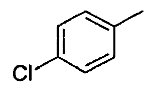 | 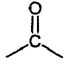 | 2 | 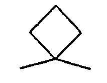 | 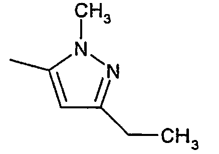 |
| 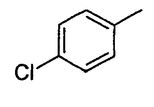 | 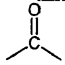 | 2 | 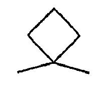 | 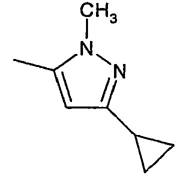 |
| 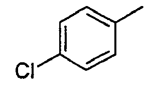 | 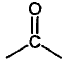 | 2 | 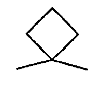 | 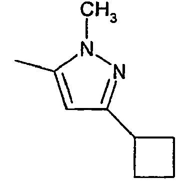 |
| 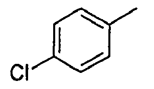 | 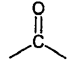 | 2 | 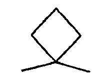 | 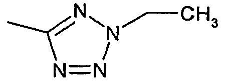 |
| 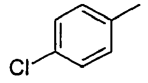 | 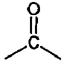 | 2 | 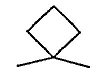 | 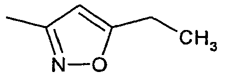 |
| 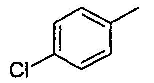 | 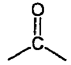 | 2 | 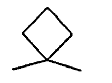 | 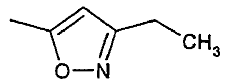 |
| 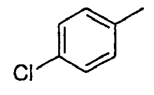 | 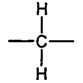 | 2 | 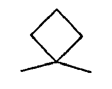 | 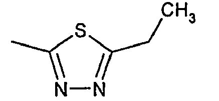 |
| 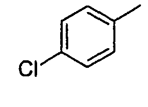 | 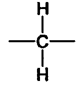 | 2 | 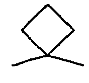 | 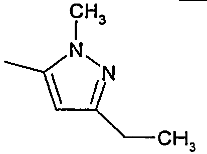 |
| 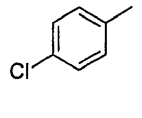 | 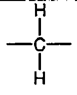 | 2 | 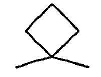 | 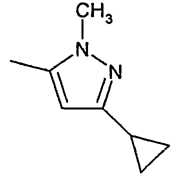 |
| 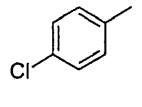 | 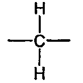 | 2 | 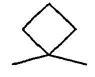 | 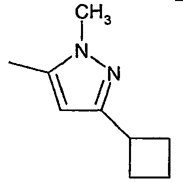 |
| 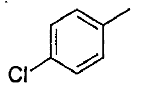 | 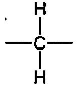 | 2 | 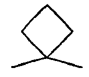 | 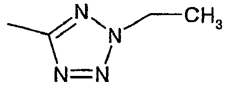 |
| 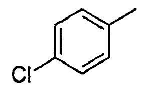 | 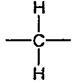 | 2 | 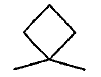 | 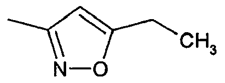 |
| 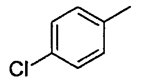 | 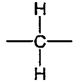 | 2 | 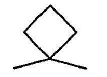 | 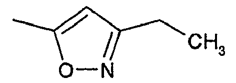 |
| 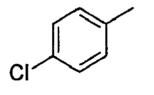 | -O- | 2 | 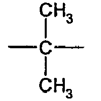 | 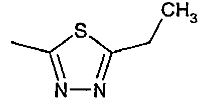 |
| 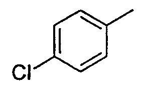 | -O- | 2 | 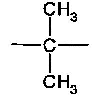 | 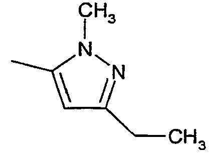 |
| 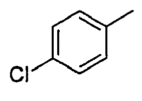 | -O- | 2 | 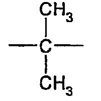 | 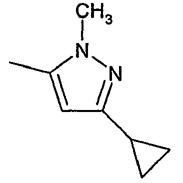 |
| 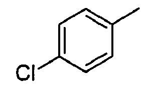 | -O- | 2 | 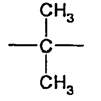 | 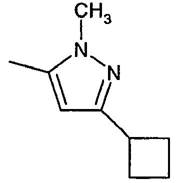 |
| 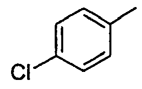 | -O- | 2 | 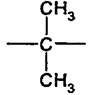 | 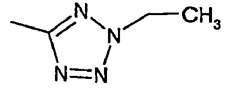 |
| 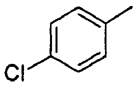 | -O- | 2 | 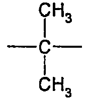 | 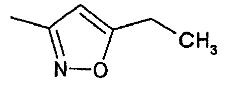 |
| 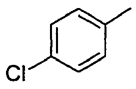 | -O- | 2 | 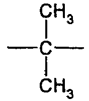 | 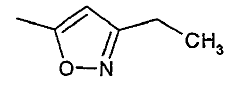 |
| 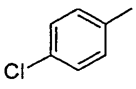 | 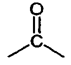 | 2 | 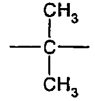 | 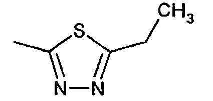 |
| 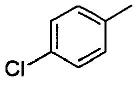 | 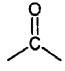 | 2 | 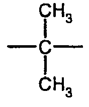 | 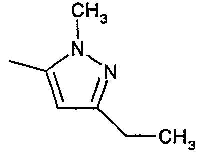 |
| 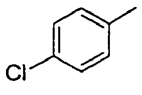 | 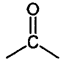 | 2 | 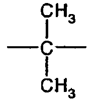 | 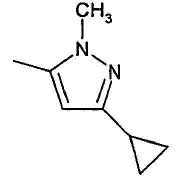 |
| 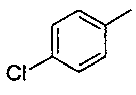 | 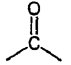 | 2 | 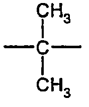 | 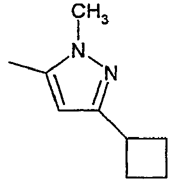 |
| 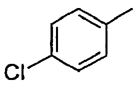 | 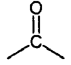 | 2 | 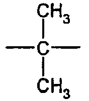 | 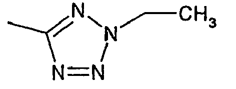 |
| 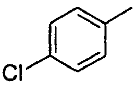 | 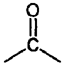 | 2 | 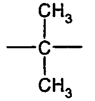 | 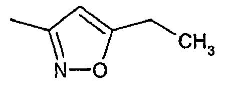 |
| 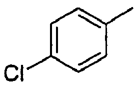 | 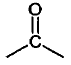 | 2 | 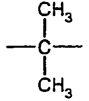 | 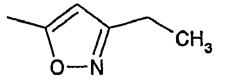 |
| 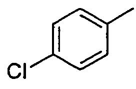 | 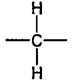 | 2 | 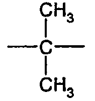 | 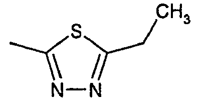 |
| 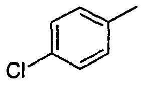 | 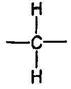 | 2 | 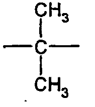 | 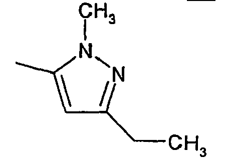 |
| 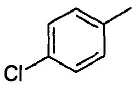 | 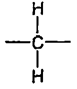 | 2 | 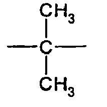 | 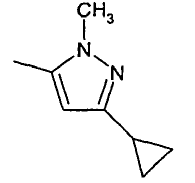 |
| 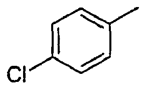 | 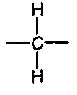 | 2 | 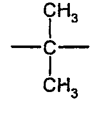 | 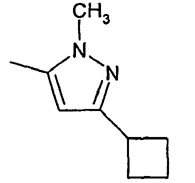 |
| 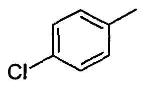 | 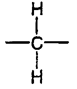 | 2 | 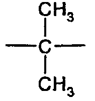 | 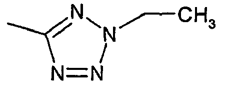 |
| 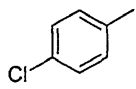 | 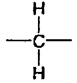 | 2 | 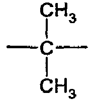 | 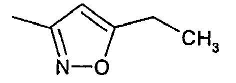 |
| 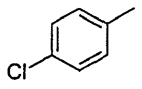 | 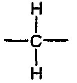 | 2 | 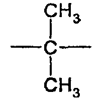 | 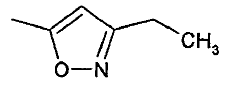 |
| 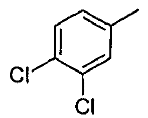 | -O- | 1 | 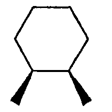 | 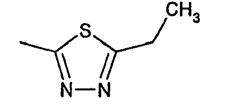 |
| 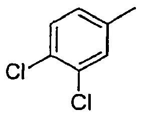 | -O- | 1 | 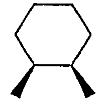 | 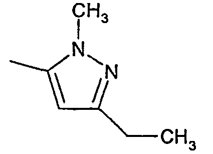 |
| 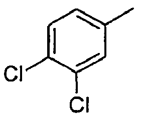 | -O- | 1 | 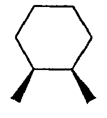 | 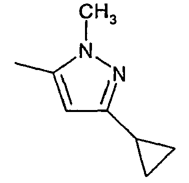 |
| 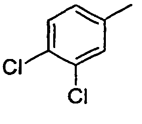 | -O- | 1 | 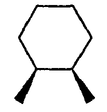 | 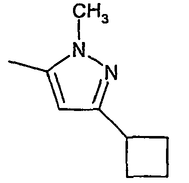 |
| 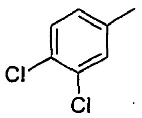 | -O- | 1 | 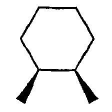 | 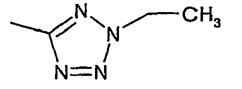 |
| 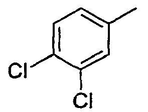 | -O- | 1 | 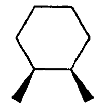 | 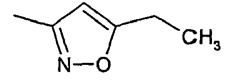 |
| 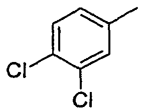 | -O- | 1 | 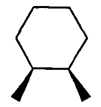 | 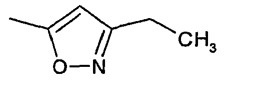 |
| 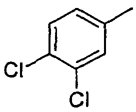 | -O- | 1 | 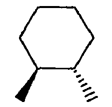 | 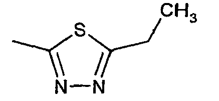 |
| 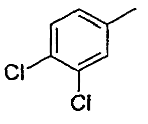 | -O- | 1 | 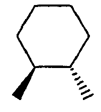 | 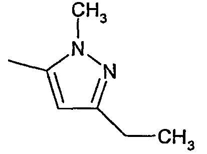 |
| 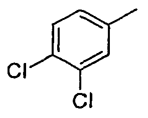 | -O- | 1 | 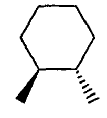 | 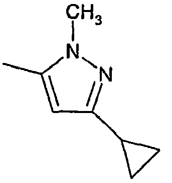 |
| 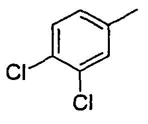 | -O- | 1 | 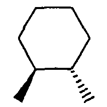 | 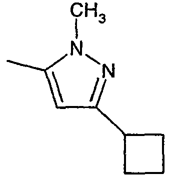 |
| 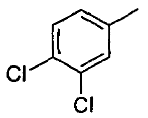 | -O- | 1 | 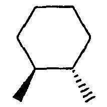 | 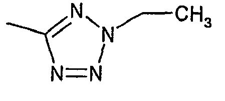 |
| 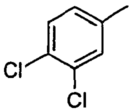 | -O- | 1 | 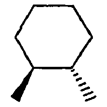 | 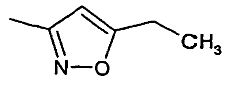 |
| 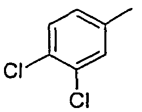 | -O- | 1 | 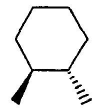 | 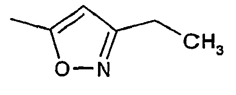 |
| 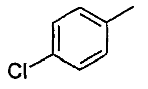 | -O- | 1 | 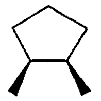 | 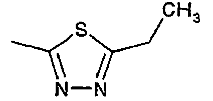 |
| 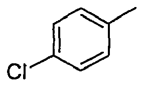 | -O- | 1 | 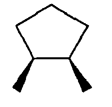 | 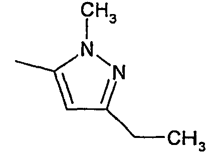 |
| 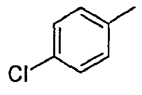 | -O- | 1 | 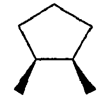 | 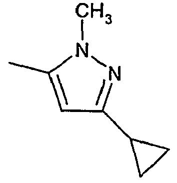 |
| 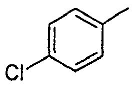 | -O- | 1 | 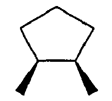 | 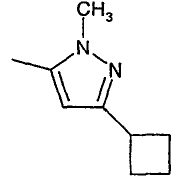 |
| 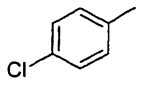 | -O- | 1 | 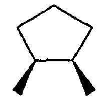 | 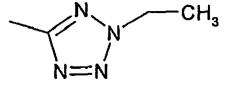 |
| 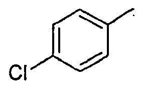 | -O- | 1 | 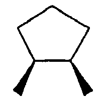 | 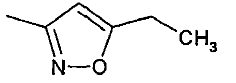 |
| 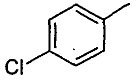 | -O- | 1 | 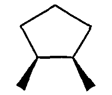 | 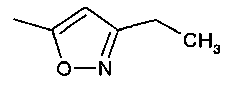 |
| 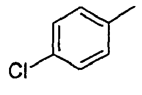 | -O- | 1 | 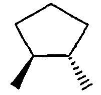 | 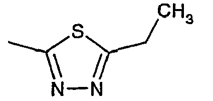 |
| 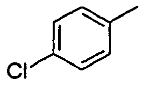 | -O- | 1 | 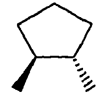 | 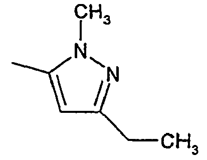 |
| 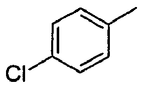 | -O- | 1 | 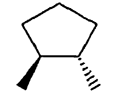 | 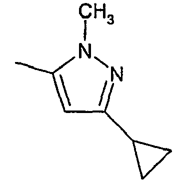 |
| 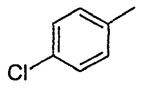 | -O- | 1 | 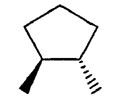 | 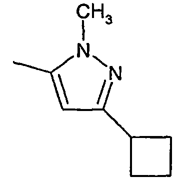 |
| 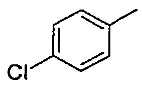 | -O- | 1 | 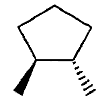 | 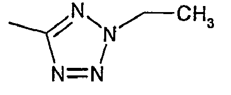 |
| 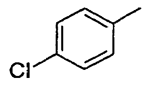 | -O- | 1 | 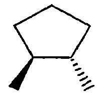 | 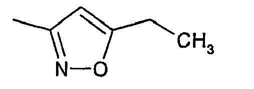 |
| 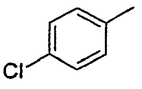 | -O- | 1 | 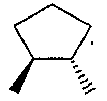 | 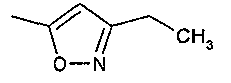 |

5. Verbindung nach einem der vorhergehenden Ansprüche in Kombination mit einem weiteren Wirkstoff, der eine antiinflammatorische, bronchodilatorische, antihistaminische oder antitussive Substanz ist.

6. Verbindung nach einem der vorhergehenden Ansprüche zur Verwendung als ein Pharmazeutikum.

7. Pharmazeutische Zusammensetzung, umfassend als Wirkstoff eine Verbindung nach einem der Ansprüche 1 bis 4.

8. Verwendung einer Verbindung nach einem der Ansprüche 1 bis 4 zur Herstellung eines Arzneimittels für die Behandlung eines durch CCR-3 mediierten Zustands.

9. Verwendung einer Verbindung nach einem der Ansprüche 1 bis 4 zur Herstellung eines Arzneimittels für die Behandlung eines inflammatorischen oder allergischen Zustands, insbesondere einer inflammatorischen oder obstruktiven Atemwegkrankheit.

10. Verfahren zur Herstellung einer Verbindung der Formel la oder Ib gemäß Anspruch 1, das umfasst
(i) (A) zur Herstellung von Verbindungen der Formel Ia, worin R² für Wasserstoff steht, eine Umsetzung der Formel IIa 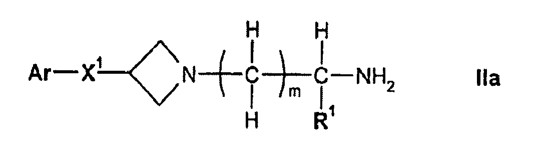 oder einer geschützten Form hiervon, worin Ar, x¹, m und R¹ wie im Anspruch 1 definiert sind, mit einer Verbindung der Formel III
Y=C=N-R³ III
worin Y und R³ wie im Anspruch 1 definiert sind,
oder
(B) zur Herstellung von Verbindungen der Formel Ia, worin Y für Sauerstoff steht, eine Umsetzung einer Verbindung der Formel IIa, worin Ar, X¹, m und R¹ wie im Anspruch 1 definiert sind, mit einer Verbindung der Formel IV 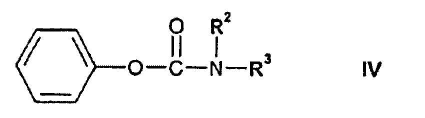 worin R² und R³ wie im Anspruch 1 definiert sind,
oder
(C) zur Herstellung von Verbindungen der Formel Ia, worin X¹ für-S(=O)₂- steht, eine Oxidation einer Verbindung der Formel Ia in geschützter Form, worin X¹ für -S- steht und Ar, m, R¹, Y, R² und R³ wie im Anspruch 1 definiert sind,
(D) zur Herstellung von Verbindungen der Formel Ib, eine Umsetzung der Formel IIb 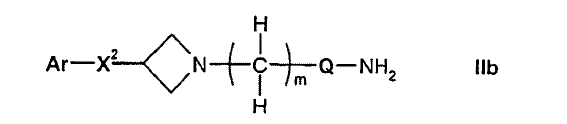 worin Ar, X², m und Q wie im Anspruch 1 definiert sind, mit einer Verbindung der Formel IV, worin R² und R³ wie im Anspruch 1 definiert sind,
(E) zur Herstellung von Verbindungen der Formel Ib, worin R² für Wasserstoff steht, eine Umsetzung einer Verbindung der Formel IIb, worin Ar, X², m und Q wie im Anspruch 1 definiert sind, mit einer Verbindung der Formel V
O=C=N-R³ V
worin R³ wie im Anspruch 1 definiert ist,
oder
(F) zur Herstellung von Verbindungen der Formel Ib, worin X für -S(=O)₂- steht, eine Oxidation einer Verbindung der Formel Ib in geschützter Form, worin X² für-S- steht und Ar, m, Q, R² und R³ wie im Anspruch 1 definiert sind, und
(ii) Gewinnung des Produkts in freier Form oder in Form eines Salzes.

## Revendications

1. Composé de la formule Ia ou Ib 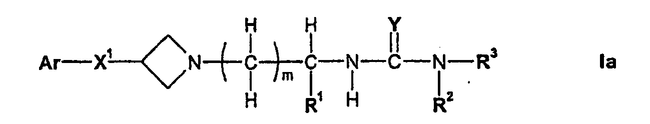 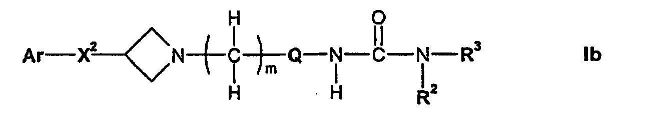 sous forme libre ou de sel, dans laquelle
Ar est un phényle éventuellement substitué par un ou plusieurs substituants choisis parmi l'halogène, un alkyle en C₁-C₈, un cyano ou nitro ;
X¹ est un -S-, -S(=O)-, ou -S(=O)₂- ;
X² est un -C(=O)-, -O-, -CH₂-, -S-, -S(=O)- ou -S(=O)2- ;
m est égal à 1, 2, 3 ou 4 :
R¹ est un hydrogène ou alkyle en C₁-C₈ éventuellement substitué par un hydroxy, un alcoxy en C₁-C₈, un acyloxy, un halogène, un carboxy, un alcoxycarbonyle en C₁-C₈, un -N(R⁴)R⁵, -CON(R⁶)R⁷ ou par un groupe organique cyclique monovalent ayant de 3 à 15 atomes dans le système de cycle ;
Q a la formule : 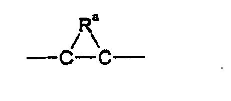
dans laquelle R^{a} est un alcylène en C₁-C₈,
ou Q est un -C(R^{b})(R^{c})- où R^{b} et R^{c} sont de manière indépendante un alkyle en C₁-C₈
ou R^{b} et R^{c} forment ensemble un cycloalkyle en C₃-C₁₀;
Y est un atome d'oxygène ou de soufre ;
R² est un atome d'hydrogène, un alkyle en C₁-C₈, un cycloalkyle en C₃-C₁₀ et R³ est un alkyle en C₁-C₈ substitué par un phényle, phénoxy, acyloxy ou naphthyle, ou R³ est un cycloalkyle en C₃-C₁₀, ayant éventuellement un groupe benzo fusioné, un groupe hétérocyclique ayant de 5 à 11 chaînons dont 1 à 4 sont des hétéroatomes, un phényle ou naphthyle, lesdits groupes phényle, phénoxy ou naphthyle étant éventuellement substitués par un ou plusieurs substituants choisis parmi un halogène, un cyano, un hydroxy, un acyle, un nitro, un -SO₂NH₂, un alkyle en C₁-C₈ éventuellement substitué par un alcoxy en C₁-C₈, un haloalkyle en C₁-C₈, un alcoxy en C₁-C₈, un haloalcoxy en C₁-C₈, un alkylthio en C₁-C₈, un - SO₂-alkyle en C₁-C₈, un alcoxycarbonyle en C₁-C₈, un acylamino en C₁-C₈ éventuellement substitué sur l'atome d'azote par un alkyle en C₁-C₈, un alkylamino en C₁-C₈, un aminocarbonyle, un alkylaminocarbonyle en C₁-C₈, un di(alkyle en C₁-C₈)amino, un di(alkyle en C₁-C₈)aminocarbonyle, un di(alkyle en C₁-C₈)aminocarbonyl-méthoxy,
Ou R² et R³, ensemble avec l'atome d'azote auquel ils sont fixés, représentent un groupe hétérocyclique ayant de 5 à 10 chaînons dont 1, 2 ou 3 sont des hétéroatomes ;
R⁴ et R⁵ sont chacun de manière indépendante de l'hydrogène ou un alkyle en C₁-C₈, ou R⁴ est un hydrogène et R⁵ est un hydroxyalkyle en C₁-C₈, un acyle, un -SO₂R⁸ ou -CON(R⁶)R⁷ ou R⁴ et R⁵, ensemble avec l'atome d'azote auquel ils sont fixés, représentent un groupe hétérocyclique à 5 ou 6 éléments ;
R⁶ et R⁷ sont chacun de manière indépendante un hydrogène ou un alkyle en C₁-C₈, ou R⁶ et R⁷, ensemble avec l'atome d'azote auquel ils sont fixés, représentent un groupe hétérocyclique à 5 ou 6 éléments ; et R⁸ est un alkyle en C₁-C₈, un haloalkyle en C₁-C₈, ou un phényle éventuellement substitué par un alkyle en C₁-C₈.

2. Composé selon la revendication 1, qui est
(i) un composé de la formule la sous forme de sel ou libre, dans lequel
Ar est un phényle substitué par un halo;
X¹ est un -S-, -S(=O)- ou -S(=O)₂- ;
m est égal à 2 ;
R¹ est un alkyle en C₁-C₈ éventuellement substitué par un hydroxy ou un alcoxy en C₁-C₈ ;
Y est un oxygène
R² est un hydrogène ; et
R³ est un groupe hétérocyclique ayant de 5 à 11 chaînons dont 1 à 4 sont des hétéroatomes ; ou
(ii) un composé de la formule Ib sous forme de sel ou libre, dans lequel
Ar est un phényle substitué par un halo ;
X² est un -O-, (C(=O)- ou un -CH₂- ;
m est égal à 1 ou 2 ;
Q a la formule 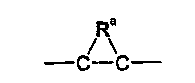 dans laquelle R^{a} est un alcylène en C₁-C₈,
ou Q est un -C(R^{b})(R^{c})- où R^{b} et R^{c} sont de manière indépendante un alkyle en C₁-C₈
ou R^{b} et R^{c} forment ensemble un cycloalkyle en C₃-C₁₀ ;
R² est un hydrogène ; et
R³ est un groupe hétérocyclique ayant de 5 à 11 chaînons dont 1 à 4 sont des hétéroatomes.

3. Composé selon la revendication 1, qui est
(i) un composé de la formule la sous forme de sel ou libre, dans lequel
Ar est un phényle substitué par un halo, de préférence un chloro;
X¹ est un -S-, -S(=O)- ou -S(=O)₂-;
m est égal à 2;
R¹ est un alkyle en C₁-C₄ éventuellement substitué par un hydroxy ou un alcoxy en C₁-C₄;
Y est un oxygène
R² est un hydrogène ; et
R³ est un groupe hétérocyclique ayant 5, 6 ou 7 chaînons dont un, deux, trois ou quatre sont des hétéroatomes choisis parmi l' azote, l'oxygène et le soufre, ledit groupe hétérocyclique étant éventuellement substitué par un alkyle en C₁-C₄, un alcoxy en C₁-C₄ ou un cycloalkyle en C₃-C₆ ; ou
(ii) un composé de la formule Ib sous forme de sel ou libre, dans lequel
Ar est un phényle substitué par un halo, de préférence un chloro ;
X² est un -O-, -C(=O)- ou un -CH₂- ;
m est égal à 1 ou 2 ;
Q a la formule 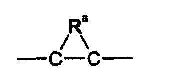 dans laquelle R^{a} est un alcylène en C₁-C₈,
ou Q est un -C(R^{b})(R^{c})- où R^{b} et R^{c} sont de manière indépendante un alkyle en C₁-C₄
ou R^{b} et R^{c} forment ensemble un cycloalkyle en C₃-C₆ ;
R² est un hydrogène ; et
R³ est un groupe hétérocyclique ayant 5, 6 ou 7 chaînons dont un, deux, trois ou quatre sont des hétéroatomes, choisis parmi l'azote, l'oxygène et le soufre, ledit groupe hétérocyclique étant éventuellement substitué par un alkyle en C₁-C₄ ou un cycloalkyle en C₃-C₆.

4. Composé selon la revendication 1, qui est soit un composé de la formule XX 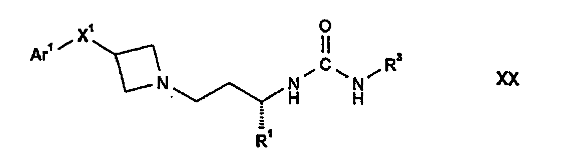 dans laquelle Ar, X¹ et R³ sont comme indiqués dans le tableau suivant,
soit un composé de la formule XXI
| Ar | X | R¹ | R³ |
|---|---|---|---|
| 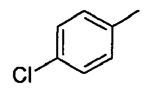 | 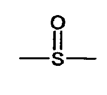 | 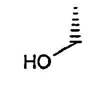 | 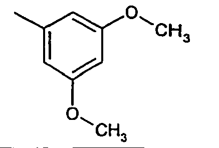 |
| 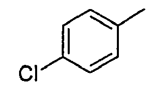 | 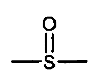 | 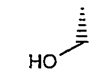 | 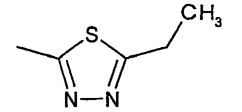 |
| 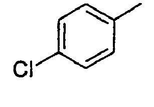 | 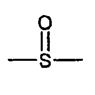 | 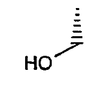 | 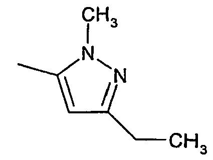 |
| 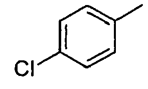 | 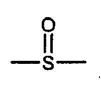 | 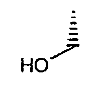 | 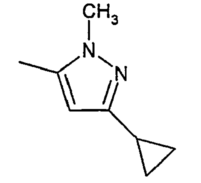 |
| 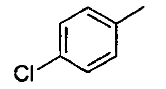 | 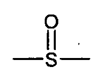 | 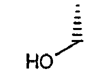 | 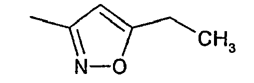 |
| 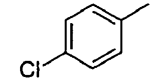 | 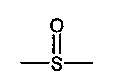 | 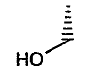 | 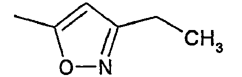 |
| 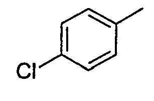 | 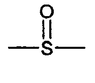 | 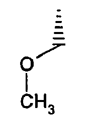 | 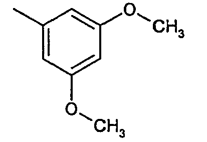 |
| 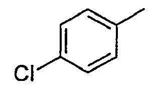 | 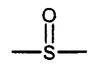 | 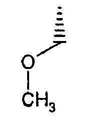 | 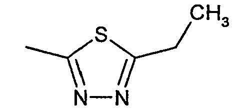 |
| 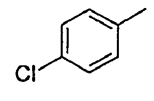 | 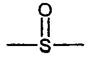 | 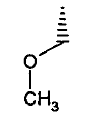 | 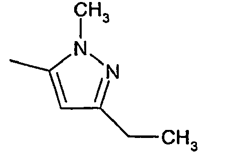 |
| 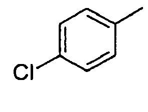 | 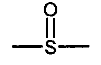 | 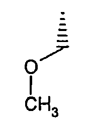 | 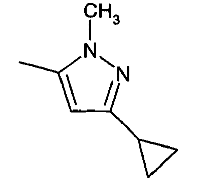 |
| 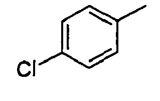 | 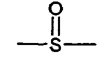 | 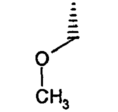 | 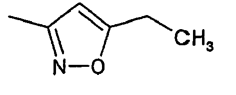 |
| 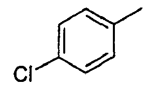 | 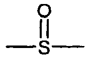 | 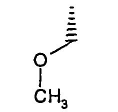 | 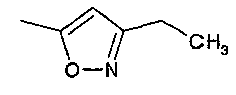 |
| 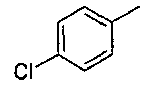 | 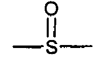 | 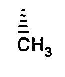 | 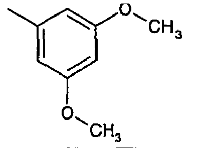 |
| 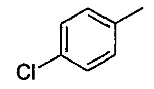 | 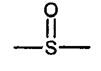 | 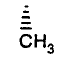 | 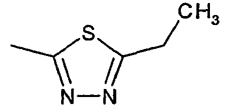 |
| 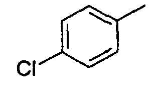 | 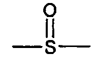 | 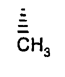 | 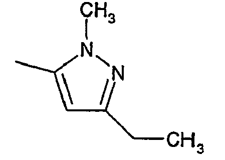 |
| 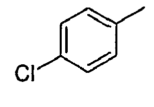 | 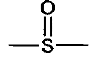 | 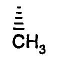 | 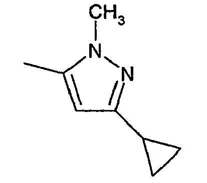 |
| 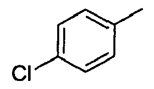 | 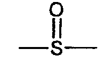 | 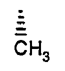 | 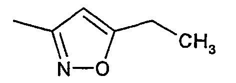 |
| 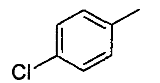 | 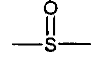 | 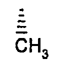 | 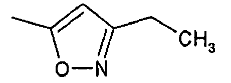 |
| 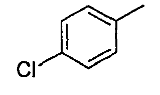 | -s- | 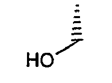 | 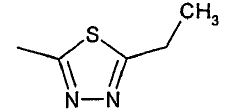 |
| 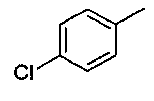 | -s- | 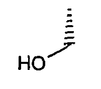 | 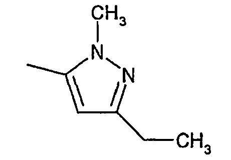 |
| 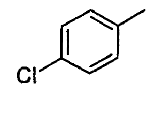 | -s- | 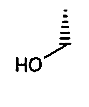 | 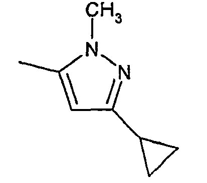 |
| 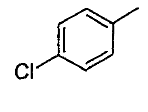 | -s- | 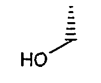 | 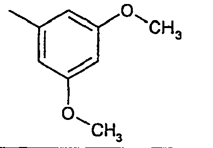 |
| 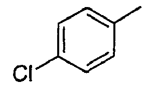 | -s- | 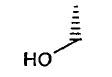 | 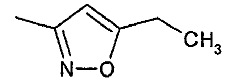 |
| 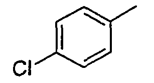 | -s- | 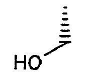 | 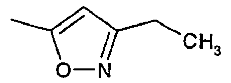 |
| 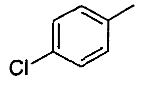 | 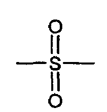 | 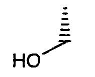 | 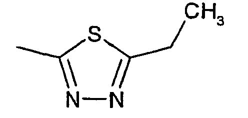 |
| 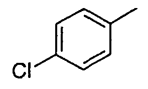 | 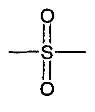 | 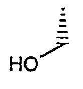 | 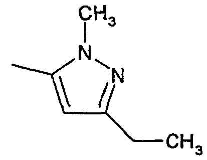 |
| 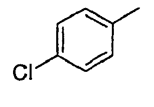 | 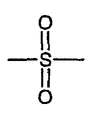 | 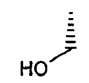 | 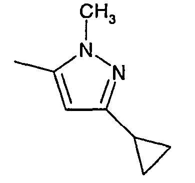 |
| 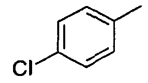 | 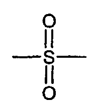 | 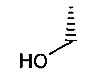 | 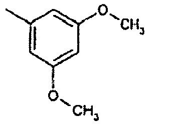 |
| 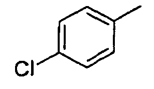 | 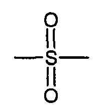 | 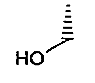 | 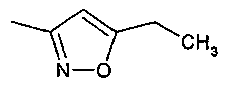 |
| 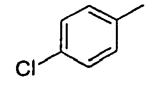 | 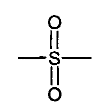 | 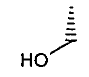 | 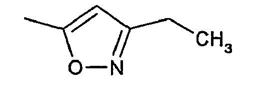 |

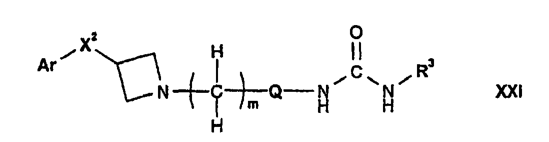
dans laquelle Ar, X², m, Q et R³ sont comme indiqués dans le tableau suivant.
| Ar | X | m | -Q- | R³ |
|---|---|---|---|---|
| 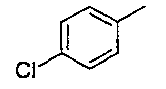 | -O- | 1 | 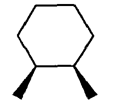 | 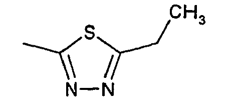 |
| 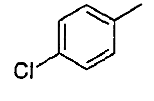 | -O- | 1 | 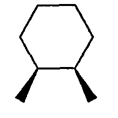 | 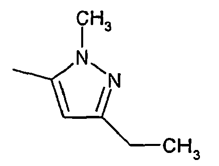 |
| 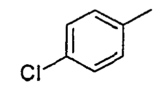 | -O- | 1 | 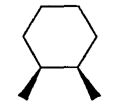 | 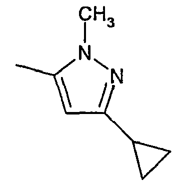 |
| 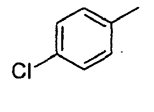 | -O- | 1 | 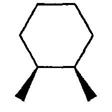 | 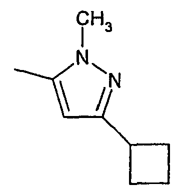 |
| 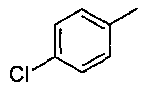 | -O- | 1 | 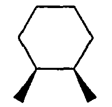 | 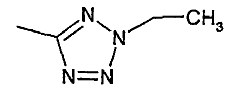 |
| 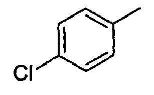 | -O- | 1 | 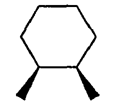 | 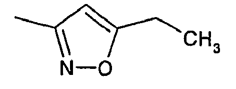 |
| 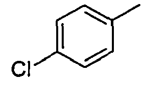 | -O- | 1 | 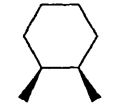 | 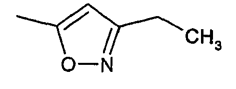 |
| 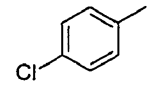 | 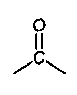 | 1 | 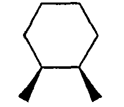 | 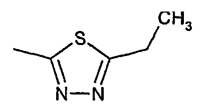 |
| 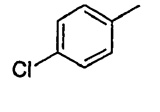 | 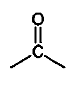 | 1 | 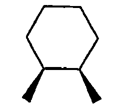 | 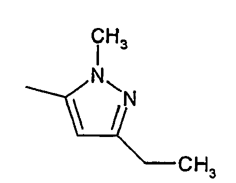 |
| 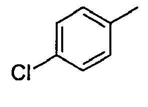 | 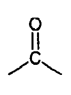 | 1 | 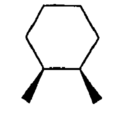 | 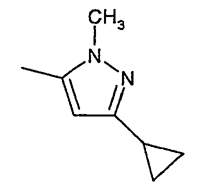 |
| 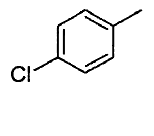 | 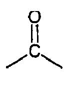 | 1 | 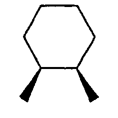 | 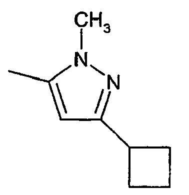 |
| 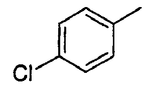 | 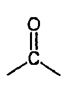 | 1 | 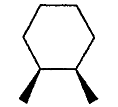 | 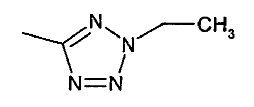 |
| 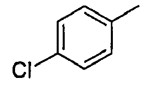 | 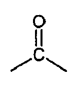 | 1 | 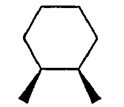 | 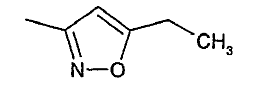 |
| 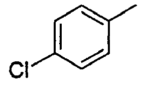 | 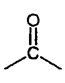 | 1 | 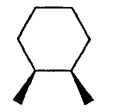 | 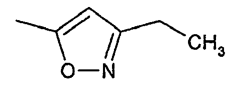 |
| 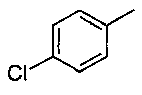 | 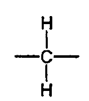 | 1 | 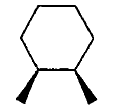 | 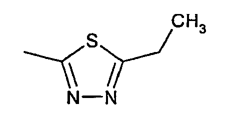 |
| 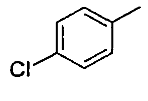 | 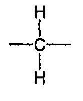 | 1 | 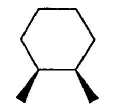 | 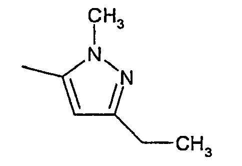 |
| 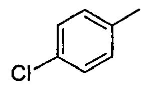 | 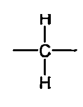 | 1 | 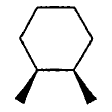 | 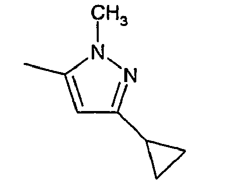 |
| 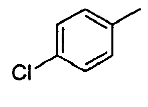 | 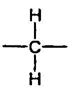 | 1 | 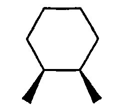 | 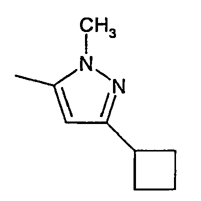 |
| 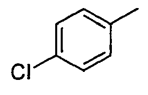 | 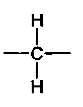 | 1 | 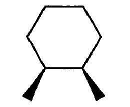 | 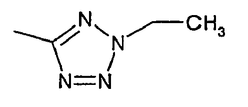 |
| 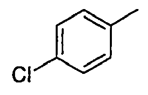 | 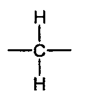 | 1 | 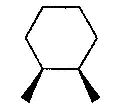 | 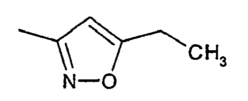 |
| 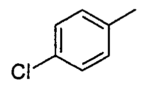 | 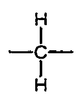 | 1 | 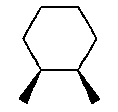 | 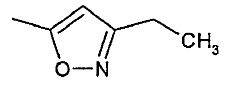 |
| 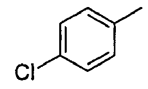 | -O- | 1 | 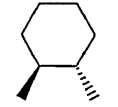 | 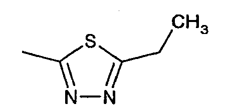 |
| 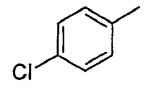 | -O- | 1 | 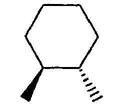 | 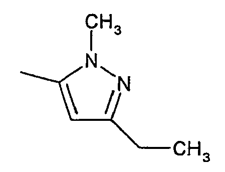 |
| 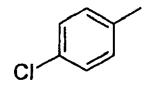 | -O- | 1 | 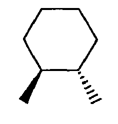 | 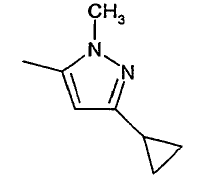 |
| 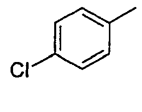 | -O- | 1 | 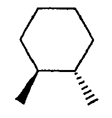 | 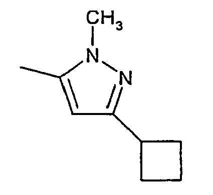 |
| 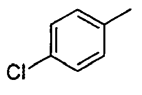 | -O- | 1 | 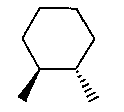 | 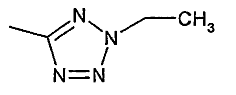 |
| 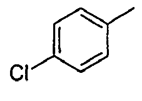 | -O- | 1 | 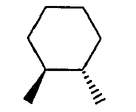 | 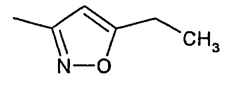 |
| 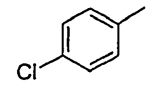 | -O- | 1 | 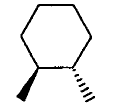 | 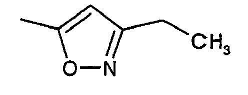 |
| 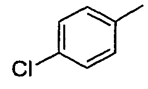 | 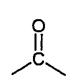 | 1 | 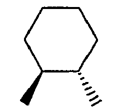 | 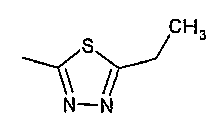 |
| 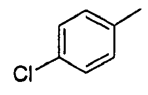 | 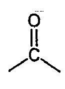 | 1 | 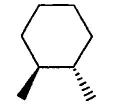 | 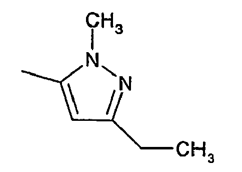 |
| 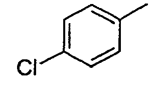 | 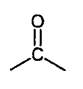 | 1 | 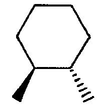 | 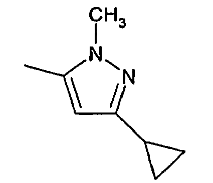 |
| 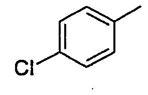 | 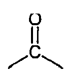 | 1 | 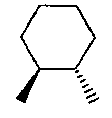 | 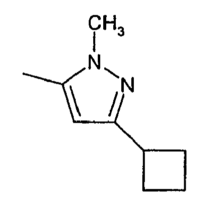 |
| 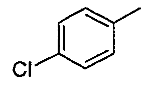 | 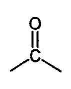 | 1 | 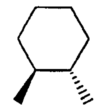 | 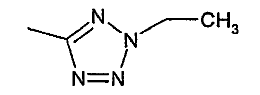 |
| 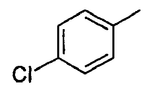 | 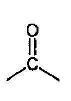 | 1 | 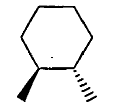 | 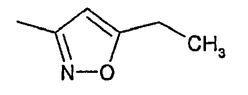 |
| 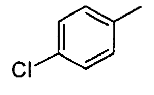 | 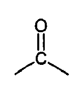 | 1 | 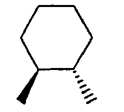 | 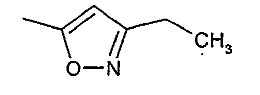 |
| 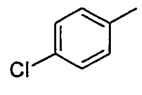 | 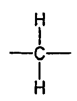 | 1 | 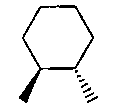 | 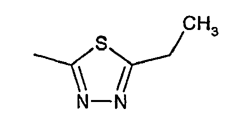 |
| 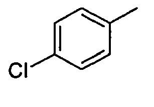 | 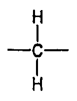 | 1 | 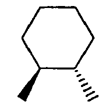 | 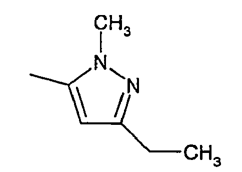 |
| 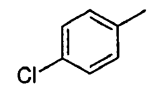 | 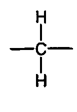 | 1 | 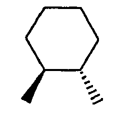 | 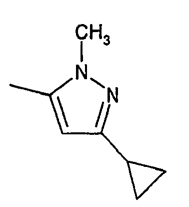 |
| 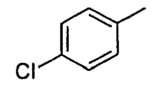 | 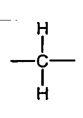 | 1 | 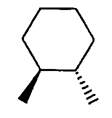 | 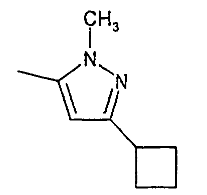 |
| 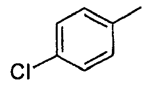 | 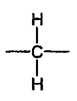 | 1 | 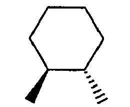 | 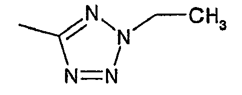 |
| 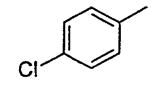 | 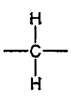 | 1 | 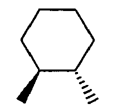 | 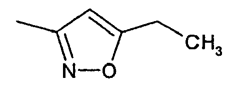 |
| 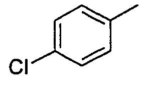 | 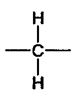 | 1 | 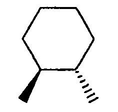 | 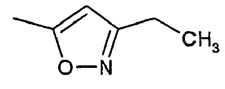 |
| 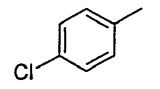 | -O- | 2 | 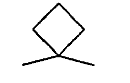 | 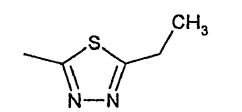 |
| 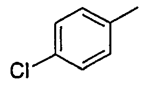 | -O- | 2 | 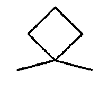 | 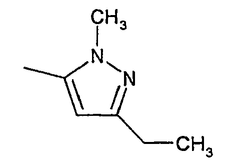 |
| 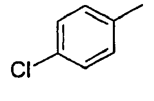 | -O- | 2 | 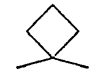 | 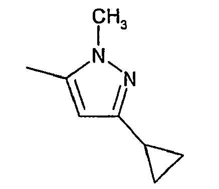 |
| 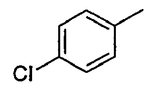 | -O- | 2 | 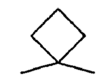 | 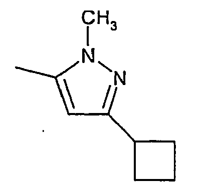 |
| 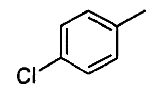 | -O- | 2 | 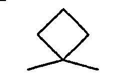 | 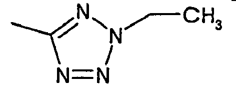 |
| 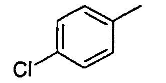 | -O- | 2 | 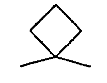 | 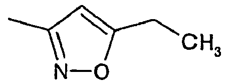 |
| 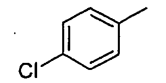 | -O- | 2 | 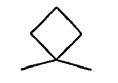 | 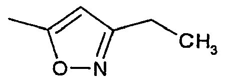 |
| 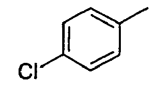 | 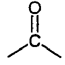 | 2 | 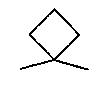 | 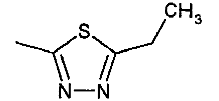 |
| 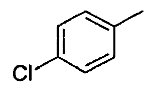 | 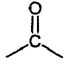 | 2 | 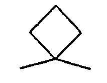 | 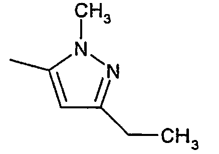 |
| 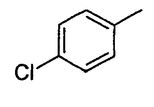 | 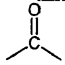 | 2 | 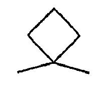 | 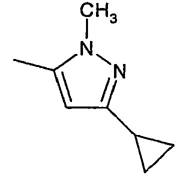 |
| 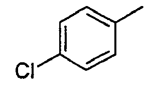 | 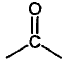 | 2 | 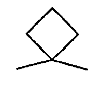 | 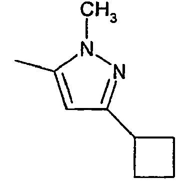 |
| 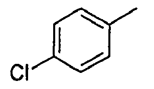 | 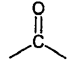 | 2 | 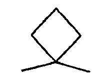 | 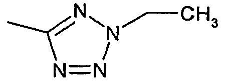 |
| 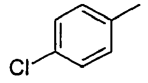 | 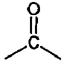 | 2 | 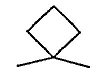 | 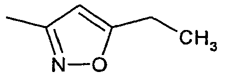 |
| 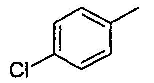 | 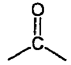 | 2 | 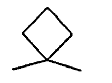 | 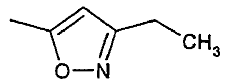 |
| 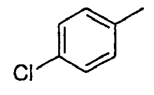 | 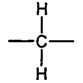 | 2 | 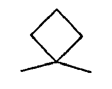 | 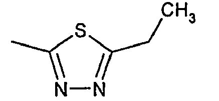 |
| 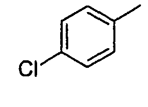 | 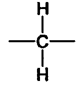 | 2 | 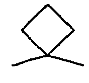 | 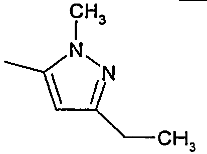 |
| 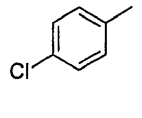 | 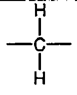 | 2 | 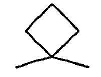 | 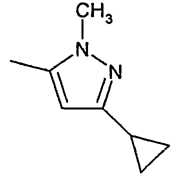 |
| 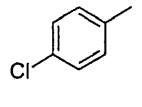 | 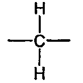 | 2 | 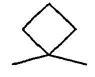 | 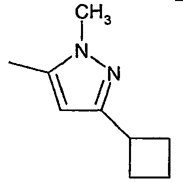 |
| 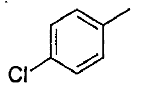 | 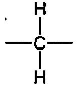 | 2 | 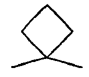 | 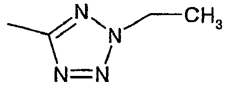 |
| 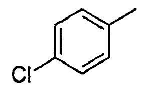 | 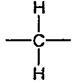 | 2 | 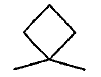 | 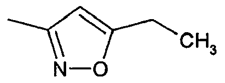 |
| 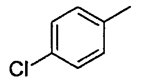 | 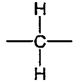 | 2 | 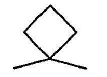 | 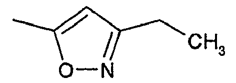 |
| 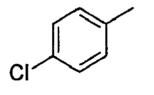 | -O- | 2 | 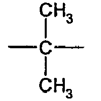 | 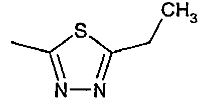 |
| 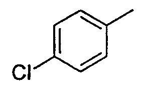 | -O- | 2 | 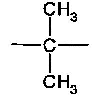 | 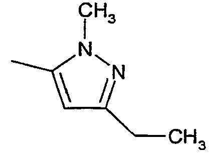 |
| 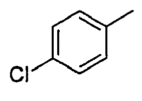 | -O- | 2 | 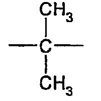 | 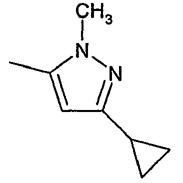 |
| 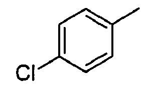 | -O- | 2 | 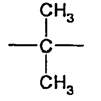 | 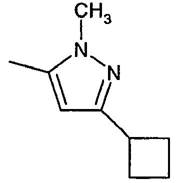 |
| 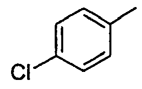 | -O- | 2 | 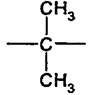 | 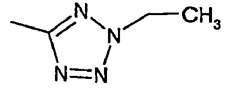 |
| 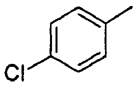 | -O- | 2 | 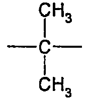 | 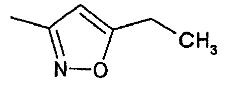 |
| 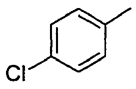 | -O- | 2 | 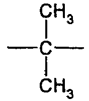 | 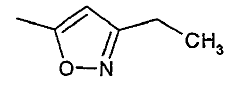 |
| 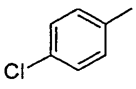 | 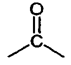 | 2 | 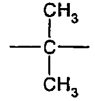 | 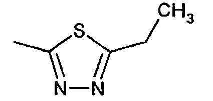 |
| 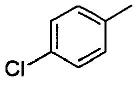 | 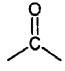 | 2 | 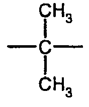 | 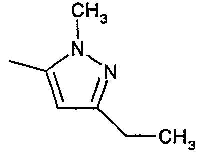 |
| 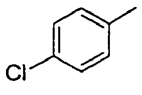 | 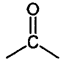 | 2 | 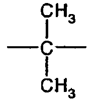 | 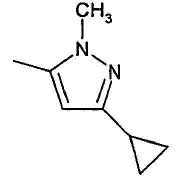 |
| 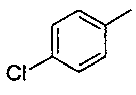 | 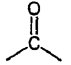 | 2 | 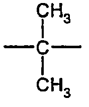 | 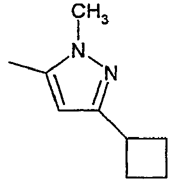 |
| 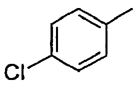 | 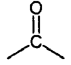 | 2 | 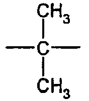 | 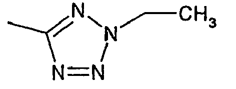 |
| 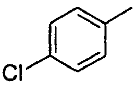 | 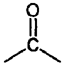 | 2 | 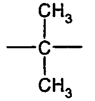 | 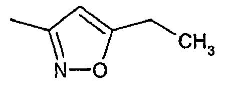 |
| 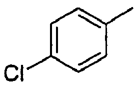 | 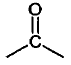 | 2 | 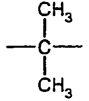 | 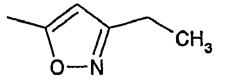 |
| 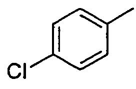 | 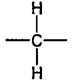 | 2 | 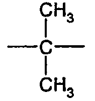 | 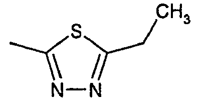 |
| 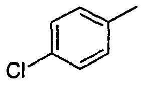 | 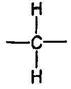 | 2 | 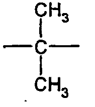 | 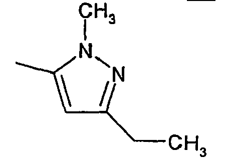 |
| 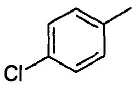 | 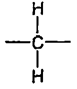 | 2 | 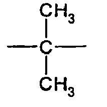 | 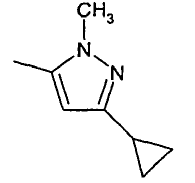 |
| 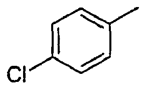 | 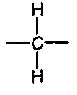 | 2 | 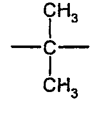 | 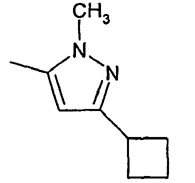 |
| 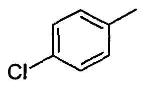 | 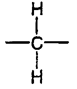 | 2 | 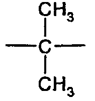 | 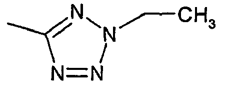 |
| 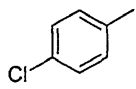 | 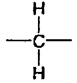 | 2 | 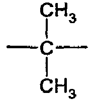 | 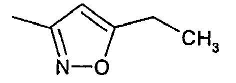 |
| 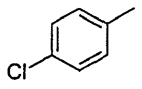 | 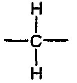 | 2 | 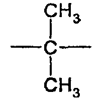 | 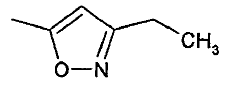 |
| 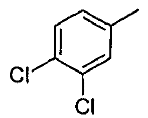 | -O- | 1 | 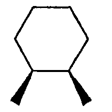 | 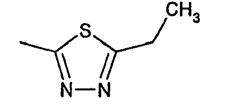 |
| 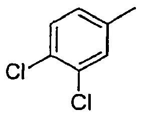 | -O- | 1 | 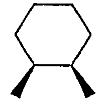 | 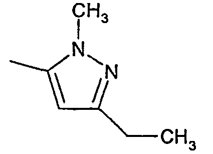 |
| 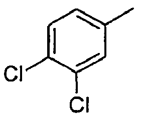 | -O- | 1 | 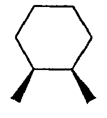 | 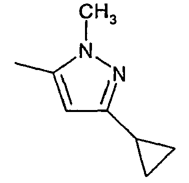 |
| 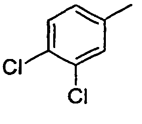 | -O- | 1 | 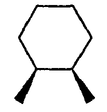 | 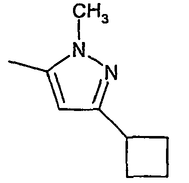 |
| 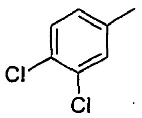 | -O- | 1 | 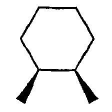 | 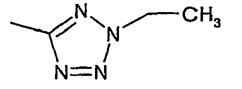 |
| 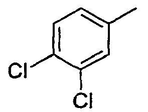 | -O- | 1 | 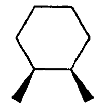 | 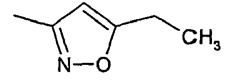 |
| 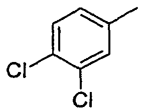 | -O- | 1 | 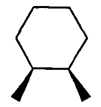 | 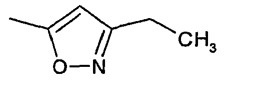 |
| 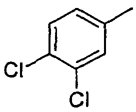 | -O- | 1 | 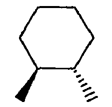 | 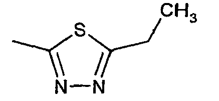 |
| 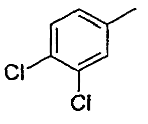 | -O- | 1 | 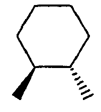 | 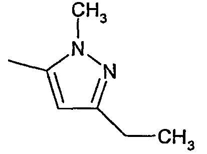 |
| 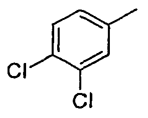 | -O- | 1 | 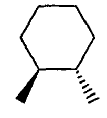 | 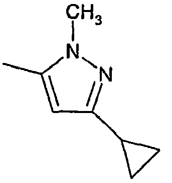 |
| 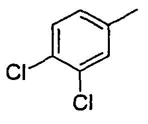 | -O- | 1 | 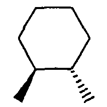 | 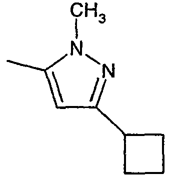 |
| 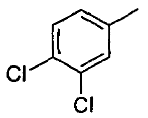 | -O- | 1 | 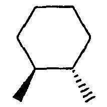 | 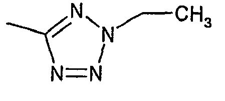 |
| 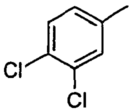 | -O- | 1 | 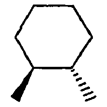 | 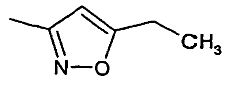 |
| 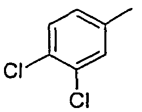 | -O- | 1 | 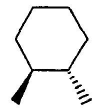 | 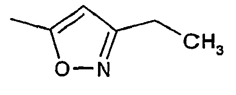 |
| 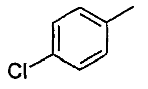 | -O- | 1 | 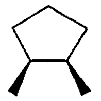 | 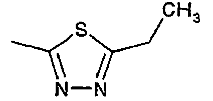 |
| 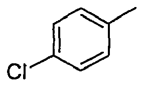 | -O- | 1 | 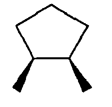 | 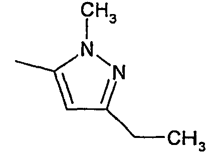 |
| 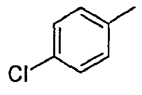 | -O- | 1 | 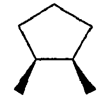 | 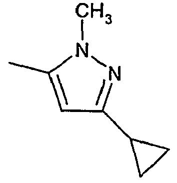 |
| 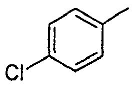 | -O- | 1 | 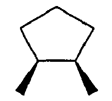 | 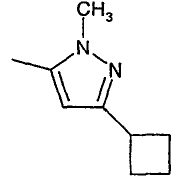 |
| 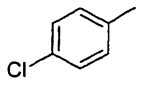 | -O- | 1 | 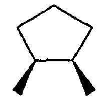 | 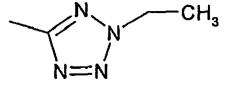 |
| 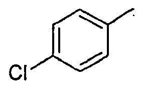 | -O- | 1 | 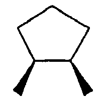 | 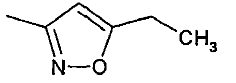 |
| 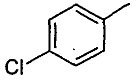 | -O- | 1 | 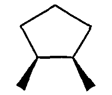 | 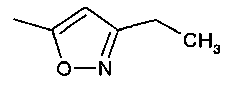 |
| 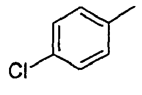 | -O- | 1 | 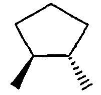 | 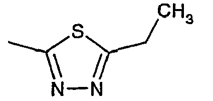 |
| 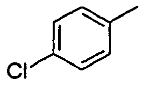 | -O- | 1 | 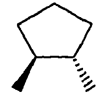 | 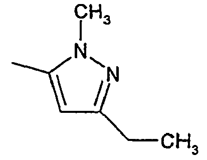 |
| 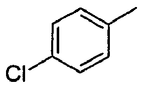 | -O- | 1 | 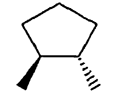 | 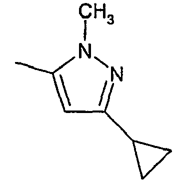 |
| 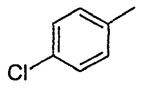 | -O- | 1 | 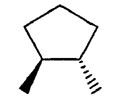 | 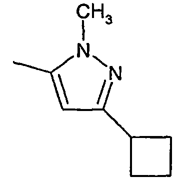 |
| 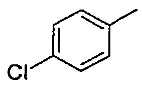 | -O- | 1 | 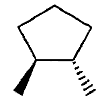 | 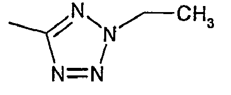 |
| 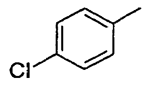 | -O- | 1 | 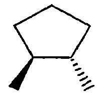 | 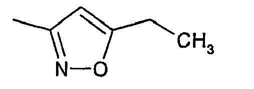 |
| 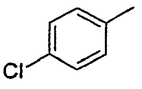 | -O- | 1 | 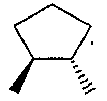 | 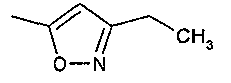 |

5. Composé selon l'une quelconque des revendications précédentes, en combinaison avec une autre substance médicamenteuse qui est une substance anti-inflammatoire, broncho-dilatatrice, antihistaminique ou antitussive.

6. Composé selon l'une quelconque des revendications précédentes, à utiliser comme un produit pharmaceutique.

7. Composition pharmaceutique comprenant, comme ingrédient actif, un composé selon l'une quelconque des revendications 1 à 4.

8. Utilisation d'un composé, selon l'une quelconque des revendications 1 à 4, pour la fabrication d'un médicament pour le traitement d'un état provoqué par le CCR-3.

9. Utilisation d'un composé, selon l'une quelconque des revendications 1 à 4, pour la fabrication d'un médicament pour le traitement d'un état inflammatoire
ou allergique, en particulier une maladie des voies aériennes inflammatoire ou obstructive.

10. Procédé pour la préparation d'un composé de la formule la ou Ib selon la revendication 1, qui comprend :
(i) (A) pour la préparation de composés de la formule la où R² est un hydrogène, la réaction d'un composé de la formule IIa 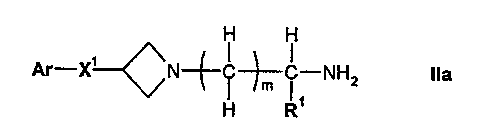 ou d'une forme protégée de celui-ci, où Ar, X¹, m et R¹ sont comme définis dans la revendication 1, avec un composé de la formule III
Y=C=N-R³ III
Où Y et R³ sont comme définis dans la revendication 1 ; ou
(B) pour la préparation de composés de la formule la où Y est un oxygène, la réaction d'un composé de la formule IIa où Ar, X¹, m et R¹ sont comme définis dans la revendication 1, avec un composé de la formule IV 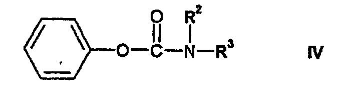 où R² et R³ sont comme définis dans la revendication 1 ; ou
(C) pour la préparation de composés de la formule la, où X¹ est un - S(=O)₂-, l'oxydation d'un composé de la formule la sous une forme protégée où X¹ est un -S- et Ar, m, R¹, Y, R² et R³ sont comme définis dans la revendication 1;
(D) pour la préparation de composés de la formule Ib, la réaction d'un composé de la formule IIb 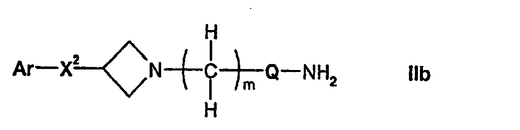 dans laquelle Ar, X², m et Q sont comme définis dans la revendication 1, avec un composé de la formule IV où R² et R³ sont comme définis dans la revendication 1 ;
(E) pour la préparation de composés de la formule Ib, où R² est un hydrogène, la réaction d'un composé de la formule IIb où Ar, X², m et Q sont comme définis dans la revendication 1, avec un composé de la formule V
O=C=N-R³ V
dans laquelle R³ est comme défini dans la revendication 1 ; ou
(F) pour la préparation de composés de la formule Ib où X est un -S(=O)₂-, l'oxydation d'un composé de la formule Ib, sous une forme protégée, où X² est un -S-, et Ar, m, Q, R² et R³ sont comme définis dans la revendication 1 ; et
(ii) la récupération du produit sous forme libre ou de sel.
